(19) Europäisches Patentamt
European Patent Office
Office européen des brevets

(11) **EP 3 283 879 B1**

(12) **EUROPÄISCHE PATENTSCHRIFT**

(45) Veröffentlichungstag und Bekanntmachung des Hinweises auf die Patenterteilung:
**27.03.2019 Patentblatt 2019/13**

(21) Anmeldenummer: **16716580.2**

(22) Anmeldetag: **15.04.2016**

(51) Int Cl.:
*C12Q 1/6806* (2018.01)     *G06T 7/00* (2017.01)
*G01N 33/543* (2006.01)     *G01N 21/64* (2006.01)
*G06K 9/00* (2006.01)     *G06K 9/46* (2006.01)
*G01N 21/76* (2006.01)

(86) Internationale Anmeldenummer:
**PCT/EP2016/058367**

(87) Internationale Veröffentlichungsnummer:
**WO 2016/166295 (20.10.2016 Gazette 2016/42)**

(54) **VERFAHREN ZUM REAKTIONSRAUMBEGRENZTEN NACHWEIS VON EINEM ODER MEHREREN ANALYTEN IN EINER PROBE**

METHOD FOR DETECTING ONE OR MORE ANALYTES IN A SAMPLE, SAID DETECTION BEING DELIMITED BY A REACTION CHAMBER

PROCÉDÉ POUR LA RECHERCHE D'UN OU DE PLUSIEURS ANALYTES, LIMITÉE À LA CHAMBRE DE RÉACTION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.04.2015 EP 15163773**

(43) Veröffentlichungstag der Anmeldung:
**21.02.2018 Patentblatt 2018/08**

(73) Patentinhaber: **Attomol GmbH Molekulare Diagnostika**
**03205 Lipten (DE)**

(72) Erfinder: **LEHMANN, Werner**
**03205 Bronkow (DE)**

(74) Vertreter: **Gulde & Partner**
**Patent- und Rechtsanwaltskanzlei mbB**
**Wallstraße 58/59**
**10179 Berlin (DE)**

(56) Entgegenhaltungen:
EP-A2- 1 053 784     WO-A1-2010/049430
WO-A1-2011/102903     WO-A2-02/078834
WO-A2-2011/109364     US-A1- 2005 019 556
US-A1- 2006 105 352     US-A1- 2010 285 573
US-A1- 2013 295 688     US-B1- 6 342 396
US-B1- 6 664 044

• KINOSHITA Y ET AL: "Novel concept microarray enabling PCR and multistep reactions through pipette-free aperture-to-aperture parallel transfer", BMC BIOTECHNOLOGY, BIOMED CENTRAL LTD. LONDON, GB, Bd. 10, Nr. 1, 6. Oktober 2010 (2010-10-06), Seite 71, XP021076466, ISSN: 1472-6750, DOI: 10.1186/1472-6750-10-71

EP 3 283 879 B1

SEQUENCE LISTING

<110> Attomol GmbH

<120> Verfahren zum Nachweis von einem oder mehreren Analyten in einer Probe

<130> P13233WO

<160> 11

<170> PatentIn version 3.5

<210> 1
<211> 18
<212> DNA
<213> Artificial Sequence

<220>
<223> Universalprimer 1

<400> 1
gcagcgtcga tggagtca          18

<210> 2
<211> 18
<212> DNA
<213> Artificial Sequence

<220>
<223> Universalprimer 2

<400> 2
actcacgctc acacggac          18

<210> 3
<211> 38
<212> DNA
<213> Artificial Sequence

<220>
<223> stx2e: Vorwärts-Primer mit 5'-Biotin-Markierung

<400> 3
gcagcgtcga tggagtcagg agcgttttga ccatcttc          38

<210> 4
<211> 39
<212> DNA
<213> Artificial Sequence

<220>
<223> stx2e: Rückwärts-Primer mit 5'-Biotin-Markierung

<400> 4
actcacgctc acacggacgc gccagatatg atgaaacca          39

<210> 5
<211> 27
<212> DNA
<213> Artificial Sequence

<220>
<223> stx2e: Sonde mit 5'- Cy5- und 3'-DeepDarkQuencher II-Modifizierung

<400> 5
ccggaatgca aatcagtcgt cactcac          27

<210> 6

<211> 40
<212> DNA
<213> Artificial Sequence

<220>
<223> stx1: Vorwärts-Primer mit 5'-Biotin-Markierung

<400> 6
gcagcgtcga tggagtcact ggatttaatg tcgcatagtg        40

<210> 7
<211> 40
<212> DNA
<213> Artificial Sequence

<220>
<223> stx1: Rückwärts-Primer mit 5'-Biotin-Markierung

<400> 7
actcacgctc acacggacga gaacgcccac tgagatcatc        40

<210> 8
<211> 31
<212> DNA
<213> Artificial Sequence

<220>
<223> stx1: Sonde mit 5'-Cy5- und 3'- DeepDarkQuencher II-Modifizierung

<400> 8
cggcaaatac agaggggatt tcgtacaaca c        31

<210> 9
<211> 41
<212> DNA
<213> Artificial Sequence

<220>
<223> eaeA: Vorwärts-Primer mit 5'-Biotin-Markierung

<400> 9
gcagcgtcga tggagtcacc tggttacaac attatggaac g        41

<210> 10
<211> 42
<212> DNA
<213> Artificial Sequence

<220>
<223> eaeA: Rückwärts-Primer mit 5'-Biotin-Markierung

<400> 10
actcacgctc acacggacgt gaaatagtct cgccagtatt cg        42

<210> 11
<211> 31
<212> DNA
<213> Artificial Sequence

<220>
<223> eaeA: Sonde mit 5'-Cy5- und 3'- DeepDarkQuencher II -Modifizierung

<400> 11
atgttgggct ataacgtctt cattgatcag g        31

## Beschreibung

Die vorliegende Erfindung beschreibt ein Verfahren zum Nachweis von Analyten in einer Probe, bevorzugt für bioanalytische, diagnostische (human und veterinär), umweltanalytische oder forensische Anwendungen, im Singleplex- als auch im Multiplex-Format.

Die Bestimmung von Spuren eines Analyten in entsprechenden Proben ist z.B. erforderlich, wenn es darum geht Frühstadien pathophysiologischer Prozesse zu erfassen. Das trifft insbesondere zu, wenn die Analyten im Normalzustand bei Gesunden gar nicht in der Probe gefunden werden oder bei Erkrankten nach Therapie nicht mehr gefunden werden sollten. Die frühe Diagnose von Erkrankungen bzw. von Rezidiven ist oftmals mit einer besseren therapeutischen Intervention verbunden und verbessert somit die Prognose des Patienten.

Für die Bestimmung von Proteinantigenen in biologischen Proben wurden Verfahren beschrieben, die aber zum Teil sehr aufwändig sind, nicht dem technischen Standard im Anwenderlabor entsprechen, eine absolute Quantifizierung nur anhand von Standards bzw. lediglich eine Singleplex-Analyse ermöglichen.

Schmidt et al. beschreiben ein Verfahren mit dem die Bindung einzelner Moleküle auf einem Array mit Hilfe einer sensitiven Kamera detektiert werden kann (Ronny Schmidt, Jaroslaw Jacak, Christopher Schirwitz, Volker Stadler, Gerd Michel, Nicole Marme, Gerhard J. Schütz, Jörg D. Hoheisel, and Jens-Peter Knemeyer, "Single-Molecule Detection on a Protein-Array Assay Platform for the Exposure of a Tuberculosis Antigen." J. Proteome Res. 2011, 10, 1316-1322). Mit diesem Verfahren war es möglich ca. 600 Analyt-Moleküle in einem Spot immobilisierter Fängermoleküle nachzuweisen, was einer Gesamtanalytkonzentration in der Probe von etwa $10^{-14}$ M entspricht. Mit diesem Verfahren können mehrere Spots für unterschiedliche Analyten gleichzeitig bestimmt werden. Die Bindung einzelner Moleküle konnte bei totaler interner Reflexion (TIRF) unter Verwendung einer Einzelmolekülfluoreszenzoptik gezeigt werden. Die Sensitivität des Systems wurde durch Hintergrundfluoreszenz an Störstellen in der Oberflächenmatrix bzw. durch unspezifisch gebundene Moleküle begrenzt. Da das System auf TIRF basiert, können die Tests als homogene Immunoassays ohne jeglichen Waschschritt durchgeführt werden. Die Autoren halten dieses Verfahren besonders für sensitive Schnelltests geeignet, wobei die Einzelmolekülanalyse den Einsatz eines kostspieligen Fluoreszenzscanners voraussetzt.

Rissin et al. beschreiben ein System (Simoa, Quanterix), welches durch Verwendung von Femtoliter-Kavitäten das Reaktionsvolumen enzymatischer Reaktionen begrenzt. Damit ist es möglich 10-20 Enzymmoleküle pro "gespickter" Probe und etwa $10^{-15}$ M Proteinantigene mittels Immunoassay in Serum, einer üblichen Probenmatrix im Anwenderlabor, zu bestimmen (Rissin et al., "Single-molecule enzyme-linked immunosorbent assay detects serum proteins at subfemtomolar concentrations." Nature Biotechnology 28, 595-599, 2010). Dieses System wurde von Wilson et al. erfolgreich zur Bestimmung von prostataspezifischem Antigen (PSA) im Serum von Patienten nach Prostataektomie mit einer Sensitivität von <1pg PSA/ml eingesetzt (Wilson et al., "Fifth-Generation Digital Immunoassay for Prostate-Specific Antigen by Single Molecule Array Technology", Clinical Chemistry 57:12, 1712-1721, 2011). Dadurch war es möglich, bei einigen Patienten das Rezidiv eines Prostatakarzinoms früher zu detektieren als mit konventionellen Immunoassays.

Todd et al. beschreiben einen sensitiven Immunoassay (Erena) zur Einzelmolekülzählung, bei dem magnetische Nanopartikel die Fangantikörper zur Bindung des Analyten aus der Probe auf der Oberfläche tragen (Todd et al., "Ultrasensitive Flow-based Immunoassays Using Single-Molecule Counting." Clinical Chemistry 53:11, 1990-1995, 2007). Die gebundenen Analyten wurden über einen Nachweisantikörper fluoreszenzmakiert und anschließend in einem laserbasierten, flow-metrischen Messsystem analysiert. Damit gelang es 10-100pg Troponin I/ml Patientenprobe nachzuweisen. Erhöhte Konzentrationen von Troponin I (>10ng/ml) im Plasma gelten als Risikofaktor für Herz-Erkrankungen. US 2006/105352 A1 offenbart ein Multiplex-Verfahren zum Nachweis von Analyten in einer Probe, wobei vor der Durchführung einer Signalverstärkungsreaktion auf einem Träger eine Mehrzahl räumlich getrennter Reaktionsräume eingerichtet werden, indem nach Aufbringen der Probe und vor Aufbringen der Reaktionslösung Mikropartikel in der Gelatineschicht des Trägers immobilisiert werden, so dass die Mikropartikel von der Reaktionslösung überschichtet sind.

Der Stand der Technik zeigt, dass mit verschiedensten Techniken bereits eine sensitive Detektion von Analyten in Patientenproben im Rahmen der Infektionsdiagnostik, der Tumordiagnostik bzw. Herz-Kreislauf-Diagnostik möglich ist. Alle Methoden weisen spezielle Vor- und Nachteile auf, wie z.B. teure Detektionsgeräte, eine strikte Notwendigkeit für hochsensitive Detektoren (Krishnaswami et al., Optical Nanoscopy 2014,bzw. Schmidt et al. 2011), den Einsatz von wenig routinetauglichen Verbrauchsmaterialien wie planare TIRF-Arrays oder Reaktionsgefäße mit Femtoliterkavitäten oder der Einsatz von Flow-Systemen, deren Arbeitsweise nur schwer zu parallelisieren ist. Insgesamt ist die Multiplex-Fähigkeit mit Ausnahme des von Schmidt et al. beschriebenen Systems nicht gegeben. Alle diese Systeme wurden für die Anwendung in Immunoassays beschrieben, doch ist dem Fachmann klar, dass sie ebenfalls für andere Analyten wie z.B. die Nukleinsäuredetektion eingesetzt werden können. Das Detektionslimit liegt für reale Proben wie Serum bei etwa $10^{-14}$ M. Die Sensitivität wird vor allem durch die Kinetik der Interaktion der als Bindungspartner beteiligten Biomoleküle, die u.a. von der unspezifischen Bindung, der Bindungskonstanten der Reaktionspartner oder Diffusion abhängt, begrenzt, so dass nie alle Moleküle in einer Probe nachgewiesen werden können.

Bei der digitalen PCR können alle Nukleinsäuretargetmoleküle einer Probe nachgewiesen werden, da die Probe komplett in eine Vielzahl (bis 10 x $10^6$) von Mikroreaktionsräumen (oft Öl-/Wasseremulsionen) überführt wird, so dass in Tropfen mit einem Durchmesser von ca. 50μm statistisch gesehen nur ein Molekül anzutreffen ist (Vogelstein, B. and Kinzler, K.W. "Digital PCR." Proc. Natl. Acad. Sci. USA 96, 9236-9241, 1999; Fan http://purl.stanford.edu/cw095xw9265, Dissertation, Stanford University, 2011). Nach der Einzelmolekül-PCR werden die Tropfen gezählt, die spezifisches PCR-Produkt enthalten, um so die Zahl der Moleküle pro Probe zu errechnen. Die digitale PCR ist in allen Bereichen der Nukleinsäureanalytik einsetzbar und hat den Vorteil der absoluten Quantifizierung. Dieses Verfahren ist insofern umständlich, da die Tropfenherstellung ein komplizierter mikrofluidischer Prozess ist und bislang nicht in einem Standardformat (96-Well-Mikrotestplatte oder 8er-Riegel) für die Laborroutine erhältlich ist.

Aufgabe der vorliegenden Erfindung ist es einen oder mehrere Nachteile des Standes der Technik zu vermindern oder zu vermeiden. Insbesondere ist es eine Aufgabe der vorliegenden Erfindung ein Verfahren zur Verfügung zu stellen, mit dem Analyten in einer Probe nachgewiesen werden können.

Der Nachweis des Analyten in einer Probe erfolgt erfindungsgemäß in räumlich getrennten Reaktionsräumen. Die Einrichtung dieser räumlich getrennten Reaktionsräume erfolgt durch Einsatz von Mikropartikeln. Auf der Oberfläche eines Trägers immobilisierte Mikropartikel sind von einer Reaktionslösung überschichtet. Anschließend wird die Reaktionslösung entfernt, so dass benachbarte sich nicht berührende Mikropartikel nicht mehr von einer zusammenhängenden Schicht Reaktionslösung überdeckt sind. Dabei wirken die Mikropartikel als Nukleationspunkte um die ein Flüssigkeitshof verbleibt, der einen räumlich getrennten Reaktionsraum bildet. Durch Kapillarkräfte an den Grenzflächen zwischen Mikropartikel und Träger werden so räumlich getrennte Reaktionsräume ausgebildet, in denen anschließend separat eine Signalverstärkungsreaktion erfolgen kann, wobei die Menge an gebildetem Reaktionsprodukt in einem räumlich getrennten Reaktionsraum als Maß für die Menge an zu messenden Analyten in diesen Reaktionsräumen verwendet werden kann.

Das erfindungsgemäße Verfahren umfasst bevorzugt die Schritte: a) Aufbringen einer Probe und einer Reaktionslösung auf einen Träger, wobei die Reaktionslösung geeignet ist in einer Signalverstärkungsreaktion in Abhängigkeit des Vorhandenseins des nachzuweisenden Analyten ein messbares Reaktionsprodukt entstehen zu lassen; b) Durchführung der Signalverstärkungsreaktion; c) optische Messung der Reaktionsprodukte; und d) ggf. Bereitstellung der Messdaten für die Berechnung einer Analytkonzentration in einer Probe, wobei vor der Durchführung der Signalverstärkungsreaktion auf dem Träger eine Mehrzahl räumlich getrennter Reaktionsräume eingerichtet wird, indem vor, während oder nach dem Aufbringen der Probe und der Reaktionslösung Mikropartikel auf dem Träger immobilisiert werden, so dass die Mikropartikel von der Reaktionslösung überschichtet sind und anschließend die Reaktionslösung entfernt wird, so dass benachbarte, sich nicht berührende immobilisierte Mikropartikel nicht mehr von einer zusammenhängenden Schicht Reaktionslösung überdeckt sind;

dadurch gekennzeichnet, dass die Entfernung der Reaktionslösung derart erfolgt, dass die Oberfläche des Trägers lediglich in einem Bereich mit Reaktionslösung bedeckt bleibt, der unmittelbar eine Kontaktstelle zwischen Mikropartikel und Träger umgibt, während Oberflächenbereiche des Trägers, die mindestens den ≥0,1-fachen Partikeldurchmesser von einer Kontaktstelle zwischen einem Mikropartikel und dem Träger entfernt sind, nicht mehr von Reaktionslösung bedeckt sind.

Mit der vorliegenden Erfindung wird dem Anwender ein skalierbares Messsystem verfügbar gemacht, dass wahlweise die Vorteile der im Stand der Technik beschriebenen Verfahren entsprechend der genannten Fragestellung des Anwenders optional kombinieren. Das erfindungsgemäße Verfahren zeichnet sich insbesondere dadurch aus, dass es mit Standardreagenzien ggf. im 96-Well-Format funktioniert, nahezu alle Analyt Moleküle in der Probe erfasst, multiplexfähig ist, die digitale Auswertung bzw. die Einzelmolekülzählung ermöglicht und mittels Standardmikroskop aber auch mit einem Mikroplatten-Readersystem preiswert ausgewertet werden kann.

Mithilfe des erfindungsgemäßen Verfahrens ist es sowohl möglich eine qualitative Aussage über das Vorhandensein oder die Abwesenheit der Analyten in der Probe zu treffen als auch eine quantitative Bestimmung der Menge der Analyten innerhalb dieser Probe durchzuführen.

Soweit nachfolgend der Kontext nicht eindeutig etwas anderes ergibt, ist bei der Verwendung von Singular-Formen bzw. Plural-Formen stets sowohl die Ein- als auch die Mehrzahl umfasst.

Das erfindungsgemäße Verfahren bezieht sich auf ein Verfahren zum in vitro Nachweis von Analyten in einer Probe. Als "in vitro" wird jede Umgebung verstanden, die sich nicht innerhalb eines lebenden Organismus, z.B. einem menschlichen oder tierischen Körper, befindet. Das erfindungsgemäße in vitro Nachweisverfahren umfasst also ausdrücklich kein Verfahren, welches am menschlichen oder tierischen Körper vorgenommen wird.

Analyt

Bei einem Analyt, der in einer Probe nachgewiesen werden soll, kann es sich um ein Analyt jeglicher Art bzw. Ursprungs handeln. So kann es sich bei einem Analyt beispielsweise um einen natürlichen Analyten aus einer tierischen, pflanzlichen, bakteriellen oder einer ähnlichen Quelle handeln oder er kann synthetischen Ursprungs sein. Der Analyt kann ein organischer oder anorganischer Analyt sein. Bei dem Analyten kann es sich um ein Peptid, Protein, Enzym, Lektin, Antikörper, Antigen, Aptamer, Polysaccharid, Lipid, eine Nukleinsäure oder ähnliches handeln. Bevorzugt weist der nachzuweisende Analyt eine enzymatische Aktivität auf, die bei Kontakt mit der Reaktionslösung in einer Signalverstärkungsreaktion zu einem messbaren Reaktionsprodukt führt. Besonders bevorzugt handelt es sich bei der enzymatischen Aktivität um die Aktivität eines Enzyms aus der Klasse der DNA-Polymerasen, RNA-Polymerasen, reversen Transkriptasen, Ligasen, Oxidasen, Reduktasen, Transferasen, Peroxidasen, Phosphatasen, Proteasen, Peptidasen, Lipasen, Glykosidasen und/oder Luciferasen.

Die Nukleinsäure kann Desoxyribonukleinsäure (DNA, Abkürzung für englisch desoxyribonucleic acid) oder Ribonukleinsäure (RNA, Abkürzung für englisch ribonucleic acid) wie z.B. mikroRNA sein. Bevorzugt sind Nukleinsäuren, die bei Amplifikationsreaktionen, wie z.B. PCR-Reaktionen oder isothermaler Amplifikation entstehen. Durch die Verwendung von Primern mit target-spezifischem 3'-Ende und mit einem target-unspezifischen 5'-Sequenzbereich können während einer Vervielfältigungsreaktion Markierungssequenzen in die Nukleinsäuren eingebracht werden, die eine selektive Bindung an einer festen Phase wie z.B. bevorzugt an fluoreszenzkodierten Mikropartikeln ermöglichen. Bei den Analyten kann es sich auch um nicht natürliche Nukleinsäureanaloga oder Aminosäureanaloga handeln sowie um Verbindungen, die diese Analoga enthalten. Ein Analyt kann beispielsweise aus Nukleotiden, DNA, Aminosäuren, Peptiden oder einer Zusammensetzung aus diesen Stoffen bestehen.

Probe

Der Begriff "Probe" bezeichnet eine zu untersuchende Zusammensetzung, die für die ex vivo oder in vitro Untersuchung verwendet werden kann. Bei der Probe handelt es sich bevorzugt um biologisches oder medizinisches Material, also Material, das aus einem Organismus, von Bestandteilen eines Organismus oder aus Zellen gewonnen wird. Das Material kann, bevor es als Probe im erfindungsgemäßen Verfahren eingesetzt wird, weiteren Behandlungsschritten unterzogen werden, z.B. um das Material in einen Zustand zu versetzen, in dem es sich als Probe für das erfindungsgemäße Verfahren besonders eignet. Zur Aufbereitung können gängige Methoden verwendet werden, die dem Fachmann bekannt sind. So können beispielsweise bestimmte Stoffe oder Stoffklassen, wie z.B. Proteine, Lipide oder Nukleinsäuren, aus dem Material aufgereinigt werden, welche dann als Probe im erfindungsgemäßen Verfahren eingesetzt werden können.

Bei der Probe kann es sich um Material handeln, dass aus einer Körperflüssigkeit gewonnen wurde oder eine Körperflüssigkeit umfasst. "Körperflüssigkeit" kann jede Flüssigkeit des Körpers darstellen z.B. Serum, Blut, Plasma, Fruchtwasser, Schweiß, Urin, Stuhl, Cerebrospinalflüssigkeit, Liquor, Sperma, Atemkondensate etc. und in Form von Punktaten, Abstrichen o.ä. gewonnen werden. Die Probe kann auch aus einem zu untersuchenden Körpergewebe stammen oder ein Körpergewebe umfassen. Bevorzugt handelt es sich dabei um ein Körpergewebe aus einer Testperson, wobei die Testperson ein Tier sein kann, bevorzugt ein Säugetier, besonders bevorzugt ein Mensch. Bei dem Körpergewebe kann es sich auch um eine Probe aus einer Pflanze handeln. Die Probe kann auch aus einer Zellkultursuspension oder einem Zellkulturüberstand gewonnen werden oder eine Zellkultursuspension oder einen Zellkulturüberstand umfassen. Ebenso kann die Probe aus einer Boden- oder Wasserprobe gewonnen werden oder eine Boden- oder Wasserprobe umfassen. Vorzugsweise umfasst die Probe ein Gemisch aus verschiedenen Analyten.

**Schritt a): Aufbringen einer Probe und einer Reaktionslösung auf einen Träger**

In Schritt a) des erfindungsgemäßen Verfahrens werden eine Probe und eine Reaktionslösung auf einen Träger aufgebracht. Dabei können die Probe und die Reaktionslösung entweder nacheinander oder gleichzeitig auf den Träger aufgebracht werden. Bevorzugt wird die Probe vor der Reaktionslösung auf den Träger aufgebracht. Ebenfalls bevorzugt wird die Probe zeitgleich mit der Reaktionslösung auf den Träger aufgebracht, wobei die Probe und die Reaktionslösung vor dem Aufbringen auf den Träger z.B. miteinander gemischt werden können.

Reaktionslösung

Der Begriff "Reaktionslösung" bezeichnet ein im Wesentlichen flüssiges Medium. Die Reaktionslösung weist ein Substrat auf, welches während der Signalverstärkungsreaktion durch eine enzymatische Aktivität umgewandelt wird und dadurch das messbare Reaktionsprodukt entsteht. Entsprechend ist die Reaktionslösung geeignet in einer Signalverstärkungsreaktion in Abhängigkeit des Vorhandenseins des Analyten ein messbares Reaktionsprodukt entstehen zu lassen. Das Substrat, wird durch die enzymatische Aktivität des Analyten und/oder des Signalverstärkungssystems umgesetzt, wodurch ein messbares Reaktionsprodukt entsteht.

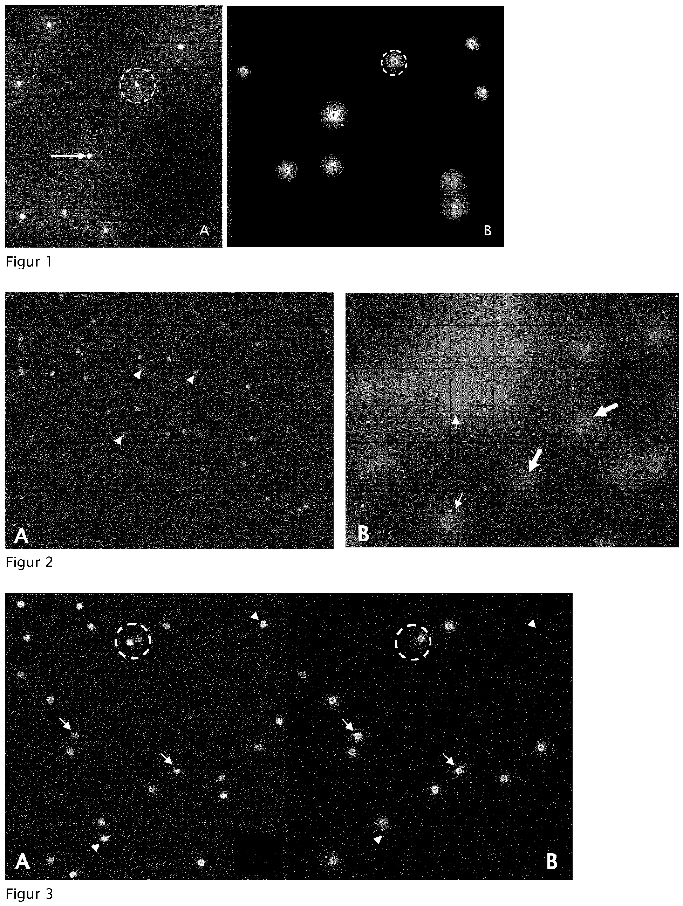

Handelt es sich bei der enzymatischen Aktivität des Signalverstärkungssystems um die Aktivität eines Enzyms z.B. einer Peroxidase, so können Substrate in der Reaktionslösung eingesetzt werden, die ein Reaktionsprodukt ergeben, welches durch seine Absorption messbar ist (siehe z.B. Fig. 1 und 2). Dazu zählen z.B. Tetramethylbenzidin (TMB), Orthophenylendiamin oder 2,2'-Azino-di-(3-ethylbenzthiazolin-6-sulfonsäure) (ABTS). Bevorzugt wird TMB als Substrat eingesetzt. Durch Umsetzung mithilfe der Peroxidase wird TMB zu einem blauen Reaktionsprodukt, welches durch seine Absorption im Bereich zwischen 600-700nm optisch messbar ist (Gallati und Pracht, "Bestimmung von Peroxidase mit H2O2 und 3,3',5,5'-Tetramethylbenzidin." J. Clin. Chem. Clin. Biochem. 23, 4453-460, 1985). Alternativ oder parallel können auch andere Absorptionspeaks desselben Farbstoffes genutzt werden wie z.B. die Absorptionspeaks des TMB-POD Reaktionsproduktes bei 370nm bzw. 450nm in Kombination mit Pyren, welches bei 340nm angeregt wird und bei 370nm emittiert.

Wenn das Signalverstärkungssystem die Aktivität eines Enzyms, z.B. einer Peroxidase (POD), aufweist, so können Substrate in der Reaktionslösung eingesetzt werden, die ein Reaktionsprodukt ergeben, welches durch seine Fluoreszenz optisch messbar ist. Dazu zählen z.B. Hydroxyphenylessigsäure (HPA), Hydroxylphenylproprionylsäure (HPPA) oder Ampliflu Red (Sigma) oder ein Tyramide Signal Amplification (TSA) Kit (Perkinelmer). TSA bindet dabei nach Aktivierung durch die POD in unmittelbarer räumlicher Nähe an Tyrosin-Gruppen von Polypeptiden. Daraus resultiert der Vorteil, dass das Analytsignal auch nach Aufbrechen der Reaktionsräume oder bei der Verwendung teilweise abgeschlossener Reaktionsräume oder auch semipermeabel abgeschlossener Reaktionsräume erhalten bleibt (siehe z.B. Fig. 3). Semipermeable Reaktionsräume werden z.B. dadurch erreicht, dass bevorzugt 0,5-2 % Gelatinelösung zur Stabilisierung der Reaktionsräume eingesetzt werden. Dazu werden die Mikropartikel nach Entfernung der Reaktionslösung mit 30-50 °C warmer Gelatinelösung überschichtet, die nach Abkühlung, die Reaktionsräume umschließt. Durch den semipermeablen Abschluß können bevorzugt Nährstoffe aber auch Enzymsubstrate für die Nachweisreaktion in die Reaktionsräume diffundieren, wenn die Gelatine ihrerseits mit diesen Stoffen überschichtet wird. Alternativ können semipermeable Reaktionsräume dadurch gestaltet werden, dass ein semipermeabler Träger eingesetzt wird, wie er dem Fachmann für Doppelkammer-Zellkulturgefäße bekannt ist. Dadurch wird es möglich, die Nachweisreaktion z.B. für sensitive Immunoassays erst nach dem Abschluss von Reaktionsräumen zu starten, was bevorzugt dazu dient, Kontaminationen benachbarter Reaktionsräume durch das von der Signalverstärkungsreaktion vor Abschluß/Stabilisierung der Reaktionsräume bereits umgewandelte Substrat zu verhindern, und dadurch unspezifische Hintergrundsignale zu vermeiden.

Wenn das Signalverstärkungssystem die Aktivität eines Enzyms, z.B. einer Peroxidase, aufweist, so ist es ebenfalls möglich Substrate in der Reaktionslösung einzusetzen, die ein Reaktionsprodukt ergeben, welches durch seine Lumineszenz optisch messbar ist. Dazu zählen Chemilumineszenzsubstrate, wie z.B. Luminol oder Lumigen ECL Ultra (Diarect).

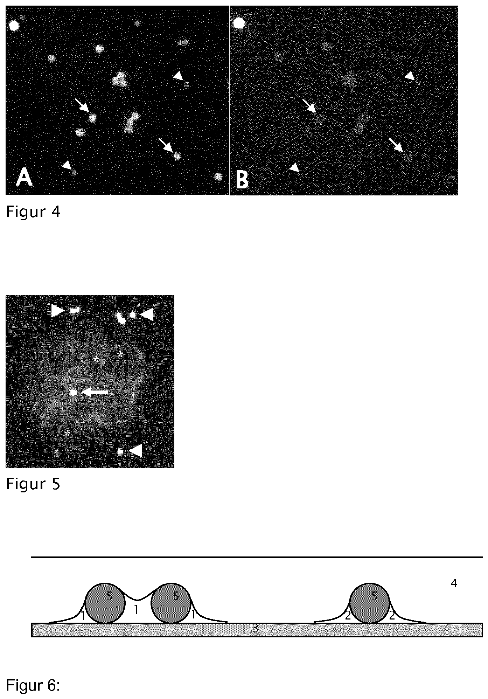

Wenn das Signalverstärkungssystem die Aktivität eines Enzyms, z.B. einer Phosphatase, aufweist, so ist es ebenfalls möglich Substrate in der Reaktionslösung einzusetzen, die ein Reaktionsprodukt ergeben, welches durch seine Lumineszenz oder Fluoreszenz optisch messbar ist. Dazu zählen Chemilumineszenzsubstrate, wie z.B. CSPD (Thermo Scientific) oder Fluoreszenzsubstrate wie z.B. DDAO-Phosphat oder 4-MUP (siehe z.B. Fig. 4).

Wenn der nachzuweisende Analyt in der Signalverstärkungsreaktion durch ein Enzym vervielfältigt werden kann, dann können als Substrat bevorzugt TaqMan-, Hybprobe-, Scorpion-, LoopTag-Sonden, Molecular-Beacons oder Amplifluor-Primer verwendet werden, die gemeinsam mit entsprechenden Primern, Reaktionspuffern und Nukleotidtriphosphaten in der Reaktionslösung für z.B. eine real-time-PCR im Reaktionsraum verwendet werden. Hierbei entsteht mit fortschreitender Amplifikation in Abhängigkeit der gebildeten Menge an Reaktionsprodukt ein Fluoreszenzsignal.

Vorzugsweise umfasst die Reaktionslösung neben dem Substrat weitere Substanzen. Bei diesen Substanzen kann es sich z.B. um Farbstoffe handeln, bevorzugt um Fluoreszenzfarbstoffe. Besonders bevorzugt handelt es sich bei diesem Fluoreszenzfarbstoffen um solche, deren Anregung und/oder Emission durch das aus der Signalverstärkungsreaktion resultierende Reaktionsprodukt gelöscht werden. Die Fluoreszenzfarbstoffe können sich ebenfalls in den Mikropartikeln, auf der Mikropartikeloberfläche befinden. Ebenso ist es möglich die Fluoreszenzfarbstoffe vor der Zugabe der Reaktionslösung in der gewünschten Dichte/Intensität an die Oberfläche des Trägers oder an die Beschichtung auf der Oberfläche des Trägers, wie z.B. eine Polylysin-Beschichtung, zu binden. Wird beispielsweise TMB als Substrat in der Reaktionslösung verwendet, so kann die Reaktionslösung bzw. die Mikropartikel oder der Träger Fluoreszenzfarbstoffe umfassen, die zwischen 600-700nm angeregt werden und emittieren wie z.B. Atto 590, Atto 647N, Cy5 und deren Derivate, welche durch das Reaktionsprodukt gelöscht werden.

Die räumliche Trennung der Reaktionsräume einzelner Mikropartikel wird durch die Verwendung detergenzfreier Reaktionslösungen weiter verbessert.

Träger

Der im erfindungsgemäßen Verfahren verwendete Träger bietet eine Oberfläche, auf der die räumlich getrennten Reaktionsräume eingerichtet werden können. Der Träger kann als Objektträger, Blister, Mikrotiterplatte, Kapillare oder Tube ausgebildet sein. Vorzugsweise handelt es sich bei dem Träger um einen planaren Träger oder um einen im Wesentlichen planaren Träger. Besonders bevorzugt handelt es sich bei dem planaren Träger um eine Kavität einer Mikrotiterplatte.

Das erfindungsgemäße Verfahren kann in Form eines Mikroarrays z.B. mittels eines Objektträgers oder einer Mikrotiterplatte durchgeführt werden.

Der Träger kann aus verschiedenen Materialien bzw. Werkstoffen bestehen wie beispielsweise Metalle, Keramik, Glas und transparente oder nicht transparente Kunststoffe wie z.B. Polycarbonat, Polystyrol, Polypropylen, Siloxan, Polycarbonat umfassen. Bevorzugte Träger sind Nylonmembranen, Siloxan, Epoxidglas und Borfluoratglas.

Die Trägeroberfläche kann in unmodifizierter Form oder in modifizierter Form vorliegen. Die Trägeroberfläche kann durch Modifikation mit funktionellen Gruppen funktionalisiert werden.

Bevorzugte funktionelle Gruppen sind z.B. Carboxy-, Azid, Alkin, Sulfohydryl-, Epoxy-, Amino-, Hydroxy-, Sulfonsäure-, Pegylyl-, Acryl-, oder Phosphat-Gruppen.

Die räumliche Trennung der Reaktionsräume einzelner Mikropartikel wird durch den Einsatz von Trägern mit hydrophoben Oberflächen weiter verbessert.

Die im erfindungsgemäßen Verfahren verwendeten Träger und/oder Mikropartikel weisen vorzugsweise auf ihrer Oberfläche Binder auf, die spezifisch sind für die nachzuweisenden Analyten. Der Begriff "Binder" wird im Folgenden noch näher erläutert.

Die Binder an den Träger können in zweidimensionaler Anordnung bevorzugt durch Piezodrucktechnik, Sprühen oder Nadeldruck in diskreten, d.h. voneinander getrennten Spots auf die Oberfläche des Trägers gedruckt werden. Bevorzugt kommt es durch die Kopplung der Binder zu einer Erhöhung der Hydrophilie im Bereich der Spots im Vergleich zur umgebenden, nicht bedruckten Oberfläche des Trägers. Diese Unterschiede in der Hydrophilie erleichtern die Ausbildung vollständig voneinander getrennter Reaktionsräume. Die Spots können einen Durchmesser von $0,5\mu m$ bis $500\mu m$ haben, bevorzugt von $1\mu m$ bis $400\mu m$, besonders bevorzugt von $2\mu m$ bis $200\mu m$, ganz besonders bevorzugt von $5\mu m$ bis $50\mu m$. Der Träger kann ebenfalls homogen mit Bindern beschichtet sein. Ein Vorteil einer homogenen Beschichtung mit Bindern ist, dass eine besonders große Menge von Analytmolekülen aus der Probe gebunden werden kann.

### Reaktionsraumbegrenzung: räumlich getrennte Reaktionsräume

Vor der Durchführung der Signalverstärkungsreaktion in Schritt b) wird auf dem Träger eine Mehrzahl räumlich getrennter Reaktionsräume eingerichtet. Dies geschieht dadurch, dass zunächst vor, während oder nach dem Aufbringen der Probe und der Reaktionslösung Mikropartikel auf dem Träger immobilisiert werden, so dass die Mikropartikel von der Reaktionslösung überschichtet sind (Schritt i). Vorzugsweise wird zunächst die Probe auf den Träger aufgebracht, anschließend die Mikropartikel auf dem Träger immobilisiert und dann die Reaktionslösung auf den Träger aufgebracht. Alternativ werden zunächst die Mikropartikel auf dem Träger immobilisiert und dann die Probe vor der Reaktionslösung auf den Träger aufgebracht. Als weitere Alternative wird die Probe mit den Mikropartikeln gleichzeitig auf den Träger aufgebracht und die Mikropartikel immobilisiert, wobei die Probe und die Mikropartikel z.B. zuvor miteinander gemischt werden, und anschließend die Reaktionslösung auf den Träger aufgebracht wird. Als weitere Alternative werden die Mikropartikel gleichzeitig mit der Probe und der Reaktionslösung auf dem Träger aufgebracht und dort immobilisiert, wobei beispielsweise zunächst die Probe mit der Reaktionslösung vermischt werden kann, bevor die Mikropartikel hinzugefügt werden. Besonders bevorzugt werden erst die Mikropartikel auf den Träger immobilisiert und danach gleichzeitig die Reaktionslösung im Gemisch mit der Probe auf den Träger aufgebracht.

Sofern die Probe vor der Reaktionslösung auf den Träger aufgebracht wird, können optional vor dem Aufbringen der Reaktionslösung überschüssige Probenbestandteile aus dem Reaktionsansatz entfernt werden, um z.B. eine weitest gehende Entfernung von ungebundenen bzw. unspezifisch gebundenen Analyten zu gewährleisten. Dies erfolgt beispielsweise durch Dekantieren oder Aspiration und kann zusätzlich durch weitere Wasch- oder Reinigungsschritte intensiviert werden. Wurden Mikropartikel verwendet, die permanent auf dem Träger immobilisiert wurden, so kann die Entfernung der überschüssigen Probenbestandteile mittels Zentrifugation erfolgen. Bei Magnetpartikeln, die nicht permanent auf dem Träger immobilisiert wurden kann außerdem ein Magnetfeld angelegt werden, so dass sichergestellt ist, dass bei Entfernung der überflüssigen Probenbestandteile nicht auch die Magnetpartikel mit entfernt werden.

Nach der Immobilisierung der Mikropartikel und ggf. von Bindern als gedruckte Spots auf dem Träger wird die Reaktionslösung entfernt, so dass benachbarte, sich nicht berührende Mikropartikel und ggf. Spots nicht mehr von einer zusammenhängenden Schicht Reaktionslösung überdeckt sind (Schritt ii). Die Entfernung der Reaktionslösung kann auch dadurch erfolgen, dass diese durch andere poröse oder nicht poröse bevorzugt nicht fluoreszenkodierte Mikropartikel wie z.B. Sepharose oder Nanocellulose verdrängt wird, die die Diffusion zwischen zwei Reaktionsräumen benachbarter Mikropartikel soweit herabsetzt, dass kein störender Austausch von reagierenden Bestandteilen zwischen diesen Reaktionsräumen stattfinden kann (siehe z.B. Fig. 5).

Als Medium für die Abgrenzung von Reaktionsräumen können auch aushärtende Harze fungieren. Zum Beispiel können in leicht flüchtigen Lösungsmitteln lösliche Kunstharze Verwendung finden, welche häufig in der Elektronenmikroskopie zum Bespannen (Befilmen) der Objektträger eingesetzt werden, wie Polyvinylformale oder Polyvinylbutyrale, welche unter den Handelsnamen Formvar oder auch Vinylec, Butvar und Pioloform kommerziell erhältlich sind.

Schritt i): Überschichtung der immobilisierten Mikropartikel mit Reaktionslösung

Die Überschichtung der Mikropartikel mit Reaktionslösung erfolgt durch deren Immobilisierung auf dem Träger und zwar vor, während oder nach dem Aufbringen der Probe und der Reaktionslösung auf den Träger.

Die Überschichtung erfolgt bevorzugt derart, dass eine im Wesentlichen gleichmäßige Benetzung aller auf dem Träger immobilisierten Mikropartikel stattfindet. Vorzugsweise wird die Reaktionslösung für ≥1 Sekunde auf der Oberfläche des Trägers belassen, bevor sie in Schritt ii) vom Träger entfernt wird. Bevorzugt wird die Reaktionslösung 1 Sekunde bis 5 Minuten, besonders bevorzugt 1 Sekunde bis 10 Minuten, ganz besonders bevorzugt 1 Sekunde bis 1 Minute auf der Oberfläche des Trägers belassen.

Mikropartikel

Unter "Mikropartikel" werden im Sinne der vorliegenden Erfindung Partikelteilchen mit einem mittleren Durchmesser von unter einem 1 Millimeter verstanden. Bevorzugt weisen die Mikropartikel einen mittleren Durchmesser von 0,01 bis 100 $\mu$m auf, besonders bevorzugt von 0,05 bis 50 $\mu$m, ganz besonders bevorzugt von 1 bis 30 $\mu$m, insbesondere bevorzugt von 2 bis 20 $\mu$m. Weisen die Mikropartikel einen mittleren Durchmesser im Nanometerbereich auf, so werden sie auch Nanopartikel genannt.

Die Mikropartikel können unterschiedliche Formen aufweisen, z.B. können sie sphärisch, elliptisch, zylindrisch oder kuboid sein. Die Mikropartikel können regelmäßig oder unregelmäßig geformt sein. Bevorzugt sind die Mikropartikel sphärisch. Die Mikropartikel können dreieckige, kreisrunde oder anders geformte Scheiben sein, wie sie beim Abspanen von einem Stab mit entsprechender Querschnittsfläche entstehen.

Die im erfindungsgemäßen Verfahren verwendeten Mikropartikel können ein oder mehrere Materialien umfassen oder daraus bestehen und dementsprechend homogene oder heterogene Mikropartikel darstellen. Die Mikropartikel bestehen vorzugsweise aus einem Polymermaterial wie z.B. Polystyrol, Polyacrylsäure, Polyacrylnitril, Polyamid, Polyacrylamid, Polyacrolein, Polybutadien, Polycaprolacton, Polycarbonat, Polyester, Polyethylen, Polyethylenterephthalat, Polydimethylsiloxan, Polyisopren, Polyurethan, Polyvinylacetat, Polyvinylchlorid, Palyvinylpyridin, Polyvinylbenzylchlorid, Polyvinyltoluol, Polyvinylidenchlorid, Polydivinylbenzol, Polymethylmethacrylat, Polylactid, Polyglycolid, Poly(lactid-co-glycolid), Polyanhydrid, Polyorthoester, Polysulfon, Polyether oder Kombinationen derselben. Ebenso können die Mikropartikel aus anderen Polymermaterialien bestehen, wie z .B. Kohlehydrate wie Carboxymethylcellulose oder Hydroxyethylcellulose, Agar, Gel, Polypeptide, aber auch andere Materialien eukaryotische und prokaryotische Zellen, Lipide, Metall, Harze, Latex, Kautschuk, Silikon wie Polydimethyldiphenylsiloxan, Glas, Melamin, Silika, Holzkohle, Kaolinit, Bentonit. Besonders bevorzugt bestehen die Mikropartikel aus Melamin, Silika, Polysulfon, Polystyrol und/oder Polyether, ganz besonders bevorzugt aus Polymethylmethacrylat.

Polymere an der Mikropartikeloberfläche können in unmodifizierter Form oder in modifizierter Form vorliegen. Mögliche Funktionalisierungen sind z.B. Carboxy-, Sulfohydryl-, Epoxy-, Amino-, Hydroxy-, Sulfonsäure-, Pegylyl-, Acryl- oder Phosphat-Gruppen. Die Oberflächenpolymere können in Abhängigkeit von Umgebungsbedingungen (z.B. pH, Temperatur) ihre Eigenschaften ändern und dadurch beispielsweise fluoreszieren oder reaktiv werden. Die Funktionalisierung der Oberfläche der Mikropartikel kann z.B. auch durch hydrogelartige Matrices erreicht werden, wie sie durch Polyacrylsäure, poly(ethyleneglycol)methacrylate (PEGMA), Dextran, Dextransulfat oder Polyethyleneglycol (PEG) gebildet werden. Derartige Funktionalisierungen stellen sicher, dass die Spezifität der Binder nicht verringert wird und Bindung von unspezifischen Analyten minimiert wird.

Die Mikropartikel können in Form von Mikrokompositen vorliegen. Mikrokomposite umfassen zwei oder mehr verschiedene Polymermaterialien, die miteinander vermischt vorliegen können und so Matrixpartikel mit weitgehend homogener Verteilung der Polymermaterialien über den Partikelquerschnitt darstellen. Die Mikrokomposite können auch in Form von Mikrokapseln vorliegen, welche einen Kern als innere Schicht und ein oder mehrere weitere Schichten aufweisen. Bei Mikrokapseln liegen die verschiedenen Polymermaterialien im Wesentlichen in unterschiedlichen Schichten vor. Der Kern von Mikrokapseln kann in fester, flüssiger oder gasförmiger Form (Hohlkugeln) vorliegen. Derartige polymerbasierte Mikrokomposite in Form von Mikrokapseln oder Matrixpartikeln sind im Stand der Technik bekannt (Ch. A. Finchand R. Bodmeier: "Microencapsulation" in Ullmann's Encyclopedia of Industrial Chemistry, 6. Ed. 2001, electronic release).

Vorzugsweise liegen die Mikropartikel in Form von Mikrokapseln vor. Bevorzugt umfassen oder bestehen sie aus einem Kern als innere Schicht und zwei weiteren Schichten, nämlich einer mittleren und einer äußeren Schicht. Bevorzugt besteht zumindest die äußere Schicht aus einem temperaturbeständigen Material wie z.B. Melamin. Besonders bevorzugt bestehen die äußere, die mittlere und die innere Schicht der Mikropartikel aus einem temperaturbeständigen Material, so dass diese Mikropartikel geeignet sind im erfindungsgemäßen Verfahren eingesetzt zu werden, bei dem einzelne oder mehrere Schritte bei erhöhten Temperaturen durchgeführt werden. Die Mikropartikel können mit dem Ziel der Oberflächenvergrößerung porös oder teilweise porös sein.

Binder

Wie bereits ausgeführt können sowohl die Träger und/oder die im erfindungsgemäßen Verfahren verwendeten Mikropartikel vorzugsweise auf ihrer Oberfläche Binder aufweisen, die spezifisch sind für die nachzuweisenden Analyten. Der Begriff "spezifisch" oder "spezifische Bindung" bedeutet, dass der Binder unter den gewählten Bedingungen vorzugsweise selektiv an den nachzuweisenden Analyten binden kann.

Dem Fachmann sind geeignete Binder als auch Verfahren zu deren Herstellung bekannt. Bei den Bindern kann es sich um natürliche oder synthetische Substanzen handeln. Die Binder können auf (Poly)Peptiden, Poly- oder Oligonukleinsäuren, Polysacchariden, Lipiden oder ähnlichem basieren. Bevorzugt handelt es sich bei dem Binder um ein Polypeptid wie z.B. ein Zelladhäsionsmolekül aus der Gruppe der Integrine, Cadherine, Selektine oder um Mitglieder der Immunglobulin (Ig) Superfamilie. Bevorzugt handelt es sich bei dem Polypeptid um einen Antikörper, wobei sowohl ein polyklonaler und monoklonaler Antikörper mit klassischer Antikörperstruktur als auch davon abgeleitete Derivate oder Fragmente umfasst sein können. Solche Fragmente sind bekannt unter der Bezeichnung Fab, F(ab)2, dsFv-Fragmente, scFV-Fragmente und single chain-Antikörper. Vorzugsweise handelt es sich bei dem Binder um einen Antikörper.

Ebenfalls bevorzugt kann es sich bei dem Binder um eine Nukleinsäure wie z.B. DNA oder RNA handeln. Bevorzugt handelt es sich um einzelsträngige Oligonukleotide, besonders bevorzugt um Aptamere. Ebenfalls sind synthetische Nukleinsäureanaloga als Binder möglich.

Die Binder können Modifikationen wie beispielsweise eine Fluoreszenzmarkierung aufweisen. Dem Fachmann sind sowohl geeignete Markierungen als auch Methoden bekannt mit denen Bindungsmoleküle modifiziert werden können.

Die Kopplung der Binder an die Mikropartikel kann kovalent oder nicht kovalent erfolgen. Eine kovalente Kopplung kann z.B. durch 1-Ethyl-3-(3-Dimethylaminopropyl)-Carbodiimid an funktionelle Carboxygruppen auf der Oberfläche der Mikropartikel erfolgen. Eine nicht kovalente Kopplung kann z.B. durch hydrophobe oder ionische Wechselwirkung der Binder mit den Mikropartikeln erfolgen, wobei die Mikropartikel oberflächenfunktionalisiert sein können oder nicht.

Die Binder können auch biotinyliert sein und somit an die Oberfläche mit Avidin beschichteter Mikropartikel binden. Ebenso ist es möglich, kovalent oder nicht kovalent gekoppelte Fängeroligonukleotide an die Mikropartikeloberfläche zu binden, an die entsprechende Binder reversibel hybridisieren, die komplementäre Sequenzabschnitte mit dem Fängeroligonukleotid aufweisen bzw. an die entsprechende komplementäre Oligonukleotide gebunden sind.

Mikropartikelpopulationen

Vorzugsweise wird im erfindungsgemäßen Verfahren mehr als eine Mikropartikelpopulation verwendet um mehr als einen Analyten in einer Probe nachzuweisen (Multiplex-Nachweis). Dabei unterscheiden sich die verschiedenen Mikropartikelpopulationen in zumindest einem Merkmal voneinander.

Vorzugsweise wird im erfindungsgemäßen Verfahren mehr als eine Mikropartikelpopulation verwendet, wobei sich die verschiedenen Mikropartikelpopulationen mindestens darin unterscheiden, dass sie Binder tragen, die sich in ihrer Bindungsspezifität unterscheiden. Bevorzugt weisen die unterschiedlichen Mikropartikelpopulationen Binder auf, die für unterschiedliche Analyten spezifisch sind. Das bedeutet also, wenn z.B. eine Mikropartikelpopulation Binder mit einer Spezifität für ein erstes Protein aufweist, dann kann eine andere Mikropartikelpopulation jede andere Spezifität aufweisen außer jener für das erste Protein. Die Mikropartikelpopulationen können sich neben ihrer unterschiedlichen Bindungsspezifitäten zusätzlich noch aufgrund anderer Merkmale voneinander unterscheiden.

Bevorzugt sind die Mikropartikel oder Mikropartikelpopulationen fluoreszenz- und/oder größenkodiert, so dass unterschiedliche Mikropartikel oder Mikropartikelpopulationen anhand ihrer Fluoreszenz und/oder Größe voneinander unterscheidbar sind. Sie können aber auch Barcodes in Form spezifischer Muster, wie Löcher im Falle scheibenförmiger Mikropartikel besitzen. Diese Scheiben aber auch Stäbchen können definiert angeordnete Löcher haben, falls der Stab von dem sie abgespant werden, im inneren hohle Kanäle verschiedener Form oder eine gleichbleibende Positionierung über die gesamte Länge des Stabes aufweist. Die dadurch entstehenden Lochmuster, die für jeden Stab spezifisch sind, können für die Kodierung unterschiedlicher Populationen, die von unterschiedlichen Stäben geschnitten werden, genutzt werden. Die Scheiben können auch z.B. im Spritzgussverfahren hergestellt werden. Dabei können die Scheiben an einer oder beiden Querschnittsflächen ein Eindellung oder eine kappenartige Verbiegung erfahren, deren Raum zur Größe des Reaktionsraumes beiträgt. Die Scheiben können auch ringförmig gestaltet sein, indem sie in der Mitte ein Loch aufweisen, welches wesentlich die Größe und Anordnung des Reaktionsraumes mitbestimmt. Der Durchmesser der Scheiben beträgt bevorzugt 5 $\mu$m bis 1 mm und besonders bevorzugt 10 $\mu$m bis 30 $\mu$m. Die darin enthaltenen Löcher können einen Durchmesser von 1 $\mu$m bis 0,9 mm und bevorzugt einen Durchmesser von 1 bis 25 $\mu$m aufweisen. Die Höhe der Scheiben beträgt 0,5 $\mu$m bis 1 mm und bevorzugt 1 $\mu$m bis 100 $\mu$m. Scheibenförmige Mikropartikel können gleichermaßen fluoreszenzkodiert werden wie sphärische Mikropartikel und dadurch bevorzugt für Multiplexnachweise genutzt werden. Der Vorteil der Verwendung von scheibenförmigen Mikropartikeln liegt darin, dass der Reaktionsraum leicht ein größeres Volumen an Reaktions- bzw. Probenlösung aufnehmen kann, was die Sensitivität der Tests als auch den dynamischen Messbereich erhöht. Bevorzugt können sich scheibenförmige Mikropartikel dadurch auszeichnen, dass die Scheibenhöhe größer ist als der Scheibendurchmesser und 1-1000$\mu$m betragen kann, bevorzugt 10-100 mm, so dass ein Hohlzylinder entsteht.

Die Mikropartikel können fluoreszierende, magnetische, paramagnetische und/oder oberflächenfunktionalisierte Eigenschaften aufweisen.

Fluoreszierende Eigenschaften der Mikropartikel

Die Mikropartikel können fluoreszierende Eigenschaften aufweisen. Die Mikropartikel können zum einen eine Eigenfluoreszenz aufweisen. Eine Eigenfluoreszenz der Mikropartikel kann beispielsweise dadurch erzeugt werden, dass die Mikropartikel das Mineral Fluorit in einer oder mehrerer ihrer Schichten beinhalten. Alternativ oder zusätzlich zum Vorhandensein von eigenfluoreszierenden Materialien können die Mikropartikel noch eine Fluoreszenzkodierung aufweisen.

Fluoreszenzkodierte Mikropartikel sind dem Fachmann bekannt und z.B. in DE 699 07 630 T2 und DE 10054382 A1 beschrieben. Unter dem Begriff "Fluoreszenzkodierung" versteht man im Kontext der vorliegenden Erfindung, dass die Mikropartikel Fluoreszenzfarbstoffe aufweisen, wobei diese Fluoreszenzfarbstoffe entweder in einer oder mehreren Schichten der Mikropartikel eingelagert sind oder die Fluoreszenzfarbstoffe direkt oder indirekt an die Oberfläche der Mikropartikel gekoppelt wurden. Fluoreszenzfarbstoffe, die an der Oberfläche der Mikropartikel gebunden sind, können somit die Funktion einer Farbkodierung als auch die Funktion der Indikation der Bindungs- oder Reaktionskinetik haben, wenn z.B. deren Anregung und/oder Emission durch das Reaktionsprodukt der Signalverstärkungsreaktion gelöscht werden. Dem Fachmann sind geeignete Fluoreszenzfarbstoffe bekannt. Fluoreszenzfarbstoffe oder Fluorophore sind alle gasförmigen, flüssigen oder festen anorganischen und/oder organischen Verbindungen, die nach Anregung die absorbierte Energie in Form von Strahlung von gleicher, längerer oder kürzerer Wellenlänge wieder abgeben. Die Fluoreszenzfarbstoffe können detektierbare Lumineszenzsignale oder Fluoreszenz oder Phosphoreszenz emittieren.

Als Fluoreszenzfarbstoffe können beispielsweise eingesetzt werden: Dansylchlorid, Fluoresceinisothiocyanat, 7-Chlor-4-nitrobenzoxadiazol, Pyrenbutyrylessigsäure-anhydrid, N-Iodoacetyl-N'-(5-sulfonsäure-1-naphthyl)-ethylendiamin, 1-Anilinonaphthalen-8-sulfonat, 2-Toluidinonaphthalen-6-sulfonat, 7-(p-Methoxybenzylamino)4-nitro-benz-2-oxa-1,3-diazol, Formycin, 2-Aminopurinribonukleosid, Ethenoadenosin, Benzoadenosin, $\alpha$- und $\beta$-Parinarsäure, $\Delta$9,11,13,15 Octaecatetraensäure und/oder Cadmiumselenit-Kristalle einer oder unterschiedlicher Größen etc. Als Fluoreszenzfarbstoffe können ebenfalls z.B. Übergangsmetallkomplexe eingesetzt werden, die folgende Substanzen enthalten: Ruthenium (II), Rhenium (I) oder Osmium und Iridium als Zentralatom und Diimin-Liganden; phosphoreszierende Prophyrine mit Platin, Palladium, Lutetium oder Zinn als Zentralatom; phosphoreszierende Komplexe der Seltenerden wie Europium, Dysprosium oder Terbium; phosphoreszierende Kristalle wie Rubin, Cr-YAG, Alexandrit oder phosphoreszierende Mischoxide wie Magnesiumfluorogermanat bzw. Cadmiumselenit-Kristalle, Fluorescein, Aminofluorescein, Aminomethylcoumarin, Coumarin 6, Rhodamin, Rhodamin 6G, Rhodamin B, Tetramethylrhodamin, Ethidiumbromid, Acridinorange und/oder Quantum Dots.

Als Fluoreszenzfarbstoffe können z.B. folgende Stoffe in Kombination eingesetzt werden: Ruthenium(II)-(tris-4,7-diphenyl-1,10-phenanthrolin)/HPTS, Ruthenium(II)-(tris-4,7-diphenyl-1,10-phenanthrolin)/Fluorescein, Ruthenium(II)-(tris-4,7-diphenyl-1,10-phenanthro-lin)/Rhodamin B, Ruthenium(II)-(tris-4,7-diphenyl-1,10-phenanthrolin)/Rhodamin B-octadecylester, Ruthenium(II)-(tris-4,7-diphenyl-1,10-phenanthrolin)/Hexadecyl-Acridinorange, Europium(III)-tris-theonyl-trifluormethylacetonat/Hydroxymethylcoumarin, Platin(II)-tetraphenylporphyrin/Rhodamin B-octadecylester, Platin(II)-tetraphenylporphyrin/Rhodamin B, Platin(II)-tetraphenylporphyrin/Naphtofluorescein, Platin(II)-tetraphenylporphyrin/Sulforhodamin 101, Platin(II)-octaethylporphyrin/Eosin, Platin(II)-octaethylporphyrin/Thionin, Platin(II)-octaethylketoporphyrin/Nilblau, Cr(III)-YAG/Nilblau,Cr(III)-YAG/Naphtofluorescein, Aminocoumarin/Aminofluorescein, Aminocoumarin/Rhodamin 6G, Aminocoumarin/Tetramethylrhodamin, Aminocoumarin/Acridinorange, Aminofluorescein/Rhodamin 6G, Aminocumarin/Atto647N, Aminofluorescein/Tetramethylrhodamin , Aminofluorescein/Ethidiumbromid und DAPI/Rhodamin. Es können aber auch Kombinationen mit drei Fluoreszenzfarbstoffen zur Kodierung der Mikropartikel zum Einsatz kommen wie z.B. Aminocumarin, Rhodamin und Atto 647N, DAPI/Aminocumarin/Rhodamin, Aminocumarin/Rhodamin/Atto647N oder Aminocumarin, Fluorescein und Rhodamin.

Vorzugsweise liegen die Mikropartikel in Form von Mikrokapseln vor. Bevorzugt umfassen oder bestehen sie aus einem Kern (innere Schicht) und zwei weiteren Schichten, nämlich einer mittleren und einer äußeren Schicht. Vorzugsweise unterscheiden sich die Schichten fluoreszenzoptisch.

Es besteht auch die Möglichkeit einer Strukturbildung innerhalb der Mikropartikel, wobei weitere fluoreszierende und/oder fluoreszenzkodierte Mikropartikeln in eine oder mehrere Schichten der Mikropartikel einpolymerisiert werden. Dadurch können in den Mikropartikeln gezielt Muster erzeugt werden wie z.B. grob oder feingranuliert oder die Zahl der Granula pro Schicht, die für die Fluoreszenzkodierung zahlreicherer Populationen von Mikropartikeln genutzt werden können.

Werden mehrere unterschiedliche Fluoreszenzfarbstoffe zur Kodierung von mehreren verschiedenen Mikropartikelpopulationen verwendet, so werden sie vorzugsweise derart ausgewählt, dass die unterschiedlichen Fluoreszenzfarbstoffe im Wesentlichen verschiedene Emissionsspektren besitzen. Vorzugsweise weisen die unterschiedlichen Fluoreszenzfarbstoffe jeweilige Emissionsmaxima auf, welche um mehr als 10nm voneinander getrennt sind, bevorzugt mehr als 25nm, besonders bevorzugt um mehr als 50nm. Die Farbstoffe können ausgewählt werden, um Emissionsbanden aufzuweisen, welche zu im Handel erhältlichen Filtern oder Multibandfiltern passen, oder zum Nachweis von Mehrfachfluorophoren mit verschiedenen Anregungs- und Emissionsbanden.

Die an der Oberfläche der Mikropartikel gemessene Fluoreszenz wird als "Oberflächenfluoreszenz" bezeichnet. Die Oberflächenfluoreszenz entsteht entweder durch Fluoreszenzfarbstoffe, die in der äußersten Schicht der Mikropartikel eingelagert sind oder, wenn sich Binder auf der Mikropartikeloberfläche befinden, die selbst fluoreszenzmarkiert sind, durch die Fluoreszenz dieser Binder. Die Oberflächenfluoreszenz kann aber auch durch Fluoreszenzmarkierung der für die Immobilisierung der Binder nötigen Funktionalisierungen erfolgen. Solche Funktionalisierungen können z.B. hydrogelartige Matrices sein, wie sie durch Polyacrylsäure, Poly(ethylenglycol)methacrylate (PEGMA) oder Polyethyleneglycol (PEG) gebildet werden.

In einer bevorzugten Variante werden für die Mikropartikelkodierung ein oder mehrere Farbstoffe der Klassen Cumarine, Rhodamine oder Nilblauderivate in die Mikropartikel einpolymerisiert und ein Atto 647N- oder Cy5-Derivat als Oberflächenfluoreszenz.

Wird mit Cumarin, Fluorescein und Rhodamin kodiert, können bevorzugt Cy5 und Fluoreszenzfarbstoffe höherer Emmissionswellenlängen für die Oberflächenfluoreszenz wie z.B. Atto647N genutzt werden. Es ist aber auch möglich, die Oberflächenfluoreszenz über ein FRET-Paar zu realisieren wie z.B. Fluorescein und Rhodamin. Dann stehen für die Kodierung Cumarin- und Cyanfarbstoffe und deren Derivate etc. zur Verfügung. Die Kodierungsmöglichkeiten erhöhen sich dadurch enorm, dass ein und derselbe Farbstoff in mehreren Schichten der Mikropartikel in unterschiedlichen Konzentrationen eingesetzt werden kann. Dadurch resultiert für jede Schicht und zwischen verschiedenen Schichten unterschiedlicher Fluoreszenzintensität eine Vielzahl von Ratio-Bildungsmöglichkeiten, die für die Kodierung/Dekodierung der Mikropartikelpopulationen genutzt werden können.

Magnetische und/oder paramagnetische Eigenschaften der Mikropartikel

Die Mikropartikel können magnetische und/oder paramagnetische Eigenschaften aufweisen. Die Mikropartikel können in einer oder in mehreren ihrer Schichten einen Magneten oder ein Material enthalten, welches auf einen Magneten reagiert. Derartige Mikropartikel werden vorliegend als Magnetpartikel bezeichnet. Das Material, das auf einen Magneten reagiert, kann superparamagnetische, paramagnetische oder ferromagnetische Eigenschaften aufweisen. Vorzugsweise handelt es sich bei dem auf einen Magneten reagierende Material um ein Metall, bevorzugt um ein Metalloxid. Durch den Magneten bzw. das auf den Magneten reagierende Material innerhalb der Schichten der Mikropartikel ist eine sichere Immobilisierung und/oder Transport der Mikropartikel im Reaktionsraum während der Messung bzw. während der Testdurchführung gewährleistet.

Oberflächenfunktionalisierte Eigenschaften der Mikropartikel

Die Mikropartikel können auf ihrer Oberfläche funktionelle Gruppen aufweisen wodurch die Mikropartikel oberflächenfunktionalisierte Eigenschaften erhalten. Bevorzugte funktionelle Gruppen sind z.B. Carboxylate, Ester, Alkohole, Carbamide, Aldehyde, Amine, Schwefeloxide, Mercaptogruppen, Stickoxide, Epoxide, Acrylate oder Halogenide. Mithilfe von funktionellen Gruppen an der Oberfläche der Mikropartikel kann beispielsweise die Bindung des Analyten an die Mikropartikel erleichtert oder verbessert werden. Auch kann durch das Vorhandensein von funktionellen Gruppen die Bindung zwischen den Mikropartikeln gehemmt, erleichtert oder verbessert werden.

Vorzugsweise weisen die Mikropartikel und der Träger unterschiedliche Oberflächenfunktionalisierungen auf. Bevorzugt umfassen die Mikropartikel und Träger funktionelle Gruppen mit unterschiedlichen Eigenschaften, vorzugsweise entgegengesetzte Affinitäten bezogen auf denselben Stoff oder dieselbe Verbindung, wie z.B. auf Wasser. Derartige unterschiedliche Oberflächenfunktionalisierungen der Mikropartikel und des Trägers wirken sich vorteilhaft auf die Begrenzung der Reaktionsräume aus und bewirken, dass benachbarte Reaktionsräume besser voneinander getrennt werden. Beispielsweise können Mikropartikel mit hydrophilen funktionellen Gruppen auf Trägern mit hydrohoben funktionellen Gruppen immobilisiert werden.

Verfahren zur Herstellung von Mikropartikeln mittels reaktiver und nicht-reaktiver Mikropartikelbildungsprozesse sind im Stand der Technik bekannt. Bei der reaktiven Mikropartikelbildung erfolgt die Bildung der Wand oder der Matrix parallel zu einem Polymerisations-, Polykondensations- oder Polyadditionsprozess. Bei den nicht-reaktiven Verfahren werden filmbildende Polymere direkt eingesetzt, die auf thermodynamische Weise zur Phasenseparation und zur Mikropartikelbildung gebracht werden. In Verfahren zur Verkapselung fester oder flüssiger Kernmaterialien werden z.B. Melamin-Formaldehyd-Harze eingesetzt. Insbesondere Melamin-Formaldehyd-Harze sind vielfältig und problemlos einsetzbar und können zur Mikropartikelbildung aus wässriger Phase appliziert werden. Die Mikrokapselgröße kann in Abhängigkeit von den Reaktionsbedingungen, z.B. Emulgatorzusatz oder Dispergiermethode eingestellt werden. Eine weitere Möglichkeit ist die hydrothermale Vernetzung von Hydroxymethyl-Melamin.

Immobilisierung der Mikropartikel

Die Mikropartikel können auf einer Oberfläche des Trägers immobilisiert werden. Die Immobilisierung der Mikropartikel auf einer Trägeroberfläche vereinfacht die nachfolgenden Schritte des erfindungsgemäßen Verfahrens, insbesondere wenn diese automatisiert und mithilfe von Standardlaborgeräten durchgeführt werden. Die Immobilisierung verhindert, dass die Mikropartikel bei nachfolgenden Verfahrensschritten versehentlich aus dem Reaktionsansatz entfernt werden. Die Immobilisierung kann permanent oder nicht permanent sein. Eine permanente Immobilisierung kann z.B. durch kovalente oder nicht kovalente Bindung der Mikropartikel am Träger erfolgen. Eine nicht permanente, also temporäre, Immobilisierung kann z.B. durch Zentrifugation erfolgen oder durch Anlegen eines Magnetfeldes wenn Magnetpartikel verwendet werden.

Scheiben- oder Hohlzylinderförmige Mikropartikel können ungerichtet, d.h. mit der Mantelfläche bzw. der Querschnittsfläche auf dem Träger immobilisiert werden. Scheiben- oder insbesondere hohlzylinderförmige Mikropartikel können bevorzugt gerichtet immobilisiert werden, dass das Material der Querschnittsfläche spezifisch und fest an den Träger binden kann und die Mantelfläche nicht, da die spezifischen Bindungseigenschaften nicht vorhanden bzw. überdeckt oder chemisch verändert wurden. Die Herstellung unterschiedlicher Bindungseigenschaften von Querschnittsfläche und Mantelfläche kann dadurch erreicht werden, dass, bevor die Scheiben oder Hohlzylinder von Hohlstäben geschnitten werden, nur die äußeren Oberflächen des Hohlstabes chemisch modifiziert oder überdeckt werden wie z.B. durch Silikonisierung, Polymerüberzug oder Bedampfung, so dass keine Bindung dieser Fläche an den Träger möglich ist.

Ebenso ist es möglich, die Oberfläche bevorzugt von Scheiben komplett zu modifizieren, so dass eine optimale Bindung an den Träger erfolgen kann. Durch das Anlegen von Schwingungen z.B. durch Ultraschall wird dann erreicht, dass die Scheiben mehrheitlich auf die Querschnittsfläche fallen und dann binden. Sofern eine kovalente Bindung z.B. durch elektromagnetische Strahlung initiiert wird, wird diese Strahlung erst eingesetzt, nachdem die Scheiben mehrheitlich mit ihrer Querschnittsfläche auf dem Träger zu liegen gekommen sind.

Es ist ebenso möglich, die Mantelfläche der Hohlstäbe von denen die Hohlzylinder geschnitten werden in der Weise chemisch zu modifizieren oder mit einem schützenden Überzug zu versehen, so dass sie im Gegenteil zur Querschnittsfläche nach dem Schneiden nicht mehr für die Bindung an den Träger bzw. die Bindung von Bindern modifiziert werden können.

Für das Schneiden von Scheiben und Hohlzylindern ist es möglich Bündel von Hohlstäben in ein schneidbares Medium einzubetten und dieses mit einem Mikrotom quer zur Faserrichtung zu schneiden. Alternativ ist es möglich Mikrofasern mit einem Polymer zu beschichten und die Mikrofaser nach dem Schneiden herauszulösen, so dass die Scheiben und Hohlzylinder zurückbleiben. Bevorzugt unterscheiden sich die physikochmischen Eigenschaften von Mikrofaser und das zur Ummantelung eingesetzte Polymer durch z.B. unterschiedliche Löslichkeit in Lösungsmitteln, der Hitzestabilität oder in Säuren und Basen, so dass die labilere Mikrofaser rausgelöst werden kann. Es ist ebenso möglich, Bündel von Mikrofasern zu assemblieren, diese mit Polymer zu ummanteln, zu schneiden und anschließend die Mikrofasern herauszulösen.

Die Immobilisierung der Mikropartikel auf einer Trägeroberfläche findet vor, während oder nach dem Aufbringen der Probe und der Reaktionslösung statt. Dadurch sind die Mikropartikel von der Reaktionslösung überschichtet. Wird im Anschluss daran die Reaktionslösung entfernt, so sind sich nicht berührende immobilisierte Mikropartikel nicht mehr von einer zusammenhängenden Schicht Reaktionslösung bedeckt. Vorzugsweise erfolgt die Immobilisierung der Mikropartikel auf einer Oberfläche des Trägers bevor die Mikropartikel mit der Probe und der Reaktionslösung in Kontakt gebracht werden.

Schritt ii): Entfernen der Reaktionslösung

Auf einer Trägeroberfläche immobilisierte Mikropartikel sind von einer Reaktionslösung überschichtet. Die Einrichtung einer Mehrzahl räumlich getrennter Reaktionsräume auf einer mit Mikropartikeln besetzten Oberfläche des Trägers erfolgt durch Entfernen der Reaktionslösung, so dass benachbarte, sich nicht berührende Mikropartikel nicht mehr von einer zusammenhängenden Schicht Reaktionslösung überdeckt sind.

Durch das Entfernen der Reaktionslösung sind vorzugsweise benachbarte, sich nicht berührende Mikropartikel mehrheitlich nicht mehr von einer zusammenhängenden Schicht Reaktionslösung überdeckt.

Erfindungsgemäss erfolgt die Entfernung der Reaktionslösung derart, dass die Oberfläche des Trägers lediglich in einem Bereich mit Reaktionslösung bedeckt bleibt, der unmittelbar eine Kontaktstelle zwischen Mikropartikel und Träger umgibt, während Oberflächenbereiche des Trägers, die mindestens den $\geq 0{,}1$-fachen Partikeldurchmesser von einer Kontaktstelle zwischen einem Mikropartikel und dem Träger entfernt sind, nicht mehr von Reaktionslösung bedeckt sind. Vorzugsweise sind Oberflächenbereiche des Trägers, die mindestens den ≥1,0-fachen Partikeldurchmesser, bevorzugt den ≥2-fachen Partikeldurchmesser, besonders bevorzugt ≥5-fachen Partikeldurchmesser von einer Kontaktstelle zwischen einem Mikropartikel und dem Träger entfernt sind, nicht mehr von Reaktionslösung bedeckt. Die Reaktionslösung kann auch soweit entfernt werden, dass sie nur noch im Spalt zwischen Partikel und Gefäßboden verbleibt und der Reaktionsraum somit kleiner als der Partikeldurchmesser sein kann. Dieser mit Reaktionslösung bedeckte Bereich des Trägers stellt den sog. Reaktionsraum dar. Die Mikropartikel wirken also als Nukleationspunkte um die ein Flüssigkeitshof verbleibt, der einen räumlich getrennten Reaktionsraum bildet. Durch Kapillarkräfte an den Grenzflächen zwischen Mikropartikel und Träger werden so die räumlich getrennten Reaktionsräume ausgebildet. Die als Nukleationspunkte verwendeten Mikropartikel für die Einrichtung räumlich getrennter Reaktionsräume können auf ihrer Oberfläche Binder aufweisen oder keine Binder aufweisen. Bei Verwendung poröser Mikropartikel oder ring- bzw. hohlzylinderförmiger Mikropartikel bleibt die Reaktionslösung auch in den Mikropartikeln zurück, so dass der Reaktionsraum auch durch die innere Oberfläche dieser Partikel begrenzt wird.

Vorzugsweise liegen die Mikropartikel im Wesentlichen vereinzelt auf dem Träger vor (siehe z.B. Fig. 6), bevorzugt mit einem durchschnittlichen Abstand, der dem 1 bis 20-fachen Partikeldurchmesser entspricht, besonders bevorzugt dem 2 bis 10-fachen Partikeldurchmesser, ganz besonders bevorzugt dem 3 bis 5-fachen Partikeldurchmesser. Je weiter der Abstand zwischen den einzelnen Mikropartikeln, desto vollständiger ist die laterale Trennung der jeweiligen Reaktionsräume, bis ein kritischer Abstand überschritten wird, der zu einer vollständigen Trennung der Reaktionsräume benachbarter Mikropartikel führt. Vollständige Trennung bedeutet, dass kein Materialaustausch zwischen den Reaktionsräumen stattfindet, der die jeweilige Signalverstärkungsreaktion bzw. den jeweiligen Analytnachweis in anderen Reaktionsräumen störend überlagert oder inhibiert.

Eine permanente oder nicht permanente Immobilisierung der Mikropartikel verhindert, dass die vorzugsweise einzeln liegenden Mikropartikel durch die Oberflächenspannung der sich zu den Mikropartikeln ziehenden Reaktionslösung nach deren Entfernung zu größeren Mikropartikelagglomeraten zusammenfügen.

In den räumlich getrennten Reaktionsraum findet die Signalverstärkungsreaktion (Schritt b) statt. In einem räumlich getrennten Reaktionsraum können ein einziges Mikropartikel, aber auch zwei oder mehr sich berührende Mikropartikel vorliegen.

Vorzugsweise erfolgt die Entfernung der Reaktionslösung mittels Absaugen, Verdunsten oder Zentrifugation oder ggf. in Kombination dieser Verfahrensschritte. Durch die Entfernung der Reaktionslösung entstehen die Reaktionsräume durch Kapillarkräfte an der Grenzschicht zwischen Mikropartikel und Träger. Bevorzugt wird die Reaktionslösung durch Zentrifugation entfernt, wobei die Zentrifugalkräfte schräg oder senkrecht von der Oberfläche des Trägers weg weisen, auf der die Mikropartikel permanent immobilisiert wurden. Dabei muss die einwirkende Zentrifugalkraft auf die Festigkeit der Immobilisierung der Mikropartikel abgestimmt sein, so dass zwar die Reaktionslösung optimal entfernt werden, die Mikropartikel aber auf dem Träger verbleiben.

Vorzugsweise erfolgt das Absaugen mit einer Pipette. Die Entfernung der Reaktionslösung kann auch durch Verdunstung erzielt werden. Ebenso ist es möglich durch eine im Wesentlichen senkrecht zur Oberfläche des Trägers hin wirkende Zentrifugalkraft, die Reaktionsräume stärker auf die Mikropartikel zu fokussieren, da dadurch mehr Reaktionslösung an den Rand des Reaktionsgefäßes und zu den Mikropartikeln gedrängt werden kann. Entsprechend bleibt nur ein kleines Areal des Trägers, welches die Grenzschicht zwischen Mikropartikel und Träger umgibt, mit Reaktionslösung benetzt und zwar mit einer deutlich schärferen Abgrenzung als ohne Zentrifugation. Dieses Vorgehen hat außerdem den Vorteil, dass die Mikropartikel während des Entfernens der Reaktionslösung nicht permanent bzw. mittels des Anlegen eines Magnetfeldes am Träger immobilisiert werden müssen, da sie durch die Zentrifugalkraft gegen den Träger gedrückt und somit nicht permanent immobilisiert werden. Der Konzentrationseffekt der Reaktionslösung nahe der Mikropartikel ist proportional zur Zentrifugalkraft bzw. zur Schrägstellung des Trägers gegen die Zentrifugalkraft, zur Hydrophobie des Trägers und zur Hydrophilie der Mikropartikeloberfläche. Es ist ebenso möglich, die Zentrifugalkraft von der Oberfläche des Trägers, auf der die Mikropartikel immobilisiert wurden, weg oder hin wirken zu lassen. Alternativ ist es möglich die Zentrifugalkraft von der Oberfläche des Trägers, auf der sich die Mikropartikel befinden, im Wechsel weg und hin wirken zu lassen. Dadurch werden besonders effektiv überschüssige Reste der Reaktionsflüssigkeit, die nicht für die Reaktionsräume benötigt werden, insbesondere aus den Kavitäten einer Mikrotestplatte, entfernt.

Im Falle der Zentrifugation weist der Träger ggf. eine Öffnung in der mit Mikropartikeln besetzten Oberfläche auf über die abzentrifugierte Reaktionslösung abgeführt werden kann. Ist der Träger in Form einer Mikrotiterplatte ausgebildet, so können deren Kavitäten vorzugsweise im Randbereich mit einer Bohrung bzw. einem Loch versehen sein. Die Öffnung im Kavitätenboden beträgt bevorzugt 0,1 bis 1,0mm. Durch Anlegen einer Zentrifugalkraft kann die überschüssige Reaktionslösung vollständiger aus der Kavität entfernt werden ohne dass ein zusätzlicher Absaugschritt durchgeführt werden muss. Alternativ reicht es ggf. aus die Flüssigkeit abzusaugen und die verbliebenen Flüssigkeitsreste in die Randbereiche der Kavität der Mikrotestplatte zu zentrifugieren.

Aufgrund der Begrenzung in einzelne Reaktionsräume kann eine ortsaufgelöste Detektion der Signale der Reaktionsprodukte stattfinden. Außerdem kann die für die Detektion einzelner Analyte nötige kritische Konzentration der Reaktionsprodukte der Signalverstärkungsreaktion im einzelnen Reaktionsraum schnell und sicher erreicht werden.

Außerdem kann die Reaktionsraumbegrenzung genutzt werden, um verschiedenartige Reaktionen auf unterschiedliche Reaktionsräume zu begrenzen, um gegenseitige Störeinflüsse der verschiedenartigen Reaktionen wie sie z.B. bei der Multiplex-PCR bekannt sind, zu minimieren. Weiterhin kann die Reaktionsraumbegrenzung genutzt werden, um Analyte im Sinne eines digitalen Assay-Formates auf verschiedene Reaktionsräume statistisch zu verteilen.

Stabilisierung der Reaktionsräume

Gegebenenfalls kann nach Einrichtung der räumlich getrennten Reaktionsräume eine Stabilisierung der getrennten Reaktionsräume stattfinden. Bevorzugt erfolgt diese Stabilisierung der getrennten Reaktionsräume durch Überschichtung der auf dem Träger immobilisierten Mikropartikel mit einer Trennflüssigkeit (siehe z.B. Fig. 4). Die Trennflüssigkeit ist mit der Reaktionslösung nicht mischbar. Vorzugsweise handelt es sich bei der Reaktionslösung um eine wässrige Lösung und bei der Trennflüssigkeit um eine ölige Zusammensetzung. Alternativ dazu kann es sich bei der Reaktionslösung um eine ölige Zusammensetzung und bei der Trennflüssigkeit um eine wässrige Lösung handeln. Vorzugsweise handelt es sich bei der öligen Zusammensetzung um ein Öl, bevorzugt um ein Mineralöl oder Siliconöl. Die ölige Trennflüssigkeit kann aber auch Detergenzien und Emulgatoren enthalten wie z.B. Triton X-100, ABIL EM 90 (Degussa), Span 80, Tween 80 in Konzentrationen von 0,1-10 % und bevorzugt 1-5 %.

Die Stabilisierung der getrennten Reaktionsräume kann auch dadurch erreicht werden, dass der Flüssigkeitsfilm der Reaktionslösung mit einem Deckglas abgedeckt wird.

Ebenfalls ist es möglich, dass eine andere Mikropartikelpopulation eingesetzt wird, um die Reaktionsräume zwischen den Mikropartikeln zu stabilisieren, wodurch diese andere Mikropartikelpopulation sozusagen als eine Art Abstandshalter oder als Diffusionssperre zwischen verschiedenen Reaktionsräumen eingesetzt wird. Diese andere Mikropartikelpopulation kann im Vergleich zu den Mikropartikeln, die für die Einrichtung der räumlich getrennten Reaktionsräume verwendet werden, andere Eigenschaften in Bezug auf beispielsweise die Größe, fluoreszierende, magnetische, paramagnetische und/oder oberflächenfunktionalisierte Eigenschaften aufweisen. Je nach Größe der Mikropartikel der anderen Mikropartikelpopulation, welche als Abstandshalter eingesetzt werden, kann der Abstand der Reaktionsräume zwischen den Mikropartikeln, welche für die Einrichtung dieser Reaktionsräume verwendet wurden, vergrößert oder verkleinert werden, um die Diffusion und die Verfügbarkeit von Reaktionslösung für die jeweilige Anwendung anpassen zu können. Solche Mikropartikel können bevorzugt aus Sepharose oder Nanozellulose aber auch aus Glas oder ähnlichen Polymermaterialien bestehen, wie sie auch für die Herstellung fluoreszenzkodierter Mikropartikel beschrieben wurden. Besonders bevorzugt können auch Gemische aus Mikropartikeln und Nanopartikeln eingesetzt werden, um besonders effektiv Diffusion in den Gefügelücken zwischen den Reaktionsräumen zu reduzieren. Diese anderen Mikro- oder Nanopartikel werden bevorzugt für die Gestaltung der Reaktionsräume genutzt, indem z.B. in deren innerer Porenstruktur Reaktionspartner, Reaktionsprodukte oder Probenmoleküle aufgenommen und durch Diffusion auch wieder abgegeben werden können. Durch Nutzung von anderen Mikropartikeln oder Nanopartikeln mit angepasster Porengröße können gezielt Ausgangsprodukte der Signalverstärkungsreaktion, wie Nukleotidtriphosphate, aufgenommen, gespeichert und abgegeben werden, wobei z.B. höhermolekulare Reaktionsprodukte, wie Amplifikatmoleküle einer PCR zu groß sind, um in die Poren der Partikel einzudringen und somit im Reaktionsraum nahe der Mikropartikel, die für die Einrichtung der Reaktionsräume genutzt werden, verbleiben. Niedermolekulare Reaktionsprodukte, die als PCR-Inhibitoren bekannt sind, können dagegen problemlos aus dem Reaktionsraum heraus diffundieren, in dem sie in die umgebenden porösen Partikel hineindiffundieren. Die Oberfläche der anderen Mikropartikel kann auch chemisch aktiviert vorliegen, wie im Falle von Bromcyano-Sepharose oder durch Oxidation im Falle von Nanozellulose. Dadurch können an die Oberfläche dieser Partikel vor, während oder nach der Signalverstärkungsreaktion Reaktionspartner oder Reaktionsprodukte der Signalverstärkungsreaktion gebunden werden, um die Signalverstärkungsreaktion zu beeinflussen. Solche Stoffe können bevorzugt Analyten aus der Probe, analytenähnliche oder -identische Standardmoleküle für Referenzzwecke, Enzyme, Antikörper aber Nukleinsäuren oder Oligonukleotide sein. So können auf der Oberfläche der Mikropartikel zur Einrichtung der Reaktionsräume alle Oligonukleotide für eine PCR gebunden sein und auf der Oberfläche der anderen Partikel die Analytmoleküle. Dabei erfolgt die Bindung der Analytmoleküle.

Durch die Stabilisierung der getrennten Reaktionsräume durch beispielsweise Überschichtung mit einer Trennflüssigkeit, können die Reaktionsräume über längere Zeiträume, z.B. über mehrere Stunden oder Tage, vor Umwelteinflüssen wie Austrocknung geschützt werden. Die Austrocknung, insbesondere, wenn die Signalverstärkungsreaktion höhere Temperaturen erfordert, wird weiter reduziert, wenn in der zur Überschichtung verwendeten Trennflüssigkeit weitere Mikropartikel mit einem Durchmesser zwischen bevorzugt 1-100 $\mu$m enthalten sind. Die in der Trennflüssigkeit enthaltenen Mikropartikel sinken nach dem Überschichten des Reaktionsraumes ab und bilden dadurch eine Sedimentschicht aus. Dadurch wird ein direkter Kontakt der Konvektionsströmung in der Trennflüssigkeit mit der Reaktionslösung vermieden.

Handelt es sich bei der Reaktionslösung um eine wässrige Lösung, so ist es ebenfalls möglich, dass die Reaktionslösung durch wasserbindende Substanzen gegen Verdunstung geschützt wird und entsprechend eine Überschichtung mit einer öligen Zusammensetzung als Trennflüssigkeit entfallen kann.

Werden beispielsweise die getrennten Reaktionsräume nicht durch Überschichtung mit einer Trennflüssigkeit stabilisiert, so kann die Reaktionslösung teilweise oder vollständig verdunsten. Die Verdunstung führt dazu, dass sich die Reaktionsräume verkleinern und sich benachbarte, konfluente Reaktionsräume z.B. zwischen zu dicht beieinander liegenden Mikropartikeln voneinander trennen. Eine vollständige Verdunstung der Reaktionslösung wird bevorzugt durch Zugabe von 0,5 - 10% Glycerol, 1-30% Saccharose und/oder durch Zugabe von 0,001-6M Betain und bevorzugt 1-3M Betain erreicht. Diese Verfahrensvariante kann auch dazu genutzt werden die Signalverstärkungsreaktion während des Eintrocknens der Reaktionslösung gezielt zu stoppen.

Durch die Stabilisierung ist sowohl eine Begrenzung der Schichtdicke der Reaktionsräume senkrecht zur Oberfläche des Trägers als auch eine laterale Begrenzung der Reaktionsräume parallel zur Oberfläche des Trägers möglich. Die Schichtdicke der Reaktionsräume kann durch Verwendung von Mikropartikeln mit definiertem Durchmesser eingestellt werden. Bevorzugt liegt die Schichtdicke je nach Anwendung zwischen 10nm und 100$\mu$m, bevorzugt zwischen 50nm und 100$\mu$m ganz besonders bevorzugt zwischen 1$\mu$m und 30$\mu$m, insbesondere bevorzugt zwischen 2$\mu$m und 20$\mu$m.

### Schritt b): Durchführung der Signalverstärkunasreaktion

Im nachfolgenden Schritt b) des erfindungsgemäßen Verfahrens wird die Signalverstärkungsreaktion durchgeführt. Diese erfolgt in den räumlich getrennten Reaktionsräumen in Abhängigkeit des Vorhandenseins des nachzuweisenden Analyten.

Während der Signalverstärkungsreaktion entsteht mithilfe des in der Reaktionslösung vorhandenen Substrats, welches durch eine enzymatische Aktivität umgewandelt wird, das messbare Reaktionsprodukt. Dabei kann die Menge an gebildetem Reaktionsprodukt in einem räumlich getrennten Reaktionsraum als Maß für die Menge an zu messenden Analyten in diesem Reaktionsraum verwendet werden.

In der Signalverstärkungsreaktion wird das messbare Signal des Reaktionsproduktes verstärkt. Je länger die Dauer der Signalverstärkungsreaktion, desto mehr Reaktionsprodukt wird hergestellt und desto stärker wird das messbare Signal des Reaktionsproduktes.

Bevorzugt erfolgt die Signalverstärkungsreaktion länger als 30 Sekunden, besonders bevorzugt länger als 5 Minuten, ganz besonders bevorzugt beträgt der Zeitraum 5 Minuten bis 48 Stunden. Insbesondere bevorzugt ist eine Signalverstärkungsreaktion von 1 Minute bis 3 Stunden.

Die enzymatische Umsetzung eines Substrats während der Signalverstärkungsreaktion umfasst vorzugsweise einen immunologischen Nachweis. Bevorzugt kann es sich bei dem immunologischen Nachweis um einen ELISA (ELISA = enzyme-linked immunosorbent assay) handeln. Umfasst die enzymatische Umsetzung eines Substrats einen ELISA als immunologischen Nachweis, so kann der Träger auf seiner Oberfläche Binder aufweisen, wobei die Binder bevorzugt spezifisch sind für die nachzuweisenden Analyten. In diesem Fall weisen die für die Einrichtung der getrennten Reaktionsräume verwendeten Mikropartikel keine Binder auf ihrer Oberfläche auf, die für den nachzuweisenden Analyten spezifisch sind.

Die enzymatische Umsetzung eines Substrats während der Signalverstärkungsreaktion umfasst vorzugsweise einen nukleinsäure-basierten Nachweis, z.B. eine Amplifikation mittels PCR (PCR = polymerase chain reaction). Umfasst die enzymatische Umsetzung eines Substrats eine PCR als Nachweis und soll z.B. eine oder mehrere Nukleinsäuren als Analyten nachgewiesen werden, so können beispielsweise die Mikropartikel mit Streptavidin beschichtet sein und einzelne, mehrere oder alle Sonden und Primer, die sich z.B. in der Reaktionslösung befinden, entweder am 5'-Ende oder intern biotinyliert sein. Sind die Mikropartikel von der Reaktionslösung überschichtet, werden die Sonden und Primer an den Mikropartikeln über die Avidin-Biotin-Bindung gebunden. Während der PCR-Denaturierungsphase denaturiert das Streptavidin, so dass die Sonden teilweise oder vollständig in den Reaktionsraum abgegeben werden. Die Freisetzung der Primer von der Mikropartikeloberfläche verbessert die Hybridisierungskinetik mit der Analytnukleinsäure als Template, und erhöht so die Signalstärken und die Sensitivität des Testsystems. Alternativ ist es möglich an der Oberfläche Avidin-beschichteter Mikropartikel biotinylierte Oligonukleotidsonden als Binder zu binden, an die die Primer und Sonden eines PCR-Systems vor oder nach der Immobilisierung der Mikropartikel hybridisiert werden. Nachdem die Reaktionsräume geschlossen wurden, dehybridisieren während der initialen PCR-Denaturierungsphase alle Primer und Sonden vom Binder und können somit in der Reaktionslösung zur Analytnukleinsäure diffundieren, die mit der Probe in die Reaktionslösung eingebracht wurde. Die weitere PCR findet bevorzugt ausschließlich in der Flüssigphase, d.h. der Reaktionslösung, statt, was Diffusionszeit und somit die Dauer der PCR im Vergleich zur Festphasen-PCR in unmittelbarer Nähe der Mikropartikeloberfläche verkürzen kann. Die Binder können bevorzugt aus repetitiven Nukleinsäuresequenzen aufgebaut sein, die komplementär zum analytspezifischen Abschnitt von Primern und Sonden sind. Besonders besitzen sie jedoch komplementäre Nukleinsäuresequenzen zu analytunspezifischen Sequenzen der Primer und Sonden. Die Verwendung repetitiver Sequenzen als Binder ermöglicht, eine größere Menge von Primer- und Sonden-Molekülen auf der Oberfläche der Mikropartikel zu binden und dann in die Reaktionslösung freizusetzen. Bevorzugt werden mit dem Ziel der Multiplex-Detektion die Primer und Sonden verschiedener PCR-Systeme an die Oberfläche von Mikropartikel unterschiedlicher Mikropartikelpopulationen hybridisiert. Dadurch gelingt es, verschiedene PCR-Systeme parallel aber räumlich getrennt in verschiedenen Reaktionsräumen durchzuführen.

Durch die Verwendung unterschiedlicher Primer/Sondensysteme die jeweils an verschiedenen, z.B. unterschiedlich fluoreszenzkodierten, Mikropartikelpopulationen gebunden sind, entstehen in den voneinander getrennten Reaktionsräumen unterschiedliche PCR-Reaktionen, die sich im Wesentlichen nicht gegenseitig beeinflussen. Derartige Beeinflussungen, die durch das erfindungsgemäße Verfahren vermindert oder vermieden werden können, sind z.B. Primerartefakte, kompetitive Hemmung oder Falsch-priming. Diese Beeinflussungen wirken sich negativ auf die Sensitivität und die Spezifität der verschiedenen PCR-Reaktionen aus und stellen somit wichtige Limitationen von Multiplex-PCRs dar. Außerdem wird der Optimierungsbedarf, der bei den meisten Multiplex-PCR auf Grund der geschilderten negativen Beeinflussungen nötig ist, drastisch verringert.

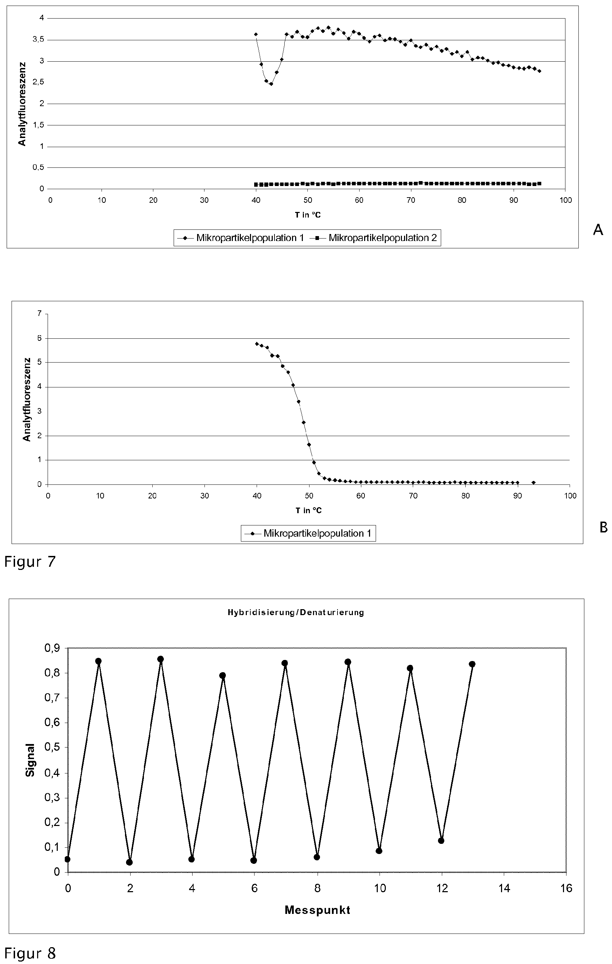

Werden z.B. fluoreszenzkodierte Mikropartikel und fluoreszenzmarkierte Sonden als Substrat in der Reaktionslösung für einen solchen nukleinsäure-basierten Nachweis verwendet, so ist zu Beginn der PCR aufgrund des Quenchings kein Fluoreszenzsignal messbar. Mit fortschreitender Amplifikation entsteht in Abhängigkeit der gebildeten Menge an Reaktionsprodukt ein Fluoreszenzsignal in diesem Reaktionsraum. Voraussetzung dafür ist, dass quencher- und oder fluophormodifizierte Oligo- oder Polynukleotidmoleküle über den Reaktionszeitraum hinweg miteinander hybridisieren können, was auch ohne PCR für Multiplex-Hybridisierungsnachweise genutzt werden kann (siehe z.B. Fig. 7 und 8). In Kombination mit einer PCR ist die Durchführung von z.B. Real-time-PCR in den Reaktionsräumen möglich.

Eine Ausführungsvariante verschiedener, voneinander separierter PCRs in verschiedenen Reaktionsräumen auf einem Träger besteht darin, dass Sondenprimer auf der Oberfläche fluoreszenzkodierter Mikropartikel reversibel über Avidin-Biotin oder Fängersonden gebunden werden. Sondenprimer sind Oligonuklotide und bestehen aus einem templatespezifischen 3'-Abschnitt mit einer Länge von bevorzugt 10-30 Nukleotiden und einem template-unspezifischen 5'-Abschnitt mit einer Länge von bevorzugt 20-30 Nukleotiden. Die 5'-Bereiche der Sondenprimer eines Amplikons unterscheiden sich bevorzugt insoweit voneinander, dass kein Cross-Priming innerhalb eines Amplikons auftritt. Die Sondenprimer ermöglichen initial die Hybridisierung des 3'-Endes an das Template und die enzymatische Verlängerung komplementär zum Template. Nachdem erste Amplikons, die beide Sondenprimer eines PCR-Amplikons umfassen, gebildet wurden, werden sie über die Universalprimer, die in einer Konzentration von bevorzugt 0,05-3$\mu$M gemeinsam mit der Polymerase und den Nukleotiden und bevorzugt mit den Sonden, in einer Sondenkonzentration von 0,01-3 M, mit in den Mastermix gegeben werden, amplifiziert. Die Universalprimer entsprechen bevorzugt der Sequenz des template-unspezifischen Abschnittes der Sondenprimer. Sie ermöglichen eine effektive Amplifikation, auch wenn die Sondenprimer in vergleichsweise geringer Konzentration im Reaktionsraum enthalten sind. Es ist auch möglich, dass Universalprimer und der ihnen entsprechende 5'-Abschnitt des Sondenprimers eine Sequenz besitzen, die während der PCR zur Bildung von Amplikon-Konkatameren führt. Diese aus mehreren Amplikon-Sequenzen repetitiv aufgebauten Amplifikatmoleküle sind in Ihrer Diffusion im Reaktionsraum auf Grund ihrer Größe eingeschränkt. In einer bevorzugten Ausführungsform sind die Universalprimer als Amplifluor- oder Scorpion-Primer strukturiert und dienen für alle im Reaktionsansatz ablaufenden PCR als universelle Signalgebende Komponente. Multiplexing in jedem Reaktionsraum ist möglich, indem verschiedene Sonden, die mit unterschiedlichen Fluoreszenzfarbstoffen für die Detektion unterschiedlicher Farbstoffe eingesetzt werden. Ebenso ist es möglich, unterschiedliche Amplikons dadurch zu differenzieren, dass sie auf Grund ihrer unterschiedlichen Anzahl von Basenpaaren spezifische Mengen DNA-bindender oder interkalierender Farbstoffe, wie SybrGreen oder EvaGreen, binden können. Durch Schmelzkurvenanalyse können bei Anwendung dieser Verfahren Schmelzkurvenpeaks bei verschiedenen Schmelztemperaturen und spezifische Signalintensitäten den verschiedenen Targets zugeordnet werden.

Signalverstärkungssystem

Vorzugsweise werden die Probe oder die auf dem Träger immobilisierten Mikropartikel vor der Durchführung der Signalverstärkungsreaktion in Schritt b) mit einem Signalverstärkungssystem kontaktiert. Das Signalverstärkungssystem weist ein erstes und ein zweites Element auf. Das erste Element bindet spezifisch an den nachzuweisenden Analyten der bevorzugt an einen Binder des Mikropartikels oder des Trägers gebunden sein kann oder ungebunden in der Reaktionslösung vorliegt. Das zweite Element weist eine enzymatische Aktivität auf, welche geeignet ist bei Kontakt mit einer Reaktionslösung in einer Signalverstärkungsreaktion ein messbares Reaktionsprodukt entstehen zu lassen. Durch die Inkubation mit dem Signalverstärkersystem kann im späteren Schritt b) des erfindungsgemäßen Verfahrens eine erhöhte Menge an messbarem Reaktionsprodukt erzielt werden.

Unter dem Begriff "kontaktieren" wird ein physikalischer Zustand des Berührens verstanden, welcher unter Bedingungen durchgeführt wird, die das in Kontakt bringen der Probe oder der auf dem Träger immobilisierten Mikropartikeln mit dem Signalverstärkungssystem ermöglichen. Geeignete Bedingungen für das in Kontakt bringen der Probe oder der auf dem Träger immobilisierten Mikropartikeln mit dem Signalverstärkungssystem sind im Stand der Technik bekannt. Diese Bedingungen umfassen vorzugsweise ein flüssiges Medium und/oder das Benetzen/Kontaktieren bei einer Temperatur von >0°C bis <95°C.

Substanzen, die unter den Begriff "erstes Element" fallen binden unter den gewählten Bedingungen vorzugsweise selektiv an den Analyten oder an den mit dem Analyten besetzten Binder. Das erste Element kann auf (Poly)Peptiden, Poly-oder Oligonukleinsäuren oder ähnlichem basieren. Vorzugsweise handelt es sich bei dem ersten Element um ein Polypeptid, bevorzugt um einen Antikörper wobei sowohl ein polyklonaler und monoklonaler Antikörper mit klassischer Antikörperstruktur als auch davon abgeleitete Derivate oder Fragmente umfasst sein können. Bei dem ersten Element kann es sich auch um eine Nukleinsäure, bevorzugt um eine Oligonukleinsäure, besonders bevorzugt um eine DNA-Oligonukleinsäure handeln.

Beim "zweiten Element" kann es sich um eine Substanz mit katalytischen Eigenschaften wie z.B. ein Enzym, DNAzymes, MNAzymes oder katalytische Antikörper (catmab) handeln. Bevorzugt handelt es sich bei dem zweiten Element um eine Peroxidase, insbesondere um eine Meerrettichperoxidase (HRP, Abkürzung für englisch horseradish peroxidase).

Vorzugsweise weist der Analyt und/oder das Signalverstärkungssystem eine enzymatische Aktivität auf. Bevorzugt weist das Signalverstärkungssystem eine enzymatische Aktivität auf, die bei Kontakt mit der Reaktionslösung in einer Signalverstärkungsreaktion zu einem messbaren Reaktionsprodukt führt. Bevorzugt handelt es sich bei der enzymatischen Aktivität um die Aktivität eines Enzyms aus der Klasse der DNA-Polymerasen, RNA-Polymerasen, reversen Transkriptasen, Ligasen, Oxidasen, Reduktasen, Transferasen, Peroxidasen, Phosphatasen, Proteasen, Peptidasen, Lipasen, Glykosidasen und/oder Luciferasen. Bevorzugt kann das Signalverstärkungssystem als erstes Element ein Polypeptid, z.B. einen Antikörper umfassen, welcher mit einem Enzym, wie z.B. Peroxidase, als zweitem Element markiert ist. Ebenfalls bevorzugt kann das Signalverstärkungssystem als erstes Element eine Nukleinsäure, z.B. eine DNA-Oligonukleinsäure umfassen, welche mit einem Enzym, wie z.B. Peroxidase, als zweitem Element markiert ist. Dabei ist es möglich, dass z.B. ein Peroxidase-Avidin-Konjugat biotinylierte Oligonukleotide als erstes Element bindet.

Der Nachweis des Analyten mithilfe des Signalverstärkungssystems kann entweder direkt oder indirekt erfolgen. Erfolgt der Nachweis direkt, so sind das erste und das zweite Element unmittelbar miteinander verbunden. So kann z.B. das erste Element ein Antikörper sein, welcher mit z.B. einer Peroxidase als zweitem Element verbunden ist. Erfolgt der Nachweis indirekt, so ist das zweite Element nicht unmittelbar mit dem ersten Element verbunden, sondern befindet sich auf einem weiteren Element. Dieses weitere Element kann auf (Poly)Peptiden, Poly-oder Oligonukleinsäuren, Mikropartikeln oder ähnlichem basieren. So kann z.B. das erste Element ein Antikörper sein, an welchen sich ein für diesen Antikörper spezifischer sekundärer Antikörper als weiteres Element bindet. Dieser sekundäre Antikörper kann z.B. eine Peroxidase als zweites Element umfassen. Wenn das erste Element z.B. eine Markierung mit Biotin umfasst, so kann das weitere Element eine Avidin-Markierung und eine Markierung mit dem zweiten Element umfassen. So ist es beispielsweise möglich, dass das erste Element ein biotinylierter Antikörper sein kann, an welchen ein avidin-markiertes Mikropartikel oder ein avidin-markierter sekundärer Antikörper als weiteres Element bindet. Dieses avidin-markierte Mikropartikel bzw. der avidin-markierte sekundäre Antikörper umfasst z.B. eine Peroxidase als zweites Element. Handelt es sich in diesem Beispiel um avidin-markierte Mikropartikel als weitere Elemente, so sind diese avidin-markierten Mikropartikel vorzugsweise nicht solche Mikropartikel, welche für die Einrichtung der räumlich getrennten Reaktionsräume verwendet werden. Vorzugsweise ist das erste Element des Signalverstärkungssystems unmittelbar mit dem zweiten Element des Signalverstärkungssystems verbunden. Bevorzugt erfolgt der Nachweis des Analyten mithilfe des Signalverstärkungssystems direkt.

Sofern der Analyt mithilfe eines Binders nachgewiesen wird, kann das Signalverstärkungssystem an den Analyten binden bevor, während oder nachdem dieser an seinen Binder bindet.

Die Art des entstehenden optisch messbaren Signals des Reaktionsproduktes hängt von dem verwendeten Signalverstärkungssystem und von dem entsprechend verwendeten Substrat der Reaktionslösung ab. Dem Fachmann sind sowohl geeignete Signalverstärkungssysteme als auch entsprechend dafür geeignete Substrate für die Reaktionslösungen bekannt.

Handelt es sich bei der enzymatischen Aktivität des Signalverstärkungssystems um die Aktivität eines Enzyms, z.B. einer Peroxidase, so können Substrate in der Reaktionslösung eingesetzt werden, die ein Reaktionsprodukt ergeben, welches durch seine Absorption, Fluoreszenz oder Lumineszenz messbar ist.

Es ist möglich in einem Reaktionsraum mehr als ein Signalverstärkungssystem zu verwenden. Beispielsweise können zwei verschiedene Signalverstärkungssysteme verwendet werden, um zwei Analyten in einem Reaktionsansatz nachzuweisen. Dabei können die zwei entstehenden Reaktionsprodukte unterschiedliche Absorptionsmaxima aufweisen, welche zwei verschiedene Fluoreszenzfarbstoffe löschen können.

Der Nachweis des Analyten mittels eines Signalverstärkungssystems kann entfallen, wenn der nachzuweisende Analyt selbst eine enzymatische Eigenschaft aufweist, die bei Kontakt mit der Reaktionslösung in einer Signalverstärkungsreaktion zu einem messbaren Reaktionsprodukt führt. Wenn der nachzuweisende Analyt die Aktivität eines Enzyms, z.B. einer Peptidase, aufweist, so können Substrate in der Reaktionslösung eingesetzt werden, die ein Reaktionsprodukt ergeben, welches durch seine Fluoreszenz optisch messbar ist. Dazu zählen z.B. Peptidsequenzen, die einen Fluoreszenzfarbstoff und einen Quencher umfassen und welche von der Peptidase gespalten werden können. Wird die Peptidsequenz gespalten, so wird die räumliche Nähe zwischen Fluoreszenzfarbstoff und Quencher aufgehoben und der Fluoreszenzfarbstoff als entstehendes Reaktionsprodukt kann nach Anregung mit Licht geeigneter Wellenlänge Licht emittieren, welches gemessen werden kann.

Ebenso kann der Nachweis mittels eines Signalverstärkungssystems entfallen, wenn der nachzuweisende Analyt in der Signalverstärkungsreaktion durch ein Enzym vervielfältigt werden kann und in der Signalverstärkungsreaktion außerdem ein Substrat verwendet wird, welches durch die Aktivität des Enzyms zu einem messbaren Reaktionsprodukt umgesetzt wird. Bevorzugt wird diese Ausführungsvariante des erfindungsgemäßen Verfahrens für die Realtime-Polymerase-Kettenreaktion (PCR), die Ligase-Kettenreaktion (LCR) oder andere, auch isothermale Realtime-Amplifikationsverfahren, wie Loop-mediated isothermal amplification (LAMP), Recombinase Polymerase Amplification (RPA), Helicase-dependend amplification (HDA), Strand-displacment-amplification (SDA) eingesetzt. Als Enzyme werden bevorzugt hitzestabile DNA-Polymerasen, wie die Taq-Polymerase in modifizierter oder nicht modifizierter Form mit einer Exonukleaseaktivität oder ohne eine Exonukleaseaktivität eingesetzt. Ebenfalls können reverse Transkriptasen oder Polymerasen mit Stranddisplacement-Aktivität oder RNA-Polymerasen eingesetzt werden. Als Substrat werden bevorzugt TaqMan-, Hybprobe-, Scorpion-, LoopTag-Sonden, Molecular-Beacons oder Amplifluor-Primer verwendet, die gemeinsam mit entsprechenden Primern, Reaktionspuffern und Nukleotidtriphosphaten für die real-time-PCR im Reaktionsraum verwendet werden. Mit fortschreitender Amplifikation wird in Abhängigkeit der gebildeten Menge an Reaktionsprodukt ein Fluoreszenzsignal gebildet oder gelöscht.

**Schritt c): optische Messung der Reaktionsprodukte**

In Schritt c) des erfindungsgemäßen Verfahrens erfolgt die optische Messung der Reaktionsprodukte. Vorzugsweise handelt es sich bei dem Reaktionsprodukt um ein optisch messbares Reaktionsprodukt. Bevorzugt handelt es sich um ein Reaktionsprodukt, welches durch seine Absorption, Lumineszenz, Phosphoreszenz oder Fluoreszenz optisch messbar ist. Das Reaktionsprodukt kann auch durch Löschung der Fluoreszenz optisch messbar sein.

Der Effekt der "Fluoreszenzlöschung" (englisch quenching) bezeichnet Vorgänge, die eine Abnahme in der Intensität der Fluoreszenz eines Fluorophors zur Folge haben, ohne dass der Fluorophor zerstört wird. Der Fachmann kennt verschiedene Effekte, welche zur Fluoreszenzlöschung führen können wie beispielsweise Komplexbildung, interne Konversion, Energieübertragung auf andere Moleküle, die als sog. Quencher bezeichnet werden. Vom Quenching zu unterscheiden ist die Abnahme der Fluoreszenz aufgrund hoher Anregungsintensitäten oder chemischer Veränderungen des Fluoreszenzfarbstoffes, z.B. durch Oxidation in Anwesenheit von Sauerstoff. Diese Art der Fluoreszenzabnahme bezeichnet man als Ausbleichen des Fluorophors, wobei der Fluorophor irreversibel zerstört wird. Dagegen ist die Fluoreszenzlöschung eines Fluorophors durch einen Quencher reversibel: Sobald der Quencher entfernt wird, steigt die Fluoreszenz wieder an.

Die optische Messung der Reaktionsprodukte wird vorzugsweise derart ausgeführt, dass die räumlich getrennten Reaktionsräume des Trägers individualisierbar erfasst werden, so dass Messwerte einzelnen Reaktionsräumen und/oder einzelnen Mikropartikeln zuordenbar sind.

**Schritt d): ggf. Bereitstellung der Messdaten für die Berechnung einer Analytkonzentration in einer Probe**

In Schritt d) des erfindungsgemäßen Verfahrens erfolgt ggf. die Bereitstellung der Messdaten für die Berechnung einer Analytkonzentration in einer Probe. Dabei werden vorzugsweise die Messdaten der optischen Messung für die Erkennung und/oder Individualisierung einzelner Reaktionsräume und/oder einzelner Mikropartikel verwendet. Bevorzugt wird für die Erkennung und/oder Individualisierung ein mathematischer Algorithmus eingesetzt, wobei es sich bevorzugt um einen Ranking-booster-, Dual-Octagon-, Inverted-Dual-Area-, Intensity-Vector-Algorithmus oder um eine Kombination davon handelt.

Die Bereiche, in denen ein Analytensignal entstanden ist, welches durch den Messvorgang erfasst wurde, werden durch den jeweils geeigneten Algorithmus einzelnen Mikropartikeln und/oder einzelnen Reaktionsräumen zugeordnet.

Zur Separierung der Mikropartikel wird bevorzugt ein kantenbasierendes Segmentierungsverfahren verwendet, z.B. der bekannte Canny-Algorithmus (John Francis Canny, "Finding Edges and Lines in Images", AI Technical Reports 1964-2004, 1. Juni 1983). Dieser ist in der Lage einzeln liegende Mikropartikel zu vollständig detektieren und Bereiche von dicht nebeneinanderliegenden Mikropartikeln als solche zu detektieren. Die schon als einzeln liegend erkannte Mikropartikel (Mikropartikel-Agglomerate) werden direkt den nachfolgenden Bildverarbeitungs-Schritten zur Bestimmung der Signalintensitäten zugeführt. Die Bereiche von dicht nebeneinanderliegenden Mikropartikeln werden mit Hilfe des Ranking-Booster-Algorithmus in einzelne Mikropartikel separiert.

Ranking-booster-Algorithmus

Die im erfindungsgemäßen Verfahren verwendeten Mikropartikel können einzeln und/oder teilweise dicht nebeneinander auf dem Träger vorliegen. Liegen die Mikropartikel dicht nebeneinander auf dem Träger vor, so wird bevorzugt der Ranking-booster-Algorithmus zur virtuellen Separation von sich gegenseitig berührenden Mikropartikeln eingesetzt.

Die im erfindungsgemäßen Verfahren eingesetzten Mikropartikel sind vorzugsweise sphärisch oder kreisrunde Scheiben. Bei Verwendung dieses Algorithmus zur Bestimmung der Analytkonzentration geht der Mikropartikel als einzelne, bevorzugt sphärische Struktur für die Mikropartikelerkennung verloren, da sich benachbarte Mikropartikel berühren und deshalb zunächst im Bild als eine konfluente Struktur erscheinen. Bei Verwendung von z.B. Mikropartikeln mit dreieckiger Grundfläche, müssen die nachfolgend beschriebenen Algorithmen entsprechend der à priori geltenden Geometrie dieser Objekte angepasst werden.

### Aufnahme des Bildes

Zunächst wird ein digitales Bild von den sich auf dem Träger befindenden Mikropartikeln erzeugt, bevorzugt durch ein vollautomatisches Aufnahmeverfahren erstellt. Die vorzugsweise verwendeten sphärischen Mikropartikel erscheinen im aufgenommenen Bild als kreisförmige Objekte. Eine Überbelichtung der Mikropartikel bei der Erstellung des Bildes sollte vermieden werden, da dadurch die Verwendbarkeit dieses Algorithmus eingeschränkt würde. Vorzugsweise wird eine Belichtungsautomatik verwendet, die eine mögliche Überbelichtung der Mikropartikel kompensiert.

### Bearbeitung des aufgenommenen Bildes

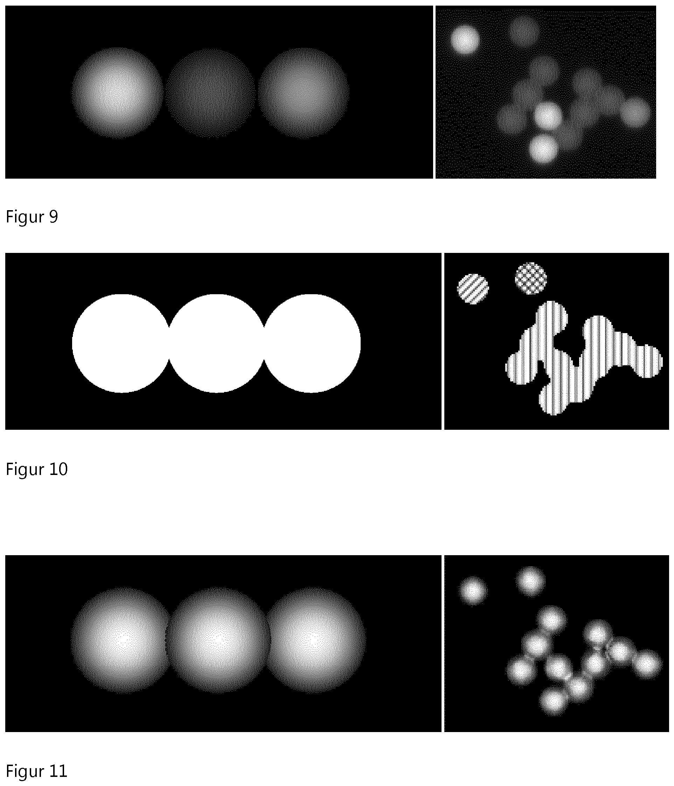

Das aufgenommene Bild wird bevorzugt zuerst mit einem konventionellen Objekterkennungsalgorithmus bearbeitet, der einzeln liegende Mikropartikel und Mikropartikel-Agglomerate als solche erkennt (siehe z.B. Fig. 9 und 10). Nur die Mikropartikel-Agglomerate werden einer Weiterverarbeitung durch den Ranking-Booster zugeführt. Bevorzugt können die betreffenden Bildausschnitte umfassend die Mikropartikel-Agglomerate mit einer höheren Auflösung neu aufgenommen werden. Die Außengrenzen der Mikropartikel-Agglomerate werden in diesem Schritt bestimmt.

### Erstellung eines "Relativ-Rang-Bildes"

Im nächsten Schritt wird eine bevorzugt im Wesentlichen kreisförmige Umgebungs-Pixelmaske konzentrisch um jedes Agglomerat-Pixel untersucht, deren Durchmesser des minimalen Mikropartikel-Durchmessers entspricht. Der relative Rang des aktuell untersuchten Pixel bzgl. seiner Umgebungs-Pixelmaske wird bestimmt und dieser Wert an entsprechender XY-Koordinate in einem Zwischenbild, dem Relativ-Rang-Bild gespeichert (siehe z.B. Fig. 11). Dieses Relativ-Rang-Bild kann optional vor der weiteren Bildverarbeitung mit beispielsweise einem Glättungsfilter, z.B. einem 3x3- oder 5x5-Medianfilter bearbeitet werden, um mögliche lokale Störungen zu eliminieren.

### Erstellung eines Binärbildes aus dem "Relativ-Rang-Bild"

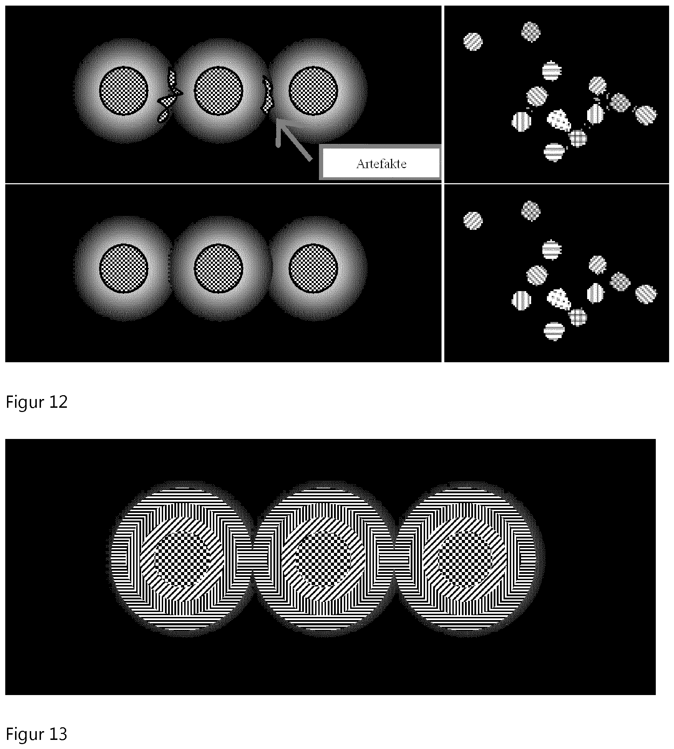

In einem weiteren Schritt wird das Relativ-Rang-Bild bevorzugt mit einem Schwellwert binarisiert, da die absoluten Helligkeiten der Mikropartikel nach dem Ranking-Booster keine Rolle mehr spielen (siehe z.B. Fig. 12). Vorzugsweise liegen die Schwellenwerte in einem Bereich von 0.1 bis 0.9, bevorzugt von 0.25 bis 0.8, besonders bevorzugt 0.3 bis 0.7, ganz besonders bevorzugt 0.4 bis 0.5. Als Folge der Binarisierung ist die Zentrumsregion jedes Mikropartikels in dem entstandenen Binärbild als kreisrunde Zentrums-Scheibe erkennbar, weil der Mittelpunkt eines Mikropartikels (im Originalbild) immer dessen hellster Pixel ist und die Pixel-Helligkeiten radial abfallen. Vorzugsweise beinhalten die Zentrums-Scheiben bevorzugt eine Fläche, die kleiner als die Fläche eines Mikropartikels ist, so dass sich die Zentrums-Scheiben der sich gegenseitig berührenden Mikropartikel nicht kontaktieren.

Mittels Objekterkennung werden die Zentrums-Scheiben identifiziert. Sonstige kleine Objekte in den Zwischenräumen sich berührender Mikropartikel, die z.B. sichelförmig oder dreieckig erscheinen, sind Artefakte, die an ihrer nichtrunden Form und zu geringer Fläche erkennbar sind. Diese Artefakte werden vorzugsweise ersatzlos verworfen und nicht in der weiteren Berechnung berücksichtigt.

### Detektion der Kontaktpunkte zwischen den Mikropartikeln

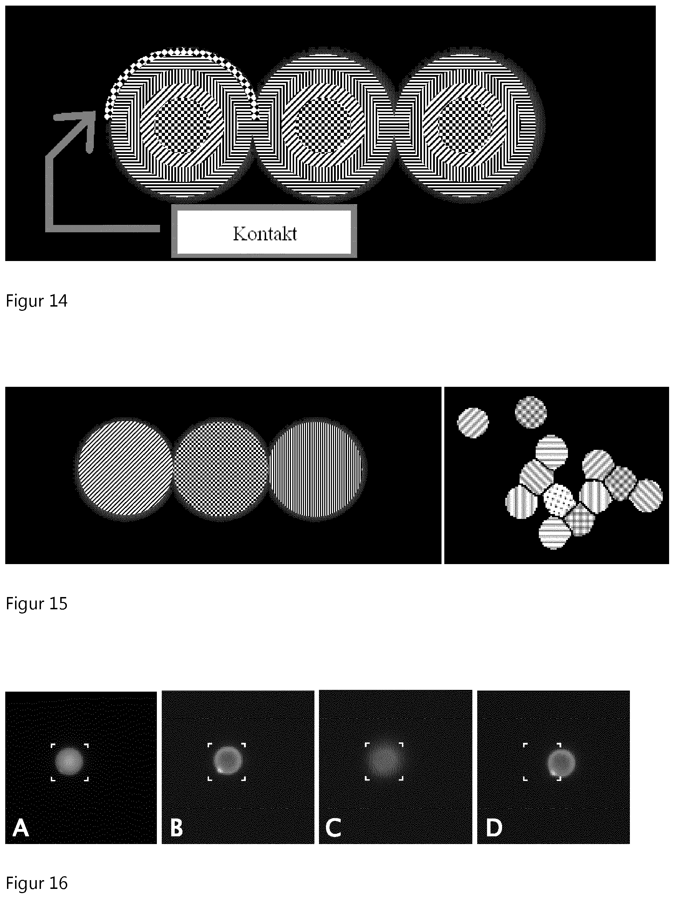

Um die ursprüngliche Fläche der Mikropartikel wieder zu erlangen, werden die Zentrums-Scheiben Schritt für Schritt vergrößert, so dass die vergrößerten Zentrums-Scheiben Markierungen für die gefundenen Mikropartikel darstellen. Um den Rand jeder identifizierten Zentrums-Scheibe wird jeweils eine Lage Pixel pro Iterations-Schritt hinzugefügt (siehe z.B. Fig. 13). Sobald ein neu hinzugefügtes Pixel einer Zentrums-Scheibe an ein originales oder bereits hinzugefügtes Pixel einer anderen Scheibe grenzt, ist ein Kontaktpunkt zwischen Mikropartikeln identifiziert (siehe z.B. Fig. 14). Die Detektion eines Kontaktpunkts beendet die Inflation beider beteiligten Zentrums-Scheiben, die Pixel der unvollständigen letzten Lage werden wieder entfernt. Für Zentrums-Scheiben, für die noch keine Kontaktpunkte detektiert wurden, werden weitere Inflations-Iterationen durchgeführt. Die Inflation der Zentrums-Scheiben wird jedoch auf das ursprüngliche Agglomerations-Objekt limitiert, d.h. die Iterationen für eine Scheibe wird gestoppt, sobald ein Pixel hinzugefügt werden müsste, das nicht zum Ausgangs-Gesamtobjekt gehört.

Optional kann auf die Detektion der Kontaktpunkte zwischen Mikropartikeln verzichtet werden und nur das ursprüngliche Agglomerations-Objekt die Inflation limitieren, d.h. der Zentrums-Scheibe können auch unvollständige Lagen hinzugefügt werden, jedoch ohne dabei anwachsende Zentrums-Scheiben angrenzender Mikropartikel zu überlappen und dadurch zu schädigen (siehe z.B. Fig. 15). Die vergrößerten Zentrumsscheiben markieren die zu den Mikropartikeln gehörenden Flächen im Bild.

Bestimmung der Signalintensität des Reaktionsprodukts

Nach erfolgter Identifizierung der einzelnen Mikropartikel in einem Mikropartikel-Agglomerat sowie gegebenenfalls der Zuordnung dieser einzelnen Mikropartikel zu einer Mikropartikelpopulation wird das von der Analytkonzentration abhängige Signal des Reaktionsprodukts bestimmt.

Dual-Octagon-Algorithmus

Für die Detektion von an der Mikropartikeloberfläche gebundenen Analyten (Ringfluoreszenz bzw. zur Detektion von Analytfluoreszenz in den Reaktionsräumen ist der im Folgenden beschriebene Dual-Octagon-Algorithmus geeignet.

Unter Umständen können die Bildkoordinaten eines Bildobjekts, z.B. der fluoreszenzkodierten Mikropartikel, nicht präzise bestimmt werden. Ursache dafür kann z.B. Unschärfe oder Bewegung des Objekts während der Aufnahme sein, wie z.B. bei nicht permanent immobilisierten Mikropartikeln, die sich im Reaktionsraum bewegen. Eine weitere Ursache kann in den optischen Eigenschaften des verwendeten Strahlenganges im Messsystem liegen (siehe z.B. Fig. 16).

Um die Gesamthelligkeit des am Mikropartikel gebundenen und markierten Analyten dennoch präzise bestimmen zu können, kann der in der vorliegenden Erfindung offenbarte Bildverarbeitungs-Algorithmus "Dual-Octagon" angewendet werden. Dieser benötigt nur die ungefähren XY-Koordinaten des Objekts. Das Verfahren basiert darauf, dass i.d.R. um das zu bewertende Objekt herum eine detektierbare Hintergrund-Helligkeit im Reaktionsraum vorhanden ist.

Folgende Voraussetzungen müssen für die Anwendung des Dual-Octagon-Algorithmus sichergestellt werden: Die Größe des Objekts in Pixeln muss bekannt sein. Diese Information kann vorhanden sein durch z.B. Berechnung aus realer Objektgröße, Vergrößerung und Kameraauflösung oder verfügbarer Spezifikationen der Mikropartikel, oder aus zusätzlichen Informationen gewonnen werden. Die ungefähren XY-Koordinaten des Objekts im Reaktionsraum müssen mit einer Genauigkeit von 1-10$\mu$m bestimmbar sein. Es müssen sich hinreichend wenige Fremdobjekte in der unmittelbaren Umgebung des untersuchten Bildobjektes befinden, so dass die Mikropartikel einzeln liegen und die für den Algorithmus erforderlichen Messfelder sich nicht beeinflussen.

Bei der Anwendung des Dual-Octagon-Algorithmus werden die reale Anzahl der zum Objekt gehörigen Pixel $N_{Or}$ und ebenso die XY-Mittelpunkt-Koordinaten des Objekts so genau wie möglich bestimmt. Diese ermittelten Koordinaten werden im Weiteren als angenommene Koordinaten $X_a$, $Y_a$ bezeichnet, da sie von den nicht bekannten realen Koordinaten $X_r$, $Y_r$ des Objekts um eine maximale Verschiebungs-Distanz $\pm D_x$, $\pm D_y$ abweichen dürfen.

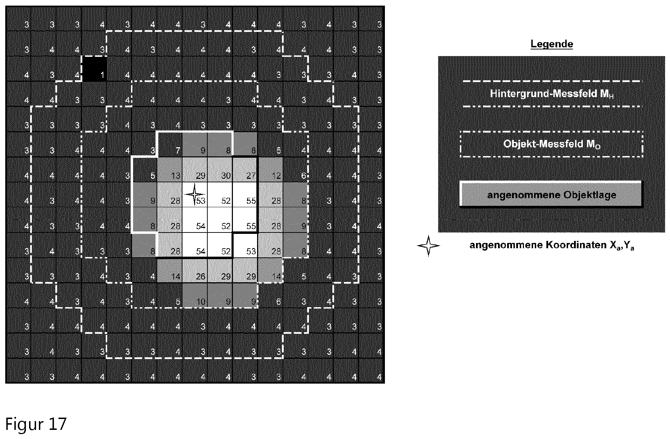

Um die angenommenen XY-Koordinaten des Objekts wird zentriert ein Messfeld $M_O$ für das Objekt mit definierter Pixelanzahl $N_O$ gelegt (siehe z.B. Fig. 17). Die Größe dieses Objekt-Messfeldes ist bevorzugt so zu dimensionieren, dass das Objekt auch bei maximal auftretender $D_x D_y$-Verschiebung zwischen realen und angenommenen Koordinaten vollständig im Messfeld enthalten ist. Die Form des Objekt-Messfeldes kann kreisförmig, elliptisch, ein konvexes Polygon oder eine andere geeignete geometrische Form sein. Insbesondere ein achteckiges Polygon ist geeignet, da dieses Rechenzeit sparend realisierbar ist. Die Summe der Grauwerte aller Pixel im Objekt-Messfeld $\sum M_O$ wird bestimmt.

Außerhalb des Objekt-Messfeldes $M_O$ wird ein zweites Messfeld $M_H$ für den Hintergrund mit definierter Pixelzahl $N_H$ definiert. Dieses Hintergrund-Messfeld umschließt das Objekt-Messfeld, d.h. $M_H$ muss ein hinreichend großes Loch für $M_O$ enthalten. Insbesondere geeignet ist ein achteckiges Polygon konzentrisch um das Objekt-Messfeld $M_O$. Zwischen den beiden Messfeldern kann optional ein Abstand verbleiben.

Optional werden die Pixel des Hintergrund-Messfeldes $M_H$ einer Ausreißerbereinigung unterzogen, d.h. eine Anzahl der dunkelsten Pixel und eine vorzugsweise gleichgroße Anzahl der hellsten Pixel werden von der Auswertung ausgeschlossen. Die Pixelanzahl $N_H$ des Hintergrund-Messfeldes wird auf die Anzahl der nach der Filterung verbliebenen Pixel herabgesetzt. Die Anzahl der zu verwerfenden Pixel sollte sich nach der Wahrscheinlichkeit richten, mit der sich Fremd-Objekte im Hintergrund-Messfeld befinden. Optional können auch andere geeignete Verfahren zur Ausreißerbereinigung angewendet werden, z.B. Verfahren die die real vorhandene Streuung der Grauwerte berücksichtigen.

Die Summe der Grauwerte aller nach Filterung verbliebenen Pixel im Hintergrund-Messfeld $\sum M_H$ wird bestimmt.

Der mittlere Hintergrundgrauwert in der Nähe des Objekts $GW_H$ wird berechnet durch:

$$GW_H = \sum M_H / N_H$$

Die Helligkeit des Objekts $GW_O$, d.h. dessen mittlerer Grauwert, wird berechnet:

$$GW_O = ( \sum M_O - ( GW_H \cdot ( N_O - N_{Or} ) ) ) / N_{Or} = ( \sum_{MO} - ( ( \sum_{MH} / N_H ) \cdot ( N_O - _{NOr} ) ) ) / N_{Or}$$

Obige Formel berücksichtigt, dass das Objektmessfeld größer ist als das reale Objekt und somit zu viele Grauwerte des Hintergrunds in die Summenbildung eingehen. Es wird davon ausgegangen, dass der Hintergrund im Bereich des Objekt-Messfeldes genauso hoch ist wie im umgebenen Hintergrund-Messfeld. Multipliziert man den Hintergrundwert mit der Differenz zwischen Pixelanzahl des Objekt-Messfeldes und Pixelanzahl des Objekts, erhält man die Summe der zuviel eingegangenen Grauwerte und kann diese von der Summe der Grauwerte im Objekt-Messfeld subtrahieren. Die so berechnete Helligkeit des Objekts $GW_O$ ist selbst noch nicht hintergrundkorrigiert. Die bevorzugte Methode zur Hintergrundkorrektur ist die Bildung der Differenz aus mittlerem Grauwert des Objektes ($GW_O$) und dem mittleren Grauwert des Hintergrundes ($GW_H$):

$$GW_{O,hintergrundkorrigiert} = GW_O - GW_H$$

Optional können andere Verfahren zur letztendlichen Hintergrundkorrektur des Objekts verwendet werden.

Berechnungsbeispiel anhand der schematischen Darstellung:

$N_{Or}$ = 21        ... reale Anzahl der zum Objekt gehörigen Pixel

$N_O$ = 69        ... Pixelanzahl des Objekt-Messfeldes

$\sum M_O$ = 1098        ... Summe der Grauwerte aller Pixel im Objekt-Messfeld

$N_H$ = 60        ... nach Filterung verbliebene Pixelanzahl des Hintergrund-Messfeldes (Ausschluss der 8 hellsten und 8 dunkelsten Pixel des Hintergrund-Messfeldes)

$\sum M_H$ = 215        ... Summe der Grauwerte aller nach Filterung verbliebenen Pixel im Hintergrund-Messfeld

$GW_H$ =3,583        ... mittlere Hintergrund-Grauwert in der Nähe des Objekts

$GW_O$ = 44,10        ... mit Dual-Octagon ermittelte Helligkeit des Objekts

zum Vergleich:

$GW_{Or}$ = 31,2        ... mittlere Helligkeit im Bereich der angenommenen Objektlage

Der Vergleich zwischen $GW_O$ und $GW_{Or}$ zeigt, dass durch Anwendung des Dual-Octagon-Algorithmus das Signal-Rausch-Verhältnis im vorliegenden Beispiel um ca. 30 % verbessert wird.

Inverted-Dual-Area-Algorithmus

Zur Detektion von Analyten in der flüssigen Umgebung von Mikropartikeln, insbesondere bei dicht auf dem Träger befindlichen Mikropartikeln, ist der Inverted Dual-Area-Algorithmus zu bevorzugen.

Im Fall von sich berührenden Reaktionsräumen können bei zu starker Hintergrund-/Messfeldüberlappung (Oktagon-Überlappung) benachbarte Reaktionsräume nicht mit dem Dual-Octagon-Algorithmus ausgewertet werden, da kein Hintergrundmessfeld zur Korrektur des Signals des Reaktionsproduktes mit ausreichender Größe definiert werden kann und sich die Messfelder benachbarter Mikropartikel zu stark überlappen können. Der Dual-Octagon-Algorithmus kann so mit zunehmender Dichte der Mikropartikelpackung auf dem Träger zu verfälschten Ergebnissen führen.

Der Inverted-Dual-Area-Algorithmus nutzt den zentralen Bereich der Mikropartikel für den Hintergrundabgleich, der vorzugsweise nur in geringem Ausmaß ein Signal des Reaktionsproduktes aufweist. Das Signal ist typischerweise als ringförmige Struktur ausgebildet, wenn es direkt auf der Oberfläche des Mikropartikels entsteht, das heißt es nimmt zum Mittelpunkt des Mikropartikels ab. Entsteht das Signal in unmittelbarer Umgebung des Mikropartikels in der flüssigen Phase des Reaktionsraumes, dann bleibt dieses Signal im Bereich der Kontaktfläche des Mikropartikels mit dem Träger nahezu komplett ausgespart. Je größer der Mikropartikel, umso mehr Pixel stehen für den Hintergrundabgleich zur Verfügung. Bevorzugt werden die Pixel für den Hintergrundabgleich genutzt (Area 1), deren Standardabweichung des Mittels der Graustufenintensitäten des Analytensignals sich um mindestens den Faktor 3 von der Standardabweichung des Mittels der Graustufen des Analytensignals im Bereich der äußeren Pixelreihe des Mikropartikels (Area 2) unterscheiden.

Die mathematischen Verfahren des Dual-Octagon-Algorithmus können vom Fachmann leicht auf den Inverted-Dual-Area-Algorithmus angepasst werden. Da die für den Hintergrundabgleich und das Signal genutzte Flächen von der Verteilung des Signals über dem jeweiligen Mikropartikel abhängen, werden bevorzugt Flächen genutzt, die bei Mehrfachmessungen und von Mikropartikel zu Mikropartikel zur Optimierung des Signal-/Rauschverhältnisses variieren können. Zur Verkürzung der Messzeit ist es alternativ ebenso möglich à priori-Wissen zu nutzen und z.B. Kreisflächen oder Oktagons feststehender Größen für die jeweilige Mikropartikelpopulation anzuwenden.

Bevorzugt wir der Inverted-Dual-Area-Algorithmus für kinetische Messungen oder für die Auswertung thermozyklisch gesteuerter Reaktionen genutzt.

Eine Voraussetzung für die Anwendung des Inverted Dual-Area-Algorithmus bei Aufzeichnung und Überlagerung von Farbaufnahmen unterschiedlicher Farbkanäle ist, dass ein chromatisch korrigierter Strahlengang für das Messsystems genutzt wird bzw. dass die Koordinatenveränderung mehrfarbiger Objekte durch die Auswertesoftware unter Berücksichtigung der chromatischen Abberation korrigiert wird. Dieses Erfordernis ergibt sich bevorzugt bei Mehrfarbenfluoreszenz-Messsystemen, wie sie z.B. bei der Auswertung von Tests von fluoreszenzkodierten Mikropartikeln angewendet werden können.

Analyse

Durch die Begrenzung der Reaktionsräume ist es möglich, die vorzugsweise an den Träger gebundenen Analyten auf einzelne Analytsignale zurückzuführen. Je weiter sich das Verhältnis der Anzahl von Reaktionsräumen und Analyten in der Probe in Richtung Reaktionsräume verschiebt, umso wahrscheinlicher wird es, dass sich nur noch ein Analytmolekül in einem Reaktionsraum befindet. Dies lässt im unteren Analyt-Konzentrationsbereich eine absolute, d.h. digitale Quantifizierung zu, da die Zahl der positiven Reaktionsräume der Zahl der Analyten in der Probe bzw. der Zahl der aus der Probe gebundenen Analyten entspricht. Dies setzt voraus, dass es keine oder nur eine sehr geringe unspezifische Bindung von Bestandteilen der Reaktionslösung an den Träger gibt. Werden Analytmoleküle und Bestandteile der Reaktionslösung in geringem Ausmaß unspezifisch an den Träger gebunden, kann der daraus resultierende Fehler vernachlässigt bzw. experimentell unter Bezug auf für den Analyten negative Kontrollproben ermittelt und kalkuliert werden.

Wird die Probe vollständig auf die Reaktionsräume verteilt, dann kann die Zahl der Analyten bevorzugt direkt vom Reaktionsraumvolumen auf das Probenvolumen hochgerechnet werden. Das Reaktionsraumvolumen kann bevorzugt bei Verwendung von Mikropartikeln aus der Kugelgeometrie berechnet werden, da es direkt proportional zum Kugelvolumen der Mikropartikel ist. Alternativ kann bei Verwendung eines Fluoreszenzfarbstoffes zur Generierung eines Anfangssignals, welches im Laufe der Signalverstärkungsreaktion gelöscht werden soll, die Fluoreszenzintensität bzw. der Intensitätsabfall des Anfangssignals radial vom Zentrum des Mikropartikels weg, genutzt werden, um das Reaktionsraumvolumen mit ausreichender Näherung zu berechnen. Fluoreszenzintensität als auch der Abfall der Fluoreszenzintensität während der Signalverstärkungsreaktion sind proportional zum Volumen des jeweiligen Reaktionsraumes. Aus der Summe des Volumens aller Reaktionsräume ergibt sich das Gesamtvolumen des Reaktionsraumes aus dem das Gesamtvolumen der eingesetzten Probe berechnet wird.

Wird bei der Signalverstärkungsreaktion eine Oberflächenfluoreszenz oder eine Fluoreszenz sehr nahe der Oberfläche gelöscht oder erzeugt, so ist es bevorzugt möglich lediglich den im Reaktionsraum liegenden Mikropartikel mit seiner Oberflächenfluoreszenz, der sehr nahe gelegenen Fluoreszenz oder seiner Kodierungsfluoreszenz in die Messung und Auswertung einzubeziehen, ohne den peripher davon gelegenen Reaktionsraum zu berücksichtigen.

Intensity-Vector-Algorithmus

Zur Detektion von Analyten in der flüssigen Umgebung von Mikropartikeln, insbesondere bei dichtgepackt auf dem Träger befindlichen Mikropartikeln, ist der Intensity-Vector-Algorithmus zu bevorzugen.

Eine weitere Möglichkeit ist die Berechnung der radialen Intensitätsabfallvektoren, welche besonders für die Auswertung lateral nicht vollständig geschlossener Reaktionsräume geeignet ist, da diese eine Beschreibung nicht kreisförmiger Reaktionsräume zulassen. Je dichter die Vektoren gelegt werden, umso genauer kann der Reaktionsraum durch entsprechenden Algorithmen beschrieben werden. Die Erfassung der Intensitätsvektoren erfolgt durch konzentrisches Erfassen des Signals des Reaktionsproduktes der Signalverstärkungsreaktion im Reaktionsraum durch Auslesen der Pixel mit entsprechendem Winkelabstand auf dem gleichen Radius. Bei einem kreisförmig konzentrischen Reaktionsraum sind die Pixelintensitäten auf dem gesamten Radius mit großer Näherung gleich. Das Zentrum der konzentrischen Ausrichtung der Intensitätsvektoren kann ein Mikropartikel aber auch ein Element eines Signalverstärkungssystems sein, welches z.B. über ein immobilisiertes Analytmolekül indirekt am Träger gebunden vorliegt.

Berühren sich die Reaktionsräume teilweise, so wird über geeignete Algorithmen die Vermischung von Reaktionsprodukt der Signalverstärkungsreaktion benachbarter Reaktionsräume kalkuliert.

Auf dem Träger gebundene Analyten in lateral nicht vollständig oder nur teilweise getrennten Reaktionsräumen lassen sich durch eine kinetische Messung der katalytischen Aktivität des Signalverstärkungssystems erfassen, sofern der Analyt nicht selbst ein Enzym ist, welches direkt durch seine Enzymaktivität nachweisbar ist. Beispielsweise kann ausgehend vom Ort des auf dem Trägers gebunden Elements des Signalverstärkungssystems die Ausbreitung des Reaktionsproduktes im Reaktionsraum durch Intensitätsvektoren beschrieben werden, welche mit fortschreitender Reaktionsdauer größer werden, so lange das Enzym seine Enzymaktivität nicht verliert.

Da die Intensität der Löschung oder Erzeugung der Fluoreszenz in der Reaktionslösung unmittelbar am aktiven Signalverstärkungssystem am höchsten ist, werden bevorzugt durch die Beschreibung der Intensitätsverteilungsmuster und deren Veränderung konfluente Bereiche der Fluoreszenzlöschung unterschiedlichen Signalverstärkungssystemen zugeordnet. Dadurch können Moleküle von Signalverstärkungssystemen mit einem kleineren Abstand als $10\mu m$ zueinander sicher identifiziert und im Rahmen der absoluten Quantifizierung gezählt werden.

Zur Standardisierung des Messverfahrens wird die Schichtdicke, die proportional zur Fluoreszenzintensität der Reaktionslösung im Reaktionsraum zum Zeitpunkt t0 ist, bevorzugt für jeden Bildausschnitt, bestimmt.

Von der Schichtdicke, der Viskosität bzw. Zusammensetzung der Reaktionslösung und der Thermokonvektion hängt maßgeblich ab, wie schnell das Produkt der Signalverstärkungsreaktion in den Reaktionsraum hinein diffundiert. Die Diffusion des Reaktionsproduktes kann auch stark beeinflusst werden durch die Verwendung präzipitierender Substrate oder von Substraten, deren Reaktionsprodukt an den Träger bzw. an Mikropartikel in unmittelbarer Nähe bindet.

Für die Berechnung der Analytkonzentration ist es möglich, die Zahl der identifizierten Signalverstärkungssystemmoleküle, die Zahl der signalverstärkten Mikropartikel, die signalverstärkten Reaktionsräume oder "Schwarze Löcher" zu bestimmen. Es ist aber auch entsprechend des erfindungsgemäßen Verfahrens möglich, das Reaktionsprodukt das durch die Signalverstärkungssysteme erzeugt wurde, als Summensignal über alle verschiedenen Arten von Reaktionsräumen zu erfassen, insbesondere bei höheren Analytkonzentration in der Probe. Dieses Summensignal kann auf eine externe Referenz kalibriert werden. Die Zeit, in der die Enzymaktivität in den Reaktionsräumen gelöscht wird, als auch das Ausmaß der Löschung, sind proportional zur Analytkonzentration in der Probe.

Externe Referenzproben, die unterschiedliche Analytkonzentrationen haben, werden bevorzugt zur Erstellung von Kalibrierkurven im parallelen Ansatz mitgeführt.

Nachfolgend wird die Anwendung des Intensity-Vector-Algorithmus zur Detektion von Analyten exemplarisch dargestellt. Die Reaktionsprodukte der Signalverstärkungsreaktion werden ausgehend von einzelnen Enzymmolekülen, die gelöst oder am Analyten gebunden im Reaktionsraum diffundibel oder z.B. an Mikropartikeln oder am Träger gebunden vorliegen können, bestimmt. Folgende Verfahrensschritte werden bevorzugt ausgeführt:
Bildung von N gleichlange Profilvektoren ausgehend vom Reaktionsmittelpunkt der Enzymsubstratlösung in alle Richtungen mit äquidistanten Winkeln. Es werden bevorzugt 8 Vektoren, bevorzugt im 45° Winkel, gebildet, vorzugsweise $\geq$ 12 Vektoren. Die Länge der Vektoren L ist so gewählt, dass sie auch bei maximaler Intensität des Analyten Signals dieses vollständig erfassen bis das Signalniveau des Hintergrunds erreicht wird.

Danach erfolgt die Bestimmung der Intensitätsprofile in jedem Vektor mit Resampling durch umgekehrten Bresenham-Algorithmus. Die Profile können optional geglättet werden. Alternativ kann ein Funktions-Fitting durchgeführt werden gegen eine geeignete Funktion, welche den üblichen Profilverlauf nachvollzieht. Das Verfahren kann bei positiven Signalverläufen (Signalanstieg bei Anwesenheit des Analyten) und auch bei invertierten Signalverläufen (Signalabfall/Löschung bei Anwesenheit des Analyten) angewendet werden.

Die Detektion des Endabfalles des Signals in jedem Profilvektor ergibt die Analytenausdehnung für diese Richtung, wodurch die Ausdehnungsform des Substratreaktionsproduktes bestimmt werden kann.

Die Detektion des Endwertes des Signals in jedem Profilvektor ergibt den Hintergrundwert in dieser Richtung. Eine ausreißerbereinigte Mittelwertbildung über die Hintergrundwerte aller Profilvektoren ist geeignet, den Hintergrund für das gesamte Objekt zu quantifizieren. Erreichen ein oder mehrere Vektoren den Gesamthintergrundwert nicht (in ausreichendem Maße) kann das Verfahren mit längeren Profilvektoren wiederholt werden. Alternativ kann dadurch eine Vereinigung mit einem Fremdobjekt iterativ detektiert werden. Gegen Ende des Profilvektors nichtmonoton verlaufende Signalverläufe, indizieren ein Zusammenfließen der Reaktionsprodukte der Signalverstärkungsreaktionen benachbarter Signalverstärkungsmoleküle lateral nicht begrenzter oder konfluenter Reaktionsräume.

Ein stufenförmiger Signalanstieg oder Signalabfall am Anfang des Profilvektors kann z.B. durch einen oder mehrere Festkörper im Reaktionsraum bedingt sein, wie z.B. einem Mikropartikel an dem das Signalverstärkungssystem im Laufe des Nachweisverfahrens immobilisiert wurde, und welcher bevorzugt selbst kein Signal in dem signalisierenden Wellenlängenbereich emittiert. Das Ausmaß dieser Stufe kann als Referenzsignal genutzt werden.

Werteberechnung:

N     ... Anzahl aller Profilvektoren

L     ... Anzahl der Elemente in jedem Profilvektor nach Resampling

$I_{p,k}$    ... Intensität des Profilvektors p an der Stelle k

$H_p$    ... Hintergrundwert des Profilvektors p

$H_0$    ... Hintergrundwert des Objekts ist eine Funktion aller Hintergrundwerte $H_p$, $H_0 = f(\underline{H_p})$

$I_O$    ... Analytenintensität des Objekts, berechnet durch

$$I_O = \frac{1}{N} \cdot \sum_{p=1}^{N} \sum_{k=1}^{L} I_{p,k} - H_0$$

Radialprofil-Algorithmus

Zur Detektion von sehr schwachen Analyten-Signalen direkt auf der Oberfläche von sphärischen Mikropartikel wird bevorzugt der Radialprofil-Algorithmus verwendet.

Ausgehend vom Mittelpunkt jedes Mikropartikel wird ein Radialprofil berechnet. Dazu wird in pixelweise wachsenden Radiusabständen der Mittelwert aller kreisförmig angeordneten Pixel gebildet, die den jeweiligen Radius entsprechen. Typischerweise erzeugen die Pixel, die sich in der Nähe des Mikropartikelrandes befinden, eine deutliche Intensitäts-Spitze im Profilverlauf. Das Signal ergibt sich aus der Höhe dieser Intensitäts-Spitze. Optional können die Werte an den unmittelbaren Nachbar-Radien neben der Intensitäts-Spitze mit in die Signalbewertung einbezogen werden.

Ausführungsformen des erfindungsgemäßen Verfahrens

In einer Ausführungsform befinden sich die Binder, die spezifisch sind für die nachzuweisenden Analyten auf der Oberfläche des Trägers.

In dieser bevorzugten Ausführungsform wird das erfindungsgemäße Verfahren für den Nachweis eines Analyten in einer Probe verwendet (bevorzugt Singleplex-Nachweis) und umfasst die Schritte a) Aufbringen einer Probe und einer Reaktionslösung auf einen Träger der bevorzugt an seiner Oberfläche Binder trägt sofern der Analyt kein Enzym ist, wobei die Reaktionslösung geeignet ist in einer Signalverstärkungsreaktion in Abhängigkeit des Vorhandenseins des nachzuweisenden Analyten ein messbares Reaktionsprodukt entstehen zu lassen; b) Durchführung der Signalverstärkungsreaktion; c) optische Messung der Reaktionsprodukte; und d) ggf. Bereitstellung der Messdaten für die Berechnung einer Analytkonzentration in einer Probe, und ist dadurch gekennzeichnet, dass vor der Durchführung der Signalverstärkungsreaktion auf dem Träger eine Mehrzahl räumlich getrennter Reaktionsräume eingerichtet wird, indem vor, während oder nach dem Aufbringen der Probe und der Reaktionslösung Mikropartikel auf dem Träger immobilisiert werden, so dass die Mikropartikel von der Reaktionslösung überschichtet sind und anschließend die Reaktionslösung bevorzugt durch Zentrifugation entfernt wird, so dass benachbarte, sich nicht berührende immobilisierte Mikropartikel nicht mehr von einer zusammenhängenden Schicht Reaktionslösung überdeckt sind.

Die Mikropartikel wirken als Nukleationspunkte für die Bildung der räumlich getrennten Reaktionsräume und weisen bevorzugt auf ihrer Oberfläche keine Binder auf. Die Probe wird vorzugsweise vor der Reaktionslösung auf den Träger aufgebracht. Bevorzugt erfolgt vor Durchführung der Signalverstärkungsreaktion in Schritt b) ein kontaktieren der Probe mit einem Signalverstärkungssystem, wobei das Signalverstärkungssystem ein erstes Element und ein zweites Element aufweist und wobei das erste Element spezifisch an den nachzuweisenden Analyten bindet und das zweite Element eine enzymatische Aktivität aufweist. Vorzugsweise ist das erste Element des Signalverstärkungssystems unmittelbar mit dem zweiten Element des Signalverstärkungssystems verbunden. Nach der Einrichtung der räumlich getrennten Reaktionsräume durch Entfernung der Reaktionslösung, z.B. durch Absaugen, und vor der Durchführung der Signalverstärkungsreaktion erfolgt vorzugsweise eine Stabilisierung dieser Reaktionsräume.

Vorzugsweise weist das Signalverstärkungssystem die Aktivität eines Enzyms auf. Bevorzugt handelt es sich bei dem ersten Element des Signalverstärkungssystems um einen Antikörper, an welchen das Enzym, z.B. eine Peroxidase, als zweites Element gebunden vorliegt. Die Reaktionslösung weist bevorzugt ein Substrat auf, welches durch die Umsetzung durch das Enzym ein Reaktionsprodukt ergibt, welches durch seine Absorption messbar ist, wobei es sich vorzugsweise um TMB als Substrat oder welches fluoresziert, wobei es sich bevorzugt um AmplifluRed oder TSA handelt.

Bei geringen Analytkonzentration in der Probe wird nur noch eine geringe Anzahl der Binder auf der Oberfläche des Trägers während der Inkubation mit der Probe besetzt. Werden z.B. 500 Binder von Analytmolekülen besetzt, so werden bei 1000 gebildeten Reaktionsräumen, die auf diesem Träger zusammengenommen 50 % der Fläche des Trägers überspannen, etwa 250 Reaktionsräume lediglich ein Analytmolekül enthalten welches vor dem Einrichten der Reaktionsräume ein Signalverstärkungssystem gebunden hat. Dieser Zusammenhang lässt sich dazu nutzen, bei schwach positiven Proben die Zahl der Analytmoleküle pro Probe zu bestimmen und dadurch die Genauigkeit des Tests zu erhöhen:

$$Na = FR_{Gesamt}/FoR \times NR_{pos}$$

$FR_{Gesamt}$     Fläche aller Reaktionsräume auf dem Träger

$FoR$     Fläche des Trägers ohne Reaktionsräume

$NR_{pos}$     Zahl positiver Reaktionsräume, die einen Analyten enthalten

Je mehr Analytmoleküle in der Probe sind, umso größer ist die Dichte gebundener Analytmoleküle/Nachweissysteme auf dem Träger. Das erhöht die Wahrscheinlichkeit, dass mehr als ein Analyt/Nachweissystem auf einen Reaktionsraum entfallen. Die Intensität der Nachweisreaktion pro Reaktionsraum wird bevorzugt dafür genutzt, die Zahl der Analytmoleküle pro Reaktionsraum zu bestimmen. Zur Kalibrierung der Intensität des Nachweissystems werden bevorzugt Referenz-Mikropartikel bevorzugt einer anderen, kodierten Mikropartikelpopulation mit vorgefertigter statistisch verteilter Zahl von Nachweissytemen auf deren Oberfläche eingesetzt. Diese Referenzierung kann intern auf den selben Träger oder aber auf negative Träger der gesamten Probenserie bzw. der Negativkontrolle wahlweise erfolgen. Der dynamische Messbereich des Testsystems wird bevorzugt durch die Erhöhung der Zahl der Reaktionsräume pro Träger vergrößert, was bevorzugt durch den Einsatz kleinerer Mikropartikel mit einem bevorzugten Durchmesser von 1-10 μm und besonders bevorzugt von 1-3 μm erreicht wird. Bevorzugt wird der Bereich des Testsystems, in dem die Zahl der Analytmoleküle gezählt werden können, in den durch die Anwendung des Tests geforderten Cut-off-Bereich gelegt. Diese Einstellung des Cut-off erfolgt durch die Veränderung der Testssensitivität bevorzugt durch die Veränderung der Anzahl der Binder pro Fläche des Trägers.

In einer weiteren Ausführungsform befinden sich die Binder, die spezifisch sind für die nachzuweisenden Analyten auf den Mikropartikeln.

In dieser bevorzugten Ausführungsform wird das erfindungsgemäße Verfahren für den Nachweis eines Analyten in einer Probe verwendet (Singleplex-Nachweis) und umfasst die Schritte a) Aufbringen einer Probe und einer Reaktionslösung auf einen Träger, wobei die Reaktionslösung geeignet ist in einer Signalverstärkungsreaktion in Abhängigkeit des Vorhandenseins des nachzuweisenden Analyten ein messbares Reaktionsprodukt entstehen zu lassen; b) Durchführung der Signalverstärkungsreaktion; c) optische Messung der Reaktionsprodukte; und d) ggf. Bereitstellung der Messdaten für die Berechnung einer Analytkonzentration in einer Probe, und ist dadurch gekennzeichnet, dass vor der Durchführung der Signalverstärkungsreaktion auf dem Träger eine Mehrzahl räumlich getrennter Reaktionsräume eingerichtet wird, indem vor, während oder nach dem Aufbringen der Probe und der Reaktionslösung Mikropartikel auf dem Träger immobilisiert werden, so dass die Mikropartikel von der Reaktionslösung überschichtet sind und anschließend die Reaktionslösung entfernt wird, so dass benachbarte, sich nicht berührende immobilisierte Mikropartikel nicht mehr von einer zusammenhängenden Schicht Reaktionslösung überdeckt sind.

Vorzugsweise wird zunächst die Probe auf den Träger aufgebracht und anschließend die Mikropartikel, welche die analyt-spezifischen Binder auf ihrer Oberfläche aufweisen, auf dem Träger immobilisiert, bevor die Reaktionslösung auf den Träger aufgebracht wird. Bevorzugt erfolgt vor Durchführung der Signalverstärkungsreaktion in Schritt b) ein kontaktieren der Probe mit einem Signalverstärkungssystem, wobei das Signalverstärkungssystem ein erstes Element und ein zweites Element aufweist und wobei das erste Element spezifisch an den nachzuweisenden Analyten bindet und das zweite Element eine enzymatische Aktivität aufweist. Vorzugsweise ist das erste Element des Signalverstärkungssystems unmittelbar mit dem zweiten Element des Signalverstärkungssystems verbunden. Nach der Einrichtung der räumlich getrennten Reaktionsräume durch Entfernung der Reaktionslösung, z.B. durch Absaugen und anschließende Zentrifugation, und vor oder während der Durchführung der Signalverstärkungsreaktion erfolgt vorzugsweise eine Stabilisierung dieser Reaktionsräume.

Vorzugsweise weist das Signalverstärkungssystem die Aktivität eines Enzyms auf. Bevorzugt handelt es sich bei dem ersten Element des Signalverstärkungssystems um einen Antikörper, an welchen das Enzym, z.B. eine Peroxidase, als zweites Element gebunden vorliegt. Die Reaktionslösung weist bevorzugt ein Substrat auf, welches durch die Umsetzung durch das Enzym ein Reaktionsprodukt ergibt, welches durch seine Absorption messbar ist, wobei es sich vorzugsweise um TMB als Substrat handelt oder welches fluoresziert, wobei es sich bevorzugt um AmplifluRed oder TSA handelt.

In einer bevorzugten Ausführungsform wird das erfindungsgemäße Verfahren für den Nachweis eines Analyten in einer Probe verwendet (Singleplex-Nachweis) und umfasst die Schritte a) Aufbringen einer Probe und einer Reaktionslösung auf einen Träger, wobei die Reaktionslösung geeignet ist in einer Signalverstärkungsreaktion in Abhängigkeit des Vorhandenseins des nachzuweisenden Analyten ein messbares Reaktionsprodukt entstehen zu lassen; b) Durchführung der Signalverstärkungsreaktion; c) optische Messung der Reaktionsprodukte; und d) ggf. Bereitstellung der Messdaten für die Berechnung einer Analytkonzentration in einer Probe, dadurch gekennzeichnet, dass vor der Durchführung der Signalverstärkungsreaktion auf dem Träger eine Mehrzahl räumlich getrennter Reaktionsräume eingerichtet wird, indem vor, während oder nach dem Aufbringen der Probe und der Reaktionslösung Mikropartikel auf dem Träger immobilisiert werden, so dass die Mikropartikel von der Reaktionslösung überschichtet sind und anschließend die Reaktionslösung entfernt wird, so dass benachbarte, sich nicht berührende immobilisierte Mikropartikel nicht mehr von einer zusammenhängenden Schicht Reaktionslösung überdeckt sind.

Vorzugsweise weist der Analyt eine enzymatische Aktivität auf, bevorzugt die Aktivität eines Enzyms wie z.B. einer Peptidase, die bei Kontakt mit der Reaktionslösung in einer Signalverstärkungsreaktion zu einem messbaren Reaktionsprodukt führt. Die Reaktionslösung weist bevorzugt ein Substrat auf, welches durch die enzymatische Aktivität des Analyten ein Reaktionsprodukt ergibt, welches durch seine Fluoreszenz messbar ist. Vorzugsweise handelt es sich bei dem Substrat um eine Peptidsequenz, welche einen Fluoreszenzfarbstoff und einen Quencher umfasst.

In einer weiteren bevorzugten Ausführungsform wird das erfindungsgemäße Verfahren für den Nachweis mehrerer Analyten in einer Probe verwendet (Multiplex-Nachweis) und umfasst die Schritte a) Aufbringen einer Probe und einer Reaktionslösung auf einen Träger, wobei die Reaktionslösung geeignet ist in einer Signalverstärkungsreaktion in Abhängigkeit des Vorhandenseins des nachzuweisenden Analyten ein messbares Reaktionsprodukt entstehen zu lassen; b) Durchführung der Signalverstärkungsreaktion; c) optische Messung der Reaktionsprodukte; und d) ggf. Bereitstellung der Messdaten für die Berechnung einer Analytkonzentration in einer Probe, dadurch gekennzeichnet, dass vor der Durchführung der Signalverstärkungsreaktion auf dem Träger eine Mehrzahl räumlich getrennter Reaktionsräume eingerichtet wird, indem vor, während oder nach dem Aufbringen der Probe und der Reaktionslösung verschiedene Mikropartikelpopulationen auf dem Träger immobilisiert werden, so dass die Mikropartikel von der Reaktionslösung überschichtet sind und anschließend die Reaktionslösung entfernt wird, so dass benachbarte, sich nicht berührende immobilisierte Mikropartikel nicht mehr von einer zusammenhängenden Schicht Reaktionslösung überdeckt sind.

Die Mikropartikelpopulationen weisen auf ihrer Oberfläche populationsspezifisch unterschiedliche Binder auf, die spezifisch sind für die nachzuweisenden Analyten. Bevorzugt sind die Mikropartikelpopulationen fluoreszenzkodiert, besonders bevorzugt weisen die Mikropartikelpopulationen eine unterschiedliche Fluoreszenzkodierung auf.

Vorzugsweise werden zunächst die Mikropartikel auf dem Träger immobilisiert, anschließend werden erst die Probe und dann die Reaktionslösung auf den Träger aufgebracht. Bevorzugt erfolgt vor Durchführung der Signalverstärkungsreaktion in Schritt b) ein kontaktieren der Probe mit einem Signalverstärkungssystem, wobei das Signalverstärkungssystem ein erstes Element und ein zweites Element aufweist und wobei das erste Element spezifisch an den nachzuweisenden Analyten bindet und das zweite Element eine enzymatische Aktivität aufweist. Vorzugsweise ist das erste Element des Signalverstärkungssystems unmittelbar mit dem zweiten Element des Signalverstärkungssystems verbunden. Nach der Einrichtung der räumlich getrennten Reaktionsräume durch Entfernung der Reaktionslösung, z.B. durch Absaugen, und vor der Durchführung der Signalverstärkungsreaktion erfolgt vorzugsweise eine Stabilisierung dieser Reaktionsräume.

Vorzugsweise weist das Signalverstärkungssystem die Aktivität eines Enzyms auf. Bevorzugt handelt es sich bei dem ersten Element des Signalverstärkungssystems um einen Antikörper, an welchen das Enzym, z.B. eine Peroxidase, als zweites Element gebunden vorliegt. Die Reaktionslösung weist bevorzugt ein Substrat auf, welches durch die Umsetzung durch das Enzym ein Reaktionsprodukt ergibt, welches durch seine Absorption messbar ist, wobei es sich vorzugsweise um TMB als Substrat handelt oder welches fluoresziert, wobei es sich bevorzugt um AmplifluRed oder TSA handelt.

In einer weiteren bevorzugten Ausführungsform wird das erfindungsgemäße Verfahren für den Nachweis mehrerer Analyten in einer Probe verwendet (Multiplex-Nachweis) und umfasst die Schritte a) Aufbringen einer Probe und einer Reaktionslösung auf einen Träger, wobei die Reaktionslösung geeignet ist in einer Signalverstärkungsreaktion in Abhängigkeit des Vorhandenseins des nachzuweisenden Analyten ein messbares Reaktionsprodukt entstehen zu lassen; b) Durchführung der Signalverstärkungsreaktion; c) optische Messung der Reaktionsprodukte; und d) ggf. Bereitstellung der Messdaten für die Berechnung einer Analytkonzentration in einer Probe, dadurch gekennzeichnet, dass vor der Durchführung der Signalverstärkungsreaktion auf dem Träger eine Mehrzahl räumlich getrennter Reaktionsräume eingerichtet wird, indem vor, während oder nach dem Aufbringen der Probe und der Reaktionslösung verschiedene Mikropartikelpopulationen auf dem Träger immobilisiert werden, so dass die Mikropartikel von der Reaktionslösung überschichtet sind und anschließend die Reaktionslösung entfernt wird, so dass benachbarte, sich nicht berührende immobilisierte Mikropartikel nicht mehr von einer zusammenhängenden Schicht Reaktionslösung überdeckt sind.

Die Mikropartikelpopulationen weisen auf ihrer Oberfläche populationsspezifisch unterschiedliche Binder auf, die spezifisch sind für die nachzuweisenden Analyten. Bevorzugt sind die Mikropartikelpopulationen fluoreszenzkodiert, besonders bevorzugt weisen die Mikropartikelpopulationen eine unterschiedliche Fluoreszenzkodierung auf.

Vorzugsweise werden zunächst die Mikropartikel auf dem Träger immobilisiert, anschließend werden erst die Probe und dann die Reaktionslösung auf den Träger aufgebracht. Bevorzugt erfolgt vor Durchführung der Signalverstärkungsreaktion in Schritt b) ein kontaktieren der Probe mit einem Signalverstärkungssystem, wobei das Signalverstärkungssystem ein erstes Element und ein zweites Element aufweist und wobei das erste Element spezifisch an den nachzuweisenden Analyten bindet und das zweite Element eine enzymatische Aktivität aufweist. Vorzugsweise ist das erste Element des Signalverstärkungssystems nicht unmittelbar mit dem zweiten Element des Signalverstärkungssystems verbunden. Das zweite Element ist bevorzugt unmittelbar mit einem weiteren Element verbunden. Nach der Einrichtung der räumlich getrennten Reaktionsräume durch Entfernung der Reaktionslösung, z.B. durch Absaugen, und vor der Durchführung der Signalverstärkungsreaktion erfolgt vorzugsweise eine Stabilisierung dieser Reaktionsräume.

Vorzugsweise weist das Signalverstärkungssystem die Aktivität eines Enzyms auf. Bevorzugt handelt es sich bei dem ersten Element des Signalverstärkungssystems um einen Antikörper, der beispielsweise biotinyliert ist. Bei dem weiteren Element handelt es sich bevorzugt um einen avidin-markierten Antikörper oder um ein avidin-markierten Mikropartikel, an welchem das Enzym, z.B. eine Peroxidase, als zweites Element gebunden vorliegt. Die Reaktionslösung weist bevorzugt ein Substrat auf, welches durch die Umsetzung durch das Enzym ein Reaktionsprodukt ergibt, welches durch seine Absorption messbar ist, wobei es sich vorzugsweise um TMB als Substrat handelt oder welches fluoresziert, wobei es sich bevorzugt um AmplifluRed oder TSA handelt.

In einer weiteren bevorzugten Ausführungsform wird das erfindungsgemäße Verfahren für den Nachweis mehrerer Analyten in einer Probe verwendet (Multiplex-Nachweis) und umfasst die Schritte a) Aufbringen einer Probe und einer Reaktionslösung auf einen Träger, wobei die Reaktionslösung geeignet ist in einer Signalverstärkungsreaktion in Abhängigkeit des Vorhandenseins des nachzuweisenden Analyten ein messbares Reaktionsprodukt entstehen zu lassen; b) Durchführung der Signalverstärkungsreaktion; c) optische Messung der Reaktionsprodukte; und d) ggf. Bereitstellung der Messdaten für die Berechnung einer Analytkonzentration in einer Probe, dadurch gekennzeichnet, dass vor der Durchführung der Signalverstärkungsreaktion auf dem Träger eine Mehrzahl räumlich getrennter Reaktionsräume eingerichtet wird, indem vor, während oder nach dem Aufbringen der Probe und der Reaktionslösung verschiedene Mikropartikelpopulationen auf dem Träger immobilisiert werden, so dass die verschiedenen Mikropartikelpopulationen von der Reaktionslösung überschichtet sind und anschließend die Reaktionslösung entfernt wird, so dass benachbarte, sich nicht berührende immobilisierte Mikropartikel nicht mehr von einer zusammenhängenden Schicht Reaktionslösung überdeckt sind.

Bevorzugt sind die Mikropartikelpopulationen fluoreszenzkodiert, besonders bevorzugt weisen die Mikropartikelpopulationen eine unterschiedliche Fluoreszenzkodierung auf. Vorzugsweise weisen die Mikropartikelpopulationen eine Streptavidinbeschichtung auf ihrer Oberfläche auf. Besonders bevorzugt weisen die Mikropartikelpopulationen auf ihrer Oberfläche zusätzlich noch biotinylierte Nukleinsäure-Bindersonden auf, welche komplementär sind zu den nachzuweisenden Analyten.

Vorzugsweise werden die Mikropartikelpopulationen gleichzeitig mit der Probe auf den Träger aufgebracht und immobilisiert, bevorzugt werden die Mikropartikelpopulationen mit der Probe gemischt und dann auf den Träger aufgebracht. Anschließend wird die Reaktionslösung auf den Träger aufgebracht. Bevorzugt erfolgt vor Durchführung der Signalverstärkungsreaktion in Schritt b) ein kontaktieren der Probe mit einem Signalverstärkungssystem, wobei das Signalverstärkungssystem ein erstes Element und ein zweites Element aufweist und wobei das erste Element spezifisch an den nachzuweisenden Analyten bindet und das zweite Element eine enzymatische Aktivität aufweist. Vorzugsweise ist das erste Element des Signalverstärkungssystems nicht unmittelbar mit dem zweiten Element des Signalverstärkungssystems verbunden. Das zweite Element ist bevorzugt unmittelbar mit einem weiteren Element verbunden. Nach der Einrichtung der räumlich getrennten Reaktionsräume durch Entfernung der Reaktionslösung, z.B. durch Absaugen, und vor der Durchführung der Signalverstärkungsreaktion erfolgt vorzugsweise eine Stabilisierung dieser Reaktionsräume.

Vorzugsweise weist das Signalverstärkungssystem die Aktivität eines Enzyms auf. Bevorzugt handelt es sich bei dem ersten Element des Signalverstärkungssystems um ein DNA-Oligonukleotid, welches beispielsweise biotinyliert ist. Bei dem weiteren Element handelt es sich bevorzugt um Avidin, besonders bevorzugt Extravidin, an welches das Enzym, z.B. eine Peroxidase, als zweites Element gebunden vorliegt. Die Reaktionslösung weist bevorzugt ein Substrat auf, welches durch die Umsetzung durch das Enzym ein Reaktionsprodukt ergibt, welches durch seine Absorption messbar ist, wobei es sich vorzugsweise um Luminol oder Lumigen ECL Ultra (Diarect) als Substrat handelt.

In einer weiteren bevorzugten Ausführungsform wird das erfindungsgemäße Verfahren für den Nachweis mehrerer Analyten in einer Probe verwendet (Multiplex-Nachweis) und umfasst die Schritte a) Aufbringen einer Probe und einer Reaktionslösung auf einen Träger, wobei die Reaktionslösung geeignet ist in einer Signalverstärkungsreaktion in Abhängigkeit des Vorhandenseins des nachzuweisenden Analyten ein messbares Reaktionsprodukt entstehen zu lassen; b) Durchführung der Signalverstärkungsreaktion; c) optische Messung der Reaktionsprodukte; und d) ggf. Bereitstellung der Messdaten für die Berechnung einer Analytkonzentration in einer Probe, dadurch gekennzeichnet, dass vor der Durchführung der Signalverstärkungsreaktion auf dem Träger eine Mehrzahl räumlich getrennter Reaktionsräume eingerichtet wird, indem vor, während oder nach dem Aufbringen der Probe und der Reaktionslösung Mikropartikel auf dem Träger immobilisiert werden, so dass die Mikropartikel von der Reaktionslösung überschichtet sind und anschließend die Reaktionslösung entfernt wird, so dass benachbarte, sich nicht berührende immobilisierte Mikropartikel nicht mehr von einer zusammenhängenden Schicht Reaktionslösung überdeckt sind.

Bevorzugt sind die Mikropartikelpopulationen fluoreszenzkodiert, besonders bevorzugt weisen die Mikropartikelpopulationen eine unterschiedliche Fluoreszenzkodierung auf.

Vorzugsweise weisen die Mikropartikelpopulationen eine Streptavidinbeschichtung auf ihrer Oberfläche auf. Besonders bevorzugt weisen die Mikropartikelpopulationen auf ihrer Oberfläche zusätzlich noch biotinylierte Primer auf, welche komplementär sind zu Teilbereichen der nachzuweisenden Analyten.

Vorzugsweise wird die Probe gleichzeitig mit der Reaktionslösung und den Mikropartikeln auf den Träger aufgebracht, bevorzugt wird die Probe mit der Reaktionslösung gemischt und dann die Mikropartikel hinzugefügt, bevor die Mischung auf den Träger aufgebracht wird und besonders bevorzugt werden erst die Mikropartikel auf dem Träger aufgebracht und immobilisiert, bevor mit der Probe und der Reaktionslösung gemeinsam oder nacheinander der Träger kontaktiert wird. Nach der Einrichtung der räumlich getrennten Reaktionsräume durch Entfernung der Reaktionslösung, z.B. durch Absaugen und Zentrifugation, und vor der Durchführung der Signalverstärkungsreaktion erfolgt vorzugsweise eine Stabilisierung dieser Reaktionsräume.

Vorzugsweise handelt es sich bei der Signalverstärkungsreaktion um eine PCR, bei der die Analyten vervielfältigt werden. Die Reaktionslösung weist bevorzugt ein Substrat auf, welches durch die Polymerase ein Reaktionsprodukt ergibt, welches durch seine Fluoreszenz messbar ist. Vorzugsweise handelt es sich bei dem Substrat um eine fluoreszenzmarkierte Sonde, z.B. eine TaqMan-Sonde.

Für die Temperierung von PCR und Schmelzkurvenanalysen wird bevorzugt ein Thermocycler eingesetzt wird, der folgende Eigenschaften aufweist:

- passgenaue Stellplätze für Reaktionsgefäße mit einem planaren Boden als Träger, wobei die Stellplätze nach unten offen sind, so dass eine simultane bildgebende oder nicht bildgebende Auswertung mit einem inversen optischen Messsystem möglich ist,

- reverse oder inverse Einstrahlung von Licht geeigneter Wellenlänge zur selektiven Temperierung der Reaktionslösung von der Annealingtemperatur bis zur Denaturierungstemperatur,

- Pelletier-Elemente- oder luftstrombasierte Temperierung der Reaktionsgefäße, der Reaktionslösung und ggf. der Trennlösung bei der Annealingtemperatur.

Die vorliegende Erfindung betrifft auch ein Kit zur Verwendung für die Durchführung des erfindungsgemäßen Verfahrens. Dazu kann das Kit Mikropartikel, Träger, ggf. ein Signalverstärkungssystem, eine Reaktionslösung, ggf. eine Trennflüssigkeit und eine Gebrauchsanweisung zur Verwendung des Kits und/oder zur Durchführung des erfindungsgemäßen Verfahrens enthalten. Die Mikropartikel können ggf. einen Binder auf ihrer Oberfläche aufweisen. Gegebenenfalls kann das Kit weitere Bestandteile zur Durchführung des erfindungsgemäßen Verfahrens enthalten. Solche Bestandteile können beispielsweise Reaktionsgefäße, Filter, Lösungen und/oder andere Mittel umfassen.

Außerdem bezieht sich die vorliegende Erfindung auf die Verwendung eines Kits zur in vitro Diagnose diverser Krankheiten wobei sich der Begriff "Diagnose" nicht nur auf die Feststellung bezieht ob eine Krankheit vorliegt oder nicht, sondern auch weitere Bedeutungen wie die allgemeine oder spezifische Prognose, die Bestimmung der Schwere der Erkrankung, das Stadium der Erkrankung oder ähnliches umfasst. Das Ergebnis des erfindungsgemäßen Verfahrens kann auch als Grundlage für die Optimierung der Behandlung genutzt werden, für den Ausschluss bestimmter anderer Krankheiten, für Entscheidungen über weitere Behandlungsmöglichkeiten etc.

Das erfindungsgemäße Verfahren kann bevorzugt verwendet werden für eine qualitative und quantitative Nukleinsäureamplifikation und -detektion, ultrasensitive reverse Hybridisierungsassays von DNA und RNA, ein Nukleinsäure-Sequenzscreening zur Detektion von Sequenzvariationen, ultrasensitive Immunoassays, Multiplex-Einzelzell-Assays in miniaturisierten Reaktionsräumen in vitro jeweils im Singleplex bzw. Multiplex-Format.

Weitere Vorteile des erfindungsgemäßen Verfahrens

Da die Mikropartikel mit beliebigen Bindern beschichtet werden können, ist es möglich unterschiedliche Arten von Bindern in einem Assay zu kombinieren. Beispielsweise können innerhalb eines Assays DNA-Moleküle an einer Mikropartikelpopulation und Proteinmoleküle an einer anderen Mikropartikelpopulation immobilisiert werden.

Für die Realisierung der Reaktionsraumbegrenzung als Voraussetzung der ultrasensitiven digitalen Detektion ist keine Mikrofluidik nötig und es sind keine teuren Reaktionsgefäße mit Nanokavitäten erforderlich.

Die Kombination aus Nanopartikeln mit begrenzten Reaktionsräumen erhöht die Reaktionsgeschwindigkeit der Analyten mit den Bindern auf den Trägern und die möglichst vollständige Immobilisierung aller Analyt Moleküle für eine möglichst hohe Sensitivität auch bei größeren Probenvolumina.

Getrennte und stark miniaturisierte Reaktionsräume erhöhen die Sensitivität bei der ortsaufgelösten Detektion von Signalverstärkungsreaktionen wie z.B. Enzymreaktionen beträchtlich und schränken störende Wechselwirkungen der Signalverstärkungsreaktionen wie z.B. bei der Real-time-PCR in verschiedenen Reaktionsräumen ein.

Die Kombination von teilweise oder vollständig begrenzten Reaktionsräumen mit fluoreszenzkodierten Mikropartikeln ist ein einfaches Verfahren, um ultrasensitive Multiplex-Testsysteme verfügbar zu machen.

Die Kombination aus Einzelmolekülzählung und Erfassung des Summensignals garantiert einen besonders großen Messbereich, wodurch ein Titrieren von Proben mit höheren Analytkonzentrationen vermieden werden kann.

Die Kombination aus Fluoreszenzdetektion und Absorptionslöschung durch ein EnzymSubstrat ist extrem sensitiv, da Fluoreszenzanregung und -emission gleichzeitig durch ein Substrat gelöscht werden können.

Sensitive Fluoreszenz-, Absorptions- und Lumineszenz-Detektion kann bei Verwendung von Standardreagenzien der Labordiagnostik bis in den Einzelmolekülbereich zur parallelen Bestimmung unterschiedlichster Analyten genutzt werden.

Es ist möglich Reaktionskinetiken zu erfassen, wodurch die Qualität der Analyse erweitert wird.

Durch Verwendung von Standardverbrauchsmaterialien, wie 96-Well-Mikrotestplatten ist die Kompatibilität der verschiedenen Testsysteme mit der Laborautomatisierung im Routinelabor gewährleistet.

Auf Grund der Vielfalt möglicher Reportersysteme kann das Verfahren an die unterschiedlichsten Detektionssysteme angepasst werden.

Die Kleinheit der Reaktionsräume erlaubt besonders schnelle Temperierungszyklen und damit kurze PCR-Protokolle.

Nachfolgend wird die Erfindung anhand von Zeichnungen und Ausführungsbeispielen näher erläutert, wobei sie nicht darauf beschränkt werden soll.

**Figuren**

Es wurden jeweils 3 Fluoreszenzbilder (Mikropartikelkodierung: blau, grün; Signalverstärkungsreaktion (z.B. fluorochrommarkierter Antikörper: rot) mit dem FI100 Fluorszenzimager aufgenommen.

Figur 1      Herstellung von räumlich getrennten Reaktionsräumen. Mikropartikel (grüne Fluoreszenz, Pfeil) wurden auf einer Mikrotestplatte als planarem Träger durch Adsorption immobilisiert und danach mit Reaktionslösung überschichtet, die Cy5 (rote Fluoreszenz, gestrichelter Kreis) enthält. Danach wurde die Reaktionslösung aus den Kavitäten der Mikrotestplatte gründlich abgesaugt. Der planare Träger wurde mit Mineralöl überschichtet (A) bzw. 1 min bei 1000 rpm mit senkrecht zur Oberfläche wirkender Zentrifugalkraft zentrifugiert und dann mit Mineralöl überschichtet (B).

             Durch Zentrifugation gelingt es, die Reaktionslösung stärker in unmittelbarer Nähe des Mikropartikels zu konzentrieren und auf dadurch die Reaktionsräume mehrheitlich voneinander zu trennen.

Figur 2      Auf der Oberfläche avidinbeschichteter Mikropartikel wurde ein 5'-biotinyliertes und 3'-FITC-markiertes Oligonukleotid gebunden (A) bzw. nicht gebunden (B). Nach dem Waschen des ungebunden Überschusses wurden beide Reaktionsansätze mit Anti-FITC-Peroxidase-IgG inkubiert und nach erneutem Waschen mit Reaktionslösung, die TMB, $H_2O_2$ und Cy5 enthält, überschichtet. Nach kurzer Einwirkzeit wurde die Reaktionslösung abgesaugt und anschließend aus der Kavität herauszentrifugiert und beide Reaktionsansätze wurden mit Öl überschichtet. Die Messung im FI100 Fluoreszenzimager erfolgte nach 7 min Signalverstärkungsreaktion. Dargestellte Fluoreszenzintensitäten aus den Messkanälen rot und blau (A) bzw. rot (B).

             In Reaktionsansatz A wurde die Cy5-Fluoreszenz vollständig gelöscht, da das am Partikel (Pfeilspitzen) über Avidin gebundene Oligonukleotid mit Anti-FITC-Peroxidase-IgG markiert wurde. Nach Zugabe der Reaktionslösung wurde das farblose TMB in blaues Reaktionsprodukt umgewandelt. Da Reaktionsansatz B nicht mit dem Oligonukleotid inkubiert wurde, erfolgte keine Löschung, so dass die Ausdehnung der Reaktionsräume durch die starke C5-Fluoreszenz gut sichtbar blieb.

             Kleine Pfeile: konfluente Reaktionsräume, große Pfeile: getrennte Reaktionsräume.

Figur 3      Auf der Oberfläche der Mikropartikel von 2 Mikropartikelpopulationen, die sich in ihrer Größe und Fluoreszenzkodierung unterscheiden (Fig. A) wurden 3'-biotinyliertes und 5'-FITC-markiertes Oligonukleotid (Population 1: dunkle, mit Avidin beschichtete Partikel [Pfeile]) und humanes Serumalbumin (Mikropartikelpopulation 2: helle Partikel [Pfeilspitzen]) als Negativkontrolle gebunden, bevor 500 Mikropartikel der Population 1 und 2 gemeinsam in einem Well einer Mikrotestplatte immobilisiert wurden. Danach wurde das Well mit einem Anti-FITC-Peroxidase-Konjugat inkubiert. Nach dem Waschen des Wells mit PBS-T wurde die Waschlösung abgesaugt und der Rest durch Zentrifugation entfernt. Unmittelbar danach erfolgte die Überschichtung mit 0,5 % Agarose, die ebenfalls abgesaugt und durch Zentrifugation entfernt wurde. Nach dem Erkalten der Agarose wurde das Well mit TSA-Cy5-Peroxidase-Substrat als Reaktionslösung überschichtet und 1 h inkubiert. Danach wurde mehrmals gewaschen, um ungebundenes Substrat zu entfernen.

Die bildgebende Erfassung der Partikelfluoreszenzen wurde in Fig. A und der Analytfluoreszenzen in Fig. B dargestellt. Diese Messwerte als auch die Abbildung in Fig. B belegen, dass das Verfahren zur Einrichtung von Reaktionsräumen geeignet ist, nach Aktivierung kovalent bindende, fluoreszierende Substrate spezifisch für die Analytdetektion an verschiedenen Mikropartikelpopulationen im Multiplexansatz zu detektieren. Die Reaktionsräume selbst sehr dicht nebeneinander liegender Mikropartikel verschiedener Populationen sind vollständig voneinander getrennt (Mikropartikel im gestrichelten Kreis).

Figur 4   Auf der Oberfläche der Mikropartikel von 2 Mikropartikelpopulationen, die sich in ihrer Größe und Fluoreszenzkodierung unterscheiden (Fig. A) wurden humanes IgG (Population 1: große Partikel [Pfeile]) und ein DNA-Oligonukeotid (Mikropartikelpopulation 2: kleine Partikel [Pfeilspitzen]) als Negativkontrolle gebunden, bevor 500 Mikropartikel der Population 1 und 2 gemeinsam in einem Well einer Mikrotestplatte immobilisiert wurden. Danach wurde das Well mit einem Anti-human-Immunglobulin-alkalische Phosphatase-Konjugat inkubiert. Nach dem Waschen des Wells mit PBS-T wurde die Waschlösung durch DDAO-Phosphat-Substrat als Reaktionslösung ersetzt und diese nach 15sec abgesaugt und der Rest durch Zentrifugation entfernt. Unmittelbar danach erfolgte die Überschichtung mit Mineralöl sowie die Messung mittels Videoscan nach 8min Substratinkubation.

Die bildgebende Erfassung der Partikelfluoreszenzen wurde in Fig. A und der Analytfluoreszenzen in Fig. B dargestellt. Die relative Fluoreszenzintensität der Analytfluoreszenz betrug entsprechend der Messwerte der FastFluoscan-Software 14,3 für Mikropartikelpopulation 1 und 1,1 für Mikropopulation 2. Durch Referenzierung der Analytfluoreszenzen auf den Mikropartikeldurchmesser können Messfehler vermieden werden. Diese Messwerte als auch die Abbildung in Fig. B belegen, dass das Verfahren zur Einrichtung von Reaktionsräumen geeignet ist, lösliche, fluoreszierende Substrate spezifisch für die Analytdetektion an verschiedenen Mikropartikelpopulationen im Multiplexansatz zu detektieren.

Figur 5   Darstellung der Wirkung der Reaktionsraumbegrenzung durch Sephadex G30 als Diffusionsbarriere zwischen Mikropartikel, die unterschiedliche Reaktionsräume begrenzen. Es wurden Mikropartikel unterschiedlicher Populationen gemischt, wobei eine Population (Mikropartikel in der Mitte des Bildes, Pfeil) einen fluorochrommarkierten Antikörper an seiner Oberfläche gebunden hat und andere Mikropartikelpopulationen (Pfeilspitze) nicht. Durch Dissoziation mittels Detergenz werden die fluorochrommarkierten Antikörper von der Mikropartikeloberfläche abgelöst und können so vom Reaktionsraum weg diffundieren. Dabei färben sie die benachbart liegenden Sephadexkugeln an, mit denen die Mikropartikel überschichtet wurden. Auch nach zwei Tagen hat die Diffusionsfront noch nicht die in der Nähe liegenden Mikropartikel anderer Mikropartikelpopulationen (Pfeilspitzen) erreicht.

Figur 6   Getrennte Reaktionsräume (1 und 2), die mit Enzymsubstratlösung gefüllt sind und mit Mineralöl (4) überschichtet wurden. Planarer Träger (3), Mineralöl (4), Partikel (5).

Figur 7   Die je etwas 300 Mikropartikel der Mikropartikelpopulationen 1 und 2 wurden im Well A und die Mikropartikelpopulation 1 im Well B einer Mikrotestplatte immobilisiert. Die Mikropartikel tragen jeweils einen spezifischen (Mikropartikelpopulation 1) bzw. einen unspezifischen Binder (Mikropartikelpopulation 2) auf ihrer Oberfläche und wurden mit einem Atto 647N-markierten Analyten, der spezifisch an Mikropartikel der Population 1 binden, inkubiert. Danach wurden die Well A die Reaktionsräume unter Verwendung von Pufferlösung als Reaktionslösung um jeden Mikropartikel eingerichet und in Well B nicht. Die Mikropartikel in Well B wurden mit 50 μl Pufferlösung überschichtet. Im Videoscann-Cycler wurde eine Schmelzkurve von 40-95°C gefahren für beide Wells gefahren. Pro Meßpunkt in Diagramm A wurden etwa 300 Reaktionsräume mit dem Ringfluoreszenzalgorithmus gemessen.

Fig. 7A: Die Reaktionsräume um Mikropartikelpopulation 1 zeigen eine maximale Analytfluoreszenz in Well A während Mikropartikelpopulation 2 ohne spezifischen Binder keine Analytfluoreszenz aufweist. Mit Erhöhung der Temperatur verringert sich die Analytfluoreszenz von Mikropartikelpopulation 1 initial um etwa 30 % (unspezifisches Quenching vermittelt über Sekundärstruktuen) um ab etwa 50°C wieder den Maximalwert zu erreichen. Ab etwa 65°C fällt das Signal um insgesamt etwa 25 % bis zum Erreichen der Endtemperatur von 95°C. Die negative Mikropartikelpopulation bleibt negativ auch wenn die Reaktionsräume zum Teil sehr dicht beieinander in Well A liegen (Daten nicht gezeigt). Die Verringerung des Gesamtsignals in Well A wird durch Bleaching verursacht.

Fig. 7B: In Well B verringert sich das Signal bis 55°C komplett mit einer Tm von etwa 48°C, da das der dehybridisierende Atto 647N-markierte Analyt (DNA-Sequenzfragment des Plasmids pSNAP) sich in 50 μl Pufferlösung zu stark verdünnen kann. Dadurch kann gezeigt werden, dass es gelingt, Reaktionsräume einzurichten, die trotz Hitzedenaturierung von Binder und Analyt den Analyt im Reaktionsraum zurückhalten.

Figur 8     Um die Funktionalität der Reaktionsräume für thermocyclische Reaktionen zeigen zu können, wurden in die Reaktionslösung zwei komplementäre Oligonukleotide in einer Konzentration von 1 μM gegeben. Eines der Oligonukleotide hat am 3'-Ende einen Quencher und das komplementäre Oligonukleotid am 5'-Ende Atto647N als Fluorophor gebunden. Die Schmelztemperatur des Hybrids liegt bei 55 °C. Durch Zweipunktmessung der Analytfluoreszenz bei 45 °C bzw. 70 °C unter thermocyclischen Reaktionsbedingungen kann gezeigt werden, dass die komplementären Oligonukleotide hybridiseren bzw. dehybridiseren. Im hybridisierten Zustand wird der Quencher in räumliche Nähe zum Fluophor gebracht, so dass dieser effektiv die Fluoreszenz von Attom647N löschen kann. Im dehybridisierten Zustand ist dies nicht möglich, so dass ein Atto647N-Signal messbar ist. Dieses Reaktionsprizip kann universell auf eine Vielzahl unterschiedlicher Nachweissysteme angewendet werden.

Figur 9     Erkennung und/oder Individualisierung einzelner Reaktionsräume und/oder einzelner Mikropartikel mittels Ranking-Booster Algorithmus. Ausgangsbild mit unterschiedlich hellen Mikropartikeln. Schematische Darstellung der Mikropartikel (links). Reale Bildszene einer Graustufen-Aufnahme mit agglomerierten Mikropartikeln (rechts).

Figur 10     Erkennung und/oder Individualisierung einzelner Reaktionsräume und/oder einzelner Mikropartikel mittels Ranking-Booster Algorithmus. links: schematische Darstellung des erkannten agglomerierten Objekts; rechts: die drei mit dem konventionellen Algorithmus erkannten Objekte in realer Szene (mit verschiedenen Mustern markiert).

Figur 11     Erkennung und/oder Individualisierung einzelner Reaktionsräume und/oder einzelner Mikropartikel mittels Ranking-Booster Algorithmus. Relativ-Rang-Bild (links: schematische Darstellung; rechts: reale Beispielbildszene). Das Relativ-Rang-Bild wird bevorzugt mit einer festen Schwelle binarisiert (z.B. Schwellwert 0.5), da die absoluten Helligkeiten der Mikropartikel nach dem Ranking-Booster Algorithmus keine Rolle mehr spielen. In dem entstandenen Binärbild ist die Zentrumsregion jedes Mikropartikels als diskrete kreisrunde Scheibe erkennbar, weil der Mittelpunkt eines Mikropartikels (im Originalbild) immer dessen hellstes Pixel ist und die Pixel-Helligkeiten radial abfallen. Die Scheiben beinhalten, abhängig vom gewählten Schwellwert, bevorzugt etwa nur die Hälfte der Fläche eines Mikropartikels und können sich deshalb nicht untereinander berühren. Mittels Objekterkennung werden die Zentrums-Scheiben identifiziert. Sonstige sichelförmige oder dreieckige kleine Objekte in den Zwischenräumen sind Artefakte, die an ihrer nichtrunden Form und zu geringer Fläche erkennbar sind. Diese Artefakte werden ersatzlos verworfen.

Figur 12     Erkennung und/oder Individualisierung einzelner Reaktionsräume und/oder einzelner Mikropartikel mittels Ranking-Booster Algorithmus. Binarisiertes Relativ-Rang-Bild vor (oben) und nach (unten) Artefakt-Filterung mit verbleibenden Zentrums-Scheiben (markiert mit Schachbrettmuster). Schematische Darstellung (links); reales Beispielbild mit erkannten Objekten, die mit verschiedenen Mustern markiert sind (rechts).

Figur 13     Erkennung und/oder Individualisierung einzelner Reaktionsräume und/oder einzelner Mikropartikel mittels Ranking-Booster Algorithmus. Je drei vollständige Inflations-Iterationen (noch ohne Kontakt) (markiert mit verschiedenen Linienmustern) um jede Zentral-Scheibe (markiert mit Schachbrettmuster).

Figur 14     Erkennung und/oder Individualisierung einzelner Reaktionsräume und/oder einzelner Mikropartikel mittels Ranking-Booster Algorithmus. Versuch einer weiteren Inflations-Iteration führt zu Kontakt (markiert mit Rautenmuster) und so zum Abbruch der Iteration für dieses Mikropartikel

Figur 15     Erkennung und/oder Individualisierung einzelner Reaktionsräume und/oder einzelner Mikropartikel mittels Ranking-Booster Algorithmus. Ergebnis des Algorithmus: schematische Darstellung von drei gefundenen diskretisierten Mikropartikeln, die mit verschiedenen Mustern markiert sind (links); reale Mikropartikel als diskrete Objekte, die mit verschiedenen Mustern markiert sind (rechts).

Figur 16     Erkennung und/oder Individualisierung einzelner Reaktionsräume und/oder einzelner Mikropartikel und Bestimmung der Signalintensität mittels Dual-Octagon-Algorithmus. Mehrkanalige Fluoreszenzaufnahmen von Mikropartikeln (Objektdetektionskanal):

A) Aufnahme im Objekt-Detektionskanal mit Markierungen des detektierten Objektes in diesem Kanal,
B) Aufnahme im zu detektierender Helligkeitskanal: Idealzustand ohne Verschiebung (zusätzlich im Bild gekennzeichnet sind die Markierungen des Objekt-Detektionsbereiches),

C) Aufnahme im zu detektierender Helligkeitskanal: Unschärfe im Helligkeitskanal durch chromatische Abberation (zusätzlich im Bild gekennzeichnet sind die Markierungen des Objekt-Detektionsbereiches),

D) Aufnahme im zu detektierender Helligkeitskanal: laterale Verschiebung aufgrund von Objektbewegung oder chromatischer Abberation zwischen den Aufnahmen im Detektions- und Helligkeitskanal (zusätzlich im Bild gekennzeichnet sind die Markierungen des Objekt-Detektionsbereiches).

Figur 17    Erkennung und/oder Individualisierung einzelner Reaktionsräume und/oder einzelner Mikropartikel mittels Dual-Octagon-Algorithmus. Schematische pixelweise Darstellung eines Objekts mit Grauwertangaben.

**Beispiele**

Inkubation der Probe

Die Inkubation der Probe mit dem binder-beschichteten Träger bzw. den binder-beschichteten Mikropartikeln erfolgt bevorzugt in einem Probenpuffer, damit die Binder spezifisch die Analyten binden. Die unspezifische Bindung des Analyten führt unweigerlich zu einer schlechteren unteren Nachweisgrenze, so dass Zusätze wie Detergenzien oder Blockproteine, die Variation der Ionenstärke des Inkubationspuffers sowie des pH-Wertes des Probenpuffers auf den jeweiligen Analyten abgestimmt werden müssen, um unspezifische Bindungen zu minimieren. Um in diesem Zusammenhang eine zu starke Verdünnung der Probe zu vermeiden, werden bevorzugt 10x konzentrierte Probenpuffer eingesetzt. Bevorzugt erfolgt die Inkubation der Probe in Kavitäten von Mikrotestplatten mit einem im Wesentlichen planarem, binder-beschichteten Kavitätenboden oder mit binder-beschichteten, am Kavitätenboden immobilisierten Mikropartikeln.

Träger

Besonders bevorzugt werden Mikrotiterplatten aus Polymer, wie z.B. NucleoLink-Platten (Nunc) für die Nukleinsäureamplifikation eingesetzt, die eine Oberflächen-Beschichtung für die kovalente Immobilisierung von Biomolekülen tragen, einen ausreichend planaren und transparenten Gefäßboden besitzen und thermostabil sind. Oberflächenbeschichtungen können auch eingesetzt werden, um die unspezifische Bindung von Probenmolekülen oder Detektionsreagenzien zu verringern. Eine Mikrostrukturierung der Träger-Oberfläche, die die optische Fokussierung nicht beeinträchtigt, ist natürlich möglich. Die Oberflächen der Träger können neben Vertiefungen weitere Strukturen mit unterschiedlichen Eigenschaften wie bevorzugt hydrophile oder hydrophobe Eigenschaften aufweisen.

Die Mikroarrays können in Form eines Objektträgers oder einer Mikroplatte (ebenfalls als Mikrotiterplatte bezeichnet) vorliegen. Bei der Mikroplatte handelt es sich um einen geschüsselten (dished) Behälter mit mehreren (mindestens zwei) Vertiefungen. Bei auf Mikroplatten basierenden Mikroarrays handelt es sich um eine Mikroplatte mit mehreren Vertiefungen, in deren Böden der Mikroarray-Biochip platziert ist. Ein Beispiel der Mikroplatte ist die bekannte ELISA Mikrotiterplatte mit 96 Vertiefungen.

Weiterhin sind auf selbst-anordnenden Schichtsystemen basierende feste Träger ebenso für die Implementierung der vorliegenden Erfindung geeignet. Die Anwendung kann unter Verwendung automatischer Verfahren erfolgen.

Binder

Als Binder werden vorzugsweise solche Stoffe eingesetzt, die spezifisch den Analyten aus der Probe oder der Referenzprobe mit hoher Sensitivität und Spezifität binden oder die mit dem Analyten um die Bindung eines Analogons konkurrieren. Alternativ können auch synthetische Affinitätsrezeptoren, die z.B. durch molekulares Prägen (Molecular Imprinting) hergestellt wurden, verwendet werden. Binder einer Sorte besitzen alle dieselbe Bindungsspezifität für den Analyten wie z.B. monoklonale Antikörper oder sie unterscheiden sich in Ihrer oder Epitopspezifität. Sie können für denselben oder für unterschiedliche Isoformen desselben Analyten spezifisch sein. Binder und Analogon bzw. Analyt können Fluoreszenzmarkierungen tragen. Vorzugsweise sind die verschiedenen Binder unterschiedlich (fluoreszenz-) markiert oder ein Binder ist beispielsweise (fluoreszenz) markiert und der andere Binder ist unmarkiert. Auf einem Mikropartikel können sich verschiedene Binder befinden, die jeweils zum Nachweis spezifischer Analyte geeignet sind. Damit ist es möglich, dass auf einem Mikropartikel der Nachweis mehrerer Analyten erfolgen kann.

Reaktionsraumbegrenzung

Die Schichtdicke als Ausdehnung des Reaktionsraumes senkrecht zur Oberfläche des Trägers und die Homogenität des Flüssigkeitsfilmes kann durch die Veränderung der Viskosität der Reaktionslösung beispielsweise durch Zusatz von 0,5-10% Glycerol und oder 0,01-1% Tween 20 verändert werden, sofern dadurch die Signalverstärkungsreaktion nicht zu stark inhibiert wird. Die Homogenität der Schichtdicke des Flüssigkeitsfilmes kann auch gezielt durch die Aufbringung von Mikropartikeln oder unterschiedlich hydrophober/hydrophiler Bereiche auf dem Träger gezielt beeinflusst werden.

Die Schichtdicke des Reaktionsraumes entspricht ca. dem Durchmesser der Mikropartikel. Eine laterale Begrenzung des Reaktionsraumes senkrecht zur Schichtdicke wird nur partiell durch die Mikropartikel selbst verursacht und verringert Flüssigkeitsströmungen im Reaktionsraum. Dennoch breiten sich die Produkte der Signalverstärkungsreaktion im Reaktionsraum durch Diffusion und Strömungen aus. Die Diffusion des Reaktionsproduktes kann dadurch beeinflusst werden, indem z.B. präzipitierender Substrate oder Substrate gewählt werden, deren Reaktionsprodukt chemisch durch die Reaktion aktiviert wurde und an den Träger oder an Mikropartikel bindet.

Ist die Analytkonzentration z.B. so hoch, dass jeder Mikropartikel ein oder mehrere Analyten trägt, dann wird das Signal des Reaktionsproduktes homogen über den ganzen Reaktionsraum verteilt und je nach Detektionsprinzip zu- oder abnehmen. Ist die Analytkonzentration niedrig, dauert es länger bis der gesamte Reaktionsraum homogen durch das Signal des Reaktionsproduktes ausgefüllt wird.

Die vollständige laterale Begrenzung des Reaktionsraumes wird durch eine weitere bevorzugte Ausführungsform, bei der die Mikropartikel einzeln auf dem Träger, mit einem durchschnittlichen Abstand der Mikropartikel, der bevorzugt dem 4-fachen Durchmesser der Mikropartikel entspricht, immobilisiert werden. Je weiter der Abstand zwischen den Mikropartikeln desto vollständiger ist die laterale Trennung des Reaktionsraumes eines Mikropartikels vom Reaktionsraum benachbarter Mikropartikel, wenn die Reaktionslösung mittels Pipette abgesaugt wird. Durch das Absaugen und ggf. sich anschließender Zentrifugation fällt der Flüssigkeitsfilm zwischen den Mikropartikeln und zieht sich durch die wirkenden Kapillarkräfte weitgehend in den Spalt zwischen Mikropartikel und Träger zurück bzw. am Mikropartikel nach oben (siehe Figur 3). Die laterale Trennung der Reaktionsräume einzelner Mikropartikel wird durch den Einsatz von Trägern mit hydrophoben Oberflächen und/oder durch die Verwendung detergenzien-freier Reaktionslösungen verbessert.

Teilweise oder vollständig voneinander getrennte Reaktionsräume können erfindungsgemäß auch als Mikrozellkulturkammern eingesetzt werden, um bevorzugt die Eigenschaften einzelner oder weniger eukaryotischer oder prokaryotischer Zellen zu charakterisieren. Dabei ist es möglich Mikropartikel und Signalverstärkungssysteme gemeinsam mit den Zellen in einem Reaktionsraum einzubringen, um in einem homogenen Testformat z.B. die Sekretion von Zytokinen in den Reaktionsraum hinein zu untersuchen. Verteilt sich statistisch gesehen nur eine Zelle auf einen Reaktionsraum, so kann die Sekretionsleistung auf Einzelzellniveau erfasst werden. Wichtig ist, dass die Reaktionslösung eine Zusammensetzung besitzt, die ein möglichst langes Überleben der Zellen im Reaktionsraum gewährleistet. Die Zellen können als Suspension der Reaktionslösung zugegeben, vorab am Träger oder am Mikropartikel adhäriert oder kultiviert werden. Die Überschichtung des Reaktionsraumes kann durch eine Trennflüssigkeit oder durch ein Gel, wie z.B. Hydrogel oder Kollagengel, erfolgen. Ein derartiges Gel kann zusätzlich mit Nährstoffen angereichert sein, um ein möglichst langes Überleben der Zellen im Reaktionsraum zu gewährleisten. Es ist ebenso möglich, ein Doppelkammer-Kultursystem einzusetzen, deren Träger vorzugsweise mikroporös ist und der von einer Seite, bevorzugt von der abluminalen Seite der Kavität mit Nährlösung für die Zellen umspült wird, so dass die Nährstoffe durch den porösen Träger zu den Zellen diffundieren können. Mikropartikel, die sich mit den Zellen in den Reaktionsräumen befinden können z.B. fluoreszenzkodiert sein oder beispielsweise populationsspezifisch mit z.B. unterschiedlichen Wachstumsfaktoren, Extrazellularmatrixfaktoren oder Zellen, wie Feeder-Zellen oder zu analysierenden Zellen beschichtet sein. Zellen, die sich auf den Mikropartikeln befinden können als Sensoren für Wachstumsfaktoren aus der Probe dienen. Bevorzugt werden exprimierte fluoreszierende Marker wie z.B. das green-fluorescent-protein (GFP) und Derivate davon oder FURA-2 als $Ca^{2+}$-Marker in den Zellen als Indikatoren für zelluläre Prozesse eingesetzt, so dass diese Prozesse mithilfe des erfindungsgemäßen Mehrfarbenfluoreszenz-Messsystem gemessen werden können.

## Signalverstärkung

Werden zwei verschiedene Enzyme, die Bestandteil unterschiedlicher Signalverstärkungssysteme sind, miteinander kombiniert, die unterschiedlich absorbierende Reaktionsprodukte bilden, dann ist es möglich das Löschen von zwei verschiedenen Fluoreszenzfarbstoffen zum Duplex-Nachweis zu nutzen. Alternativ ist es möglich, dass als erstes Signalverstärkungssystem bevorzugt HRP in Kombination mit TMB eingesetzt wird und als zweites Signalverstärkungssystem bevorzugt eine Nuklease. Während die HRP ultrasensitiv einen roten Fluoreszenzfarbstoff löscht, wird z.B. durch eine Nuklease wie die RNASe-freie DNASe I eine Fluoreszenzlöschung eines Fluoreszenzfarbstoffes aufgehoben der bei einer Wellenlänge zwischen 450-550nm angeregt wird bzw. emittiert. Dies geschieht dadurch, dass die Nuklease bevorzugt ein Oligonukleotid spaltet, das an einem Ende mit einem Fluoreszenzlöscher, wie z.B. Dabcyl, und am anderen Ende ein Fluoreszenzfarbstoff wie z.B. Fluorescein gebunden hat. Damit ist es möglich in einem Ansatz zwei Analyten bzw. einen Analyten und einen internen Standard gleichzeitig in einem Reaktionsraum zu detektieren. Ein interner Standard gibt Informationen darüber, ob der Nachweis bei Proben, die keinen Analyten enthalten, ordnungsgemäß funktioniert hat. Ist der interne Standard ein in jeder Probe vorhandener Analyt, wie z.B. ein Referenzgen (o.a. House-keeping-Gen), kann die Konzentration des weiteren Analyten darauf referenziert werden. Während als Maß für die Detektion des internen Standards im Reaktionsraum eine Fluoreszenz bei einer Wellenlänge von 520nm bei dieser Ausführungsvariante des erfindungsgemäßen Verfahrens entsteht, wird die Fluoreszenz eines roten Fluoreszenzfarbstoffes als Bestandteil der Reaktionslösung im Reaktionsansatz als Maß für einen weiteren Analyten gelöscht. Avidin-Nuklease-Konjugate zur Bindung an biotinylierte Nachweisantikörper oder biotinylierte Fangsonden werden bevorzugt in Immunoassays oder in RNA-Hybridisierungsassays eingesetzt. Alternativ werden bevorzugt sequenzspezifische Peptidasen und die entsprechenden spezifischen Quencher-/Fluorochrom-markierten Peptide oder sequenzspezifische DNasen und deren markierte Substrate in Immunoassays und DNA-Hybridisierungsassays verwendet, um während der Reaktion eine Fluoreszenz zu generieren.

Messsysteme

Geräte zur erfindungsgemäßen Durchführung und Messung von Signaländerungen vor, während oder nach der Durchführung der verschiedenen Ausführungsformen des erfindungsgemäßen Verfahrens, die vollautomatisch mittels Computer steuerbar sind, um ortsaufgelöst bzw. bildgebend Fluoreszenzen, Lumineszenzen oder Absorptionen zu messen, umfassen: eine vollautomatische Steuerung mittels Computer, eine Positioniereinrichtung für den Träger, eine dem Reaktionsbereich zugeordnete Beleuchtungseinrichtung mit denen Anregungslicht unterschiedlicher, definierter Wellenlängen strahlbar ist, eine Optikeinrichtung die vorzugsweise für die reverse und inverse Fluoreszenz-, Absorptions- und/oder Lumineszenz-Detektion mit entsprechenden optischen Filtern oder Multibandfiltern geeignet ist, eine oder mehrere Detektoreinrichtung(en) wie z.B. CCD oder CMOS), die in Abhängigkeit von einer gemessenen Signalintensität Bilder erzeugt; und eine Auswerteeinheit, die aus den Bildern Messwerte erzeugt.

Für den Einsatz von Mikropartikeln können die Geräte zusätzlich Immobilisierungseinrichtungen für z.B. magnetische oder paramagnetische Mikropartikel umfassen. Die Geräte können optional einen Thermocycler enthalten, der den planaren Träger aufnimmt und die Durchführung von real-time-PCR bzw. isothermischer Amplifikationen bei gleichzeitiger reverser oder inverser fluoreszenzoptischer Analyse der Träger ermöglichen. Der Thermocycler umfasst bevorzugt 1-384 und besonders bevorzugt 8-96 passgenaue Stellplätze für Reaktionsgefäße. Durch die kompakte Bauweise des Thermocycler und der nach unten offenen Stellplätze ist es bevorzugt möglich, den Bereich der inneren Oberfläche des Bodens der Reaktionsgefäße, in dem sich die Reaktionsräume befinden, während des Temperiervorganges und bevorzugt während isothermischer Phasen von PCR-Zyklen wie z.B. am Ende der Annealingphase oder isothermischer Phasen von Schmelzkurvenanalysen, Messwerte bildgebend oder nicht bildgebend aufzuzeichnen. Zusätzlich kann das Gerät über Pump- und Pipettiervorrichtungen ergänzt werden, mit denen automatisiert Substrate, Probenmaterial oder andere Reaktionskomponenten zugeführt oder entnommen werden können.

In einer bevorzugten Ausführungsvariante der vorliegenden Erfindung erfolgt die thermocyclische Heizung der Reaktionsräume durch die Strahlungsenergie bevorzugt einer Hochleistungs-LED oder eines Diodenlasers, die bevorzugt im roten oder ultraroten Wellenlängenbereich, ohne den Ausschluss möglicher anderer Wellenlängenbereiche, emittiert, bevorzugt ohne dabei jedoch das Material des Trägers zu erwärmen. Die Einstrahlung kann revers oder bevorzugt invers über das Objektiv der Auswerteeinheit erfolgen. Der Träger und ggf. die Trennflüssigkeit wird bevorzugt isothermal im Bereich oder unter der erforderlichen Reaktions- oder Hybridisierungstemperatur von bevorzugt 45°C-70°C durch z.B. Pelletier-Elemente, einem Luftstrom oder einen Flüssigkeitsstrom gehalten. Durch LED-Impulse ist es so möglich, die Temperatur während z.B. einer PCR zwischen der Temperatur des Trägers und der gewünschten Maximaltemperatur von bevorzugt 95°C oszillieren zu lassen. Dadurch können sehr schnelle Reaktionszyklen erzielt werden, was zu einer Gesamtdauer der PCR von weniger als 10min und besonders bevorzugt von weniger als 3 min führt. Bevorzugt wird die Beleuchtung so gewählt, dass nur die Reaktionslösung die Strahlung absorbiert und weder der Träger noch die ggf. zur Begrenzung des Reaktionsraumes eingesetzte Trennflüssigkeit. Dadurch bleibt der für die Erwärmung der kleinen Reaktionsräume nötige Energieeintrag ebenfalls sehr gering, was die erforderliche Kühlung durch Wärmestrahlung oder Leitung über die Wand des Reaktionsgefäßes oder z.B. durch Konvektion im Falle der Überschichtung der Reaktionsräume mit einer Trennflüssigkeit auf ein Minimum beschränkt. Bevorzugt wird für die Erwärmung des Reaktionsraumes diejenige Wellenlänge der Beleuchtung eingesetzt, die ohnehin schon für die Vermessung der Reaktionsräume benötigt wird, wie z.B. 640nm wenn Cy5 als Marker für die Analyt-Fluoreszenz genutzt wird. Besonders bevorzugt wird jedoch eine Wellenlänge zur Erwärmung der Reaktionslösung genutzt, die größer als die der Analyt-Fluoreszenz ist und durch die z.B. inverse Mikroskop-Optik bzw. extern von der Mikroskop-Optik evers oder invers eingestrahlt wird. Die Lichtimpulsdauer zur Erwärmung der Reaktionsräume kann empirisch oder durch Thermographie bestimmt und für alle folgenden Reaktionen angewendet werden. Es ist aber auch möglich ein Temperatursensormolekül oder mehrere für verschiedene Temperaturen spezifische Temperatursensormoleküle in die Reaktionslösung zu geben, das beim Erreichen einer bestimmten Temperatur ein Fluoreszenzsignal aussendet. Dies könnte z.B. eine DNA-Haarnadel oder ein Moleular Beacon sein, die bündig mit dem 3'-Ende an das eigene 5'-Ende hybridisiert und an einem Ende einen Quencher und am anderen Ende ein Fluorophor trägt, deren Absorptions- bzw. Anregungsspektrum auf die Beleuchtung abgestimmt ist. Zur Verbesserung der Energieabsorption ist es ebenso möglich im Wellenlängenbereich der zur Erwärmung eingesetzten elektromagnetischen Strahlung Quencher oder Nanopartikel in die Reaktionslösung zu geben, die im entsprechenden Wellenlängenbereich der elektromagnetischen Strahlung absorbieren und die Energie in Wärme umwandeln.

Durch die Verwendung optisch transparenter Gefäßböden als Träger und/oder Trägerabdeckungen mit geringer Eigenfluoreszenz, welche die fluoreszenzoptische Auswertung während der thermozyklischen oder isothermischen Durchführung der Nachweisreaktion ermöglichen, sind Messungen mit Auflicht- und Durchlichtfluoreszenz/- lumineszenz bzw. der Absorption möglich. Bei Auflichtfluoreszenz können handelsübliche Mikrotiterplatten eingesetzt werden wie z.B. NucleoLink oder NucleoSorb aber auch z.B. handelsübliche Flachbodenmikrotestplatten, Einzelkavitäten oder 8-Kavitäten-Module (F8-Riegel). Die verwendeten handelsüblichen Reaktionsgefäße können zur Verringerung der Eigenfluoreszenz beschichtet werden oder zur Messung der Lumineszenz weiß sein.

Da bei Einsatz nicht permanent immobilisierter Mikropartikeln alle Mikropartikelfluoreszenzen während der Aufnahme weiterer Messpunkte gemessen werden müssen, sind besonders hohe Anforderungen an die Schnelligkeit der Hardware gestellt wie z.B. die Verwendung einer möglichst geringen Auflösung der Objektive bei hoher Lichtstärke, die Verwendung von Multibandfiltern zur Vermeidung von Filterwechseln in Kombination mit einer Mehrfarben-LED-Beleuchtung, die Verwendung einer elektronenverstärkenden Kamera, Parallelisierung der Detektoreinheiten. Besonders bevorzugt kommen LEDs mit Emissionen im Bereich von 350nm, 480nm, 530nm und 640nm zum Einsatz und werden bevorzugt mit den dazu passenden Vierfarben-Multiband-Anregungs-/Emissionfiltern kombiniert eingesetzt. Die Erweiterung des Farbspektrums in den roten oder ultraroten Bereich kann besonders für die Temperierung der Reaktionsräume genutzt werden. Detektoreinheiten können im Sinne einer Parallelisierung bevorzugt in 8er-Zeilen angeordnet sein oder durch Glasfaser- oder Flüssigleitern auf eine Kamera gebündelt werden. Ebenfalls ist es möglich durch Weitwinkelobjektive mehrere Kavitäten gleichzeitig zu messen. Durch Pixelbinning lässt sich die Messzeit ebenfalls reduzieren, da die Sensitivität steigt, was die Belichtung verkürzt, und die Datenmenge reduziert.

Bevorzugt wird bei Verwendung fluoreszenzkodierter Mikropartikel mit einem Durchmesser von 5-20$\mu$m die Optik so ausgelegt, dass das System eine Pixelauflösung von 0,5-2,5$\mu$m besitzt. Besitzen die Mikropartikel einen Durchmesser von 1-5$\mu$m, dann wird bevorzugt eine Pixelauflösung der Optik aus dem Bereich zwischen 0,1-1$\mu$m eingesetzt, ebenso bei der Aufnahme "Schwarzer Löcher" zum Zwecke der Einzelmolekülzählung.

Eine bevorzugte Möglichkeit zur Aufnahme der Signale wenn Mikropartikel in Multilayeranordnung eingesetzt werden, ist die Verwendung einer konfokalen Optik. Mit Hilfe der konfokalen Optik können schrittweise mehrere Ebenen der Multilayer aufgezeichnet werden, um möglichst viele signalgebende Mikropartikel in unterschiedlichen Schärfeebenen zu erfassen. Bevorzugt wird eine Schrittweite von 1-10$\mu$m zwischen zwei Aufnahmen gewählt.

In einem weiteren bevorzugten Verfahren ist es ebenso möglich, die Messung nicht ortsaufgelöst (bildgebend) durchzuführen, was den Vorteil hat, dass eine preiswerte Auswertetechnik eingesetzt werden kann, die nicht auf Mikroskop Technik basiert. Dabei können bevorzugt konventionelle Absorptions-, Lumineszenz-, Fluoreszenz-Reader oder - Scanner zum Einsatz kommen. Es muss sichergestellt sein, dass der optische Fokus dieser Geräte auf den Reaktionsraum oder die Reaktionsräume eingestellt ist, da auf Grund der geringen Schichtdicken der begrenzten Reaktionsräume die Intensitäten der Messsignale vergleichsweise gering sein können. Geringe Messsignale können bevorzugt durch Verwendung sehr empfindlicher Photodioden oder Photomultiplier bzw. durch die Erhöhung der Konzentration des zu löschenden Fluoreszenzfarbstoffes im Reaktionsraum kompensiert werden. Mit diesem nicht ortsaufgelösten Messansatz ist es nicht möglich eine Einzelmolekülzählung durchzuführen, so dass nur das Summensignal über alle Analytmoleküle, Analytmolekül-/Signalverstärkungssysteme oder Enzyme in den verschiedenen Reaktionsräumen erfasst werden kann. Alternativ ist es möglich, das Objekt mit einem Laserstrahl, der einen punktförmigen Querschnitt hat, gepulst abzuscannen und die Signale mit einem Photomultiplier zu messen. Nach der Messung werden die einzelnen Messpunkte dann zu einem Bild zusammengesetzt und analysiert.

Es ist ebenso möglich die Schichtdicke des Reaktionsraumes auf ein bis mehrere Millimeter zu erhöhen, insbesondere, wenn bei dieser Ausführungsvariante bevorzugt durch Peroxidase TMB zu einem blauen Reaktionsprodukt umgesetzt wird und dadurch ein Fluoreszenzfarbstoff mit Anregung und Emission im Wellenlängenbereich zwischen 600 bis 700nm in der Reaktionslösung oder auf der Oberfläche von Mikropartikeln oder im Inneren von Mikropartikeln hochsensitiv gelöscht wird. Der bevorzugt lateral nicht unterteilte Reaktionsraum ist in diesem Falle bevorzugt die komplette Kavität einer Mikrotestplatte.

Aufnahme von Messpunkten

Die Erfindung sieht vor, dass im Verlauf der Signalverstärkungsreaktion oder wenigstens nach Abschluss der Reaktion ein Messpunkt aufgezeichnet wird, um berechnen zu können wie stark die Reaktion für den einzelnen Analyten verlief oder in welcher Konzentration der Analyt in der Probe vorliegt. Sind vor der Messung bereits Ausgangssignale messbar, die zwischen verschiedenen Reaktionsräumen oder innerhalb eines Reaktionsraumes stark schwanken, so ist es möglich bereits vor oder zu Beginn der Reaktion einen ersten Messpunkt aufzuzeichnen und die daraus abgeleiteten Messwerte als Nullpunkt oder Referenz zu nutzen. Dieses Verfahren eignet sich besonders, wenn Mikropartikel verwendet werden, die eine Oberflächenfluoreszenz besitzen, welche zwischen den verschiedenen Mikropartikeln zwischen 1-50% schwanken kann. Zuerst können bevorzugt 1%-10% der am stärksten abweichenden Mikropartikel, die sogenannten Ausreißer aus der Auswertung ausgeschlossen werden. Danach wird z.B. eine Ratio aus dem Messwert vor der Reaktion und mindestens einem nachfolgenden Messwert oder allen weiteren Messwerten im Verlauf oder nach der Reaktion berechnet oder der Ausgangswert vom Messwert subtrahiert. Die Aufzeichnung und Verrechnung mehrerer Messwerte desselben Reaktionsraumes ermöglicht die Darstellung einer Messreihe für die Analyse der Reaktionskinetik.

Die Messwerte können aufgrund von Signaländerungen oder einer Zu- oder Abnahme bzw. Löschung des Signals des Reaktionsprodukts im Reaktionsraum entstehen. Die zu messende Signaländerung kann bevorzugt eine Zunahme der Oberflächenfluoreszenz auf Mikropartikeln und Träger umfassen, z.B. wenn eine Signalverstärkungsreaktion eine Quenchersonde in der Reaktionslösung abbaut, die komplementär zu einer auf dem Träger immobilisierten Fangsonde ist, die den die Oberflächenfluoreszenz-verursachenden Fluoreszenzfarbstoff trägt. Ebenfalls bevorzugt kann die Signaländerung eine Abnahme der Oberflächenfluoreszenz auf Mikropartikeln und Träger umfassen, z.B. wenn eine Signalverstärkungsreaktion ein lösliches oder präzipitierendes Reaktionsprodukt, z.B. einen Farbstoff, bildet, der die Oberflächenfluoreszenz löscht. Auch bevorzugt kann die Signaländerung eine Zunahme der Fluoreszenz im jeweiligen Reaktionsraum umfassen, z.B. wenn ein Signalverstärkungssystem einen nichtfluoreszierenden Farbstoff in einen fluoreszierenden Farbstoff umwandelt oder z.B. eine TaqMan-Sonde hydrolysiert wird, wodurch die Löschung des an der Sonde gebundenen Fluoreszenzfarbstoffes aufgehoben wird. Außerdem bevorzugt kann die Signaländerung eine Abnahme der Fluoreszenz im jeweiligen Reaktionsraum umfassen, z.B. durch die Bildung eines farbigen Reaktionsproduktes aus farbloser Substratlösung, wie z.B. TMB, das im Bereich der Anregung und/oder Emission des Fluoreszenzfarbstoffes absorbiert. Ebenfalls bevorzugt kann die Signaländerung eine Zunahme der Lumineszenz im jeweiligen Reaktionsraum umfassen, z.B. dadurch, dass eine Signalverstärkungsreaktion ein lumineszierendes Reaktionsprodukt im Reaktionsraum bildet. Auch bevorzugt kann die Signaländerung eine Zunahme der Intensität und der räumlichen Ausbreitung der Absorption in den Bereichen des fluoreszierenden Reaktionsraumes, bevorzugt ohne oder ohne vollständige laterale Begrenzung, umfassen, in denen einzelne Moleküle eines oder mehrerer Signalverstärkungssysteme am Träger gebunden sind und ein farbiges Reaktionsprodukt bilden. Dieses Reaktionsprodukt absorbiert im Bereich der Anregungs-und Emissionswellenlänge des Fluoreszenzfarbstoffes und die Fluoreszenz im Reaktionsraum wird somit effektiv gelöscht. So entstehen während der Enzymreaktion zunehmend dunkle Flecken, in der vorwiegend homogen über den gesamten Reaktionsraum verteilten Fluoreszenz. D.h. sogenannte "Schwarze Löcher" entstehen dadurch, dass der gebildete Farbstoff vom Signalverstärkungssystem in alle Richtungen in den Reaktionsraum diffundiert.

Bei Verwendung von nichtfluoreszenzkodierten Mikropartikeln in zumindest teilweise begrenzten Reaktionsräumen ist die Aufzeichnung von Messreihen besonders geeignet, um "Schwarze Löcher" zu detektieren. Bevorzugt werden die Parameter Intensität der Fluoreszenzlöschung und räumliche Ausbreitung der Fluoreszenzlöschung durch Diffusion konzentrisch vom Ort der Lokalisation des Enzymmoleküls in den Reaktionsraum hinein erfasst. Die Ausbreitung der Löschung ist von der Diffusion der Reaktionsprodukte aber auch von Strömungen der Reaktionslösung insbesondere bei lateral unbegrenzten Reaktionsräumen im Reaktionsraum abhängig. Durch Strömungen wird die à priori stattfindende konzentrische Diffusion z.B. annäherungsweise elliptisch deformiert, so dass eine Korrekturrechnung zur Rückführung auf das konzentrische Modell erforderlich ist bzw. aus der berechneten Fläche direkt die Intensität der Enzym-Reaktivität berechnet wird. Durch die mathematische Analyse und Korrektur werden, die Intensitätsverteilung der Enzymaktivität über die Zeit analysiert, um z.B. entscheiden zu können, ob die Enzymaktivität einem oder mehreren benachbart immobilisierten Enzymmolekülen zuzuordnen ist. Da die Zahl der immobilisierten Enzymmoleküle proportional zur Zahl der Analytmoleküle in der Probe ist, kann durch diese Datenanalyse die Genauigkeit der Messung gesteigert werden. Werden die Reaktionsräume durch die Aufzeichnung von Z-Stapeln räumlich erfasst, können auch die "schwarzen Löcher" dreidimensional aufgezeichnet und somit ihr Volumen für die weiteren Berechnungen genutzt werden.

Die Genauigkeit der Messung hängt direkt von der verwendeten Pixelauflösung des Messystems ab. Bevorzugt werden für diese Ausführungsvariante eine Pixelauflösung von $<0,5\mu m$ genutzt. Damit können "Schwarze Löcher" mit einem lateralen Durchmesser von $2-3\mu m$ bei einer Schichtdicke der Reaktionsräume von $2\mu m$ sicher erfasst werden. Der Abstand zwischen zwei Enzymen oder Signalverstärkungssystemen auf dem Träger sollte bevorzugt im Mittel $10\mu m$ nicht unterschreiten. Bei dieser Enzymdichte auf dem Träger hat die Kavität einer Mikrotestplatte mit einem Durchmesser von 6mm die Kapazität von etwa 30 Mio. Enzym-Molekülen. Mit zunehmender Enzymdichte auf dem Träger, wird auch die Zahl dicht beieinander liegender Moleküle zunehmen, die optisch nicht mehr sicher getrennt werden können. Die Erfassung der Signalintensität, der Signalausbreitung im Reaktionsraum für das jeweilige "Schwarze Loch" bzw. die Kinetik beider Parameter, werden bevorzugt für die Bewertung eines "Schwarzen Loches" herangezogen, ob es von einem oder mehreren Enzymen verursacht wurde. Adäquat kann man natürlich vorgehen, wenn alternativ ein Fluoreszenz- oder Lumineszenzsubstrat genutzt wird.

Die Intensität der Signalverstärkungsreaktion und Diffusion des Reaktionsproduktes sind so für die jeweilige, Anwendung anzupassen, dass eine Messung nach 10-20min im Plateau der Signalverstärkungsreaktion bzw. mehrmals nach 0,1-20min erfolgt. Parameter zur Anpassung der Signalverstärkungsreaktion sind z.B. die Veränderung der Temperatur bzw. der Zusatz von Glycerol zur Reaktionslösung.

Zur Erfassung der Messpunkte ist es möglich, den Träger komplett zu erfassen, was bei einer Pixelauflösung von <0,5μm die Aufnahme mehrerer bis vieler Bilder erfordern kann. Wenn die am Analyten gebundenen Enzymmoleküle sich jedoch statistisch gesehen gleichmäßig über den Träger verteilen, kann die Zahl der Aufnahmen reduziert werden, wobei bevorzugt wenigstens zehnmal mehr Moleküle gemessen werden als die durchschnittliche unspezifische Bindung beträgt. Ist die Zahl der erfassten Moleküle geringer, wird automatisch eine größere Anzahl von Bildern erfasst. Dieses Vorgehen ermöglicht effektiv die Messzeit zu optimieren.

Es ist durch den Fachmann leicht erkennbar, dass anstelle eines löschenden Reaktionsproduktes durch das Enzym ein leuchtendes Reaktionsprodukt gebildet werden kann, wenn z.B. Ampliflu Red als Enzymsubstrat (Sigma-Aldrich) eingesetzt wird. In diesem Falle wird die Intensitätsverteilung der Enzymaktivität der Peroxidase als Fluoreszenz und nicht als Löschung wie bei den "Schwarzen Löchern" erfasst und verrechnet. Gleichermaßen kann das Verfahren bei Verwendung der entsprechenden Enzyme und Enzymsubstrate auf die Lumineszenzdetektion übertragen werden.

Bei Verwendung von fluoreszenzkodierten Mikropartikeln ist es erforderlich, alle für die Auswertung in Betracht kommenden Mikropartikel zu identifizieren indem die Kodierungsfluoreszenzen erfasst werden, um alle Mikropartikel einer Mikropartikelpopulation zuordnen zu können. Bevorzugt wird die für die Auswertung erforderliche x/y-Position einer notwendigen Anzahl identifizierter Mikropartikel pro Population abgespeichert, um die Mess-Orte für die Aufzeichnung der Oberflächen- bzw. Analytfluoreszenz bei der Erfassung weiterer Messpunkte wiederzufinden. Des Weiteren wird die Oberflächen- bzw. Analytfluoreszenz als Referenz für das in den folgenden Messpunkten aufgezeichnete Oberflächen- bzw. Ligandensignal erfasst und gegebenenfalls auch zur Dekodierung der Mikropartikelpopulation eingesetzt. Es ist ebenso möglich, anstelle der Analytfluoreszenz eine Analytlumineszenz zu erfassen. In Abhängigkeit des verwendeten Signalverstärkungssystems kann das Reaktionsprodukt sich im gesamten Reaktionsraum verteilen oder an der Oberfläche des Träger oder des Mikropartikels präzipitieren oder kovalent binden.

Die Aufzeichnung der weiteren Messpunkte kann in regelmäßigen Zeitabständen vor, während und nach der Reaktion erfolgen oder aber nachdem eine bestimmte Temperatur oder ein bestimmter Abschnitt eines Thermozyklus erreicht wurde. Bevorzugt erfolgen die Messungen während eines isothermischen Abschnittes der Reaktion, wenn z.B. eine PCR durchgeführt wird, um Einflüsse die durch Temperaturschwankungen wie Materialdehnung und verstärkte Konvektion im Reaktionsgemisch zu minimieren.

Es kann von Vorteil sein, während der Reaktion von Zeit zu Zeit neben der Aufnahme der Oberflächen bzw. Ligandenfluoreszenz auch eine Mikropartikelfluoreszenz aufzunehmen, da bei abnehmender Oberflächenfluoreszenz die Gefahr besteht, dass ein Mikropartikel nicht korrekt wiedergefunden wird. Um Bleaching zu minimieren, kann bevorzugt bei Verwendung von LED insbesondere bei der Aufzeichnung weiterer Messpunkte eine Anpassung der Beleuchtungsstärke auf die Oberflächenfluoreszenz vorgenommen werden. Die variierte Beleuchtungsstärke ist gegebenenfalls zu referenzieren. Als Referenz können bevorzugt Mikropartikel mit standardisierten Fluoreszenzeigenschaften verwendet werden. Eine Reduktion des Bleachings erfolgt durch die Begrenzung der Beleuchtung auf die Zeit der Bildaufnahme. Außerdem kann durch die Verringerung der Objektivvergrößerung eine größere Messfläche abgebildet werden, wodurch weniger Bilder pro well benötigt werden.

Die Vermeidung von Nachfokussierung und Nachbelichtung durch Algorithmen wie "extended-depth-of-field" (Erhöhung der Tiefenschärfe durch Zusammenrechnen mehrerer Bilder in Z-Richtung) und "high-dynamic-range" (Erhöhung der Belichtungsdynamik durch Zusammenrechnen eines sehr hellen und eines sehr dunklen Bildes) verkürzen ebenfalls die Messzeit und erhöhen die Präzision der Messung und verbessern den Dynamikbereich des Messwertes. Bevorzugt fluoreszierende Mikropartikel auf der Oberfläche des Trägers können als Fokussierhilfe genutzt werden.

Pro Messzeitpunkt werden 1-10000 fluoreszenzkodierte Mikropartikel einer Population erfasst, bevorzugt 1-100 Mikropartikel, besonders bevorzugt 1-10 Mikropartikel.

Zuordnung der Messpunkte zu Mikropartikeln und Mikropartikelpopulationen

Bevorzugt werden zur Dekodierung der Mikropartikelpopulationen die Fluoreszenzen der in die Mikropartikel insgesamt oder in die verschiedenen Schichten einpolymerisierten Farbstoffe eingesetzt. Aus den verschiedenen z.B. Fluoreszenzintensitäten werden Verhältnisse (Ratios, PopID) berechnet, die für jede Mikropartikelpopulation eine eindeutige Kennzeichnung erlauben. Als sekundäres Unterscheidungsmerkmal können verschiedene Größenklassen, Formen und Muster von Mikropartikel eingesetzt werden.

So kann die Fluoreszenzintensität einer bestimmten Emissionswellenlänge, in der Mikropartikelperipherie als Ringfluoreszenz erkennbar, vor dem Start einer Nukleasereaktion zur Bestimmung der Mikropartikelpopulation aber auch zur Bestimmung des Maximalsignals der Oberflächenfluoreszenz des betreffenden Mikropartikels vor der Reaktion bestimmt werden. Im Verlauf der Reaktion nimmt dieses Maximalsignal ab, da die Biomoleküle enzymatisch degradiert werden und somit der Farbstoff von der Mikropartikeloberfläche in den Reaktionsraum freigesetzt wird. Für die Dekodierung der Mikropartikelpopulationen steht der Farbstoff dann nicht mehr zur Verfügung. Da der Mikropartikel permanent immobilisiert wurde, braucht die Dekodierung aber auch nicht mehr erfolgen. Zur Kontrolle der korrekten Mikropartikelwiederfindung ohne Dekodierung sind in diesem Falle die Fluoreszenzen der in die Mikropartikel einpolymerisierten Farbstoffe selbst ausreichend. Kodierungs-, Oberflächen- und/oder Ligandenfluoreszenz können ebenfalls dazu herangezogen werden, um Mikropartikel unterschiedlicher Größen in Bezug auf detektierte Fluoreszenzintensitäten zu referenzieren. Dies wird bevorzugt zur Minimierung von Fehlern genutzt, die sich aus Größenschwankungen der Mikropartikel innerhalb einer Population ergeben können, da die Intensitäten der Mikropartikelfluoreszenzen und der Oberflächenfluoreszenz proportional mit dem Durchmesser der Mikropartikel variieren. Die verschiedenen Mikropartikelfluoreszenzen werden aber ebenfalls dazu genutzt, Mikropartikelpopulationen voneinander zu unterscheiden, die die gleiche PopID besitzen, sich aber im Durchmesser um bevorzugt 2-50$\mu$m unterscheiden. Besonders bevorzugt unterscheiden sich solche Mikropartikelpopulation um 3-15$\mu$m im mittleren Mikropartikeldurchmesser.

Ranking-booster-Algorithmus

In einer Ausführungsvariante befinden sich z.T. mehrere der vorzugsweise fluoreszierenden oder fluoreszenzkodierten Mikropartikel in einem Reaktionsraum (siehe Fig. 9). Dabei können bevorzugt verschiedene Fluoreszenzkodierungen genutzt werden, um über mikropartikelpopulationsspezifische Binder Multiplex-Tests bereitzustellen. Es ist aber auch möglich, dass sich im Reaktionsraum nur eine Population befindet, um für digitale Testsysteme ausreichend Messpunkte verfügbar zu haben. Bei dieser Ausführungsform des Verfahrens geht der Mikropartikel als à priori kugelförmige Struktur für die Mikropartikelerkennung verloren, da sich benachbarte Mikropartikel berühren und deshalb als konfluente Struktur erscheinen. Deshalb wird für dieses Verfahren bevorzugt folgender erfindungsgemäßer Auswertealgorithmus, der Ranking-Booster-Algorithmus, durchgeführt:
Mikropartikel haben vorzugsweise eine nahezu perfekte Kugelform, d.h. sie sind im aufgenommenen Bild als kreisförmige Objekte dargestellt. Ihr minimaler und maximaler Durchmesser (in Pixeln im Bild) ist in etwa (+/- 25 %) à priori bekannt. Die Helligkeiten der Mikropartikel untereinander variieren bevorzugt bis etwa 1:20, ihr Durchmesser im Verhältnis 1:3. Überbelichtung der Mikropartikel sollte nicht auftreten und wird deshalb durch eine Belichtungsautomatik kompensiert. Die Mikropartikel sind stochastisch verteilt bis dicht gepackt bevorzugt in einer Ebene liegend.
Das Bild wird bevorzugt zuerst mit einem konventionellen Objekterkennungsalgorithmus bearbeitet, der physisch einzeln liegende Mikropartikel und Agglomerate als Solche erkennt (siehe Fig. 10). Nur die Agglomerate werden einer Weiterverarbeitung durch den Ranking-Booster zugeführt. Bevorzugt können die betreffenden Bildausschnitte mit einer höheren Auflösung neu aufgenommen werden. Die Außengrenzen der Agglomerate werden bereits hier bestimmt.
Es wird eine bevorzugt nahezu kreisförmige Umgebungs-Pixelmaske konzentrisch um jedes Agglomerat-Pixel untersucht, deren Durchmesser des a-priori bekannten minimalen Mikropartikel-Durchmessers entspricht. Der relative Rang des aktuell untersuchten Pixel bzgl. seiner Umgebungs-Pixelmaske wird bestimmt und dieser Wert an entsprechender XY-Koordinate in einem Zwischenbild, dem Relativ-Rang-Bild gespeichert (siehe Fig. 11). Dieses Relativ-Rang-Bild kann optional vor der weiteren Bildverarbeitung mit einen Glättungsfilter (z.B. 3x3- od. 5x5-Medianfilter) bearbeitet werden, um kleine lokale Störungen zu eliminieren.
Das Relativ-Rang-Bild wird bevorzugt mit einer festen Schwelle binarisiert (z.B. Schwellwert 0.5), da die absoluten Helligkeiten der Mikropartikel nach dem Ranking-Booster keine Rolle mehr spielen (siehe Fig. 12). In dem entstandenen Binärbild ist die Zentrumsregion jedes Mikropartikels als diskrete kreisrunde Scheibe erkennbar, weil der Mittelpunkt eines Mikropartikels (im Originalbild) immer dessen hellstes Pixel ist und die Pixel-Helligkeiten radial abfallen. Die Scheiben beinhalten, abhängig vom gewählten Schwellwert, bevorzugt etwa nur die Hälfte der Fläche eines Mikropartikels und können sich deshalb nicht untereinander berühren.
Mittels Objekterkennung werden die Zentrums-Scheiben identifiziert. Sonstige sichelförmige oder dreieckige kleine Objekte in den Zwischenräumen sind Artefakte, die an ihrer nichtrunden Form und zu geringer Fläche erkennbar sind. Diese Artefakte werden ersatzlos verworfen.
Um die ursprüngliche Fläche der Mikropartikel wieder zu erlangen, werden die Zentrums-Scheiben Schritt für Schritt vergrößert. Um den Rand jeder identifizierten Zentrums-Scheibe wird jeweils eine Lage Pixel pro Iterations-Schritt hinzugefügt (siehe Fig. 13). Sobald ein neu hinzugefügtes Pixel einer Zentrums-Scheibe an ein originales oder bereits hinzugefügtes Pixel einer anderen Scheibe grenzt, ist ein Kontaktpunkt zwischen Mikropartikeln identifiziert (siehe Fig. 14). Die Detektion eines Kontaktpunkts beendet die Inflation beider beteiligten Zentrums-Scheiben, die Pixel der unvollständigen letzten Lage werden wieder entfernt. Für Zentrums-Scheiben, für die noch keine Kontaktpunkte detektiert wurden, werden weitere Inflations-Iterationen durchgeführt. Die Inflation der Zentrums-Scheiben wird jedoch auf das ursprüngliche Agglomerations-Objekt limitiert, d.h. die Iterationen für eine Scheibe wird gestoppt, sobald ein Pixel hinzugefügt werden müsste, das nicht zum Ausgang-Gesamtobjekt gehört.

Bestimmung von Kinetiken

Kinetiken der Bildung des Reaktionsproduktes einer Signalverstärkungsreaktion in den Reaktionsräumen zur Beschreibung der Analyt-Konzentration bzw. der Analyt-Eigenschaften werden bevorzugt für z.B. Anwendungen zur real-time-PCR oder zur Bestimmung der enzymatischen Aktivität von Analyten aufgezeichnet.

Durch die Analyse der Fluoreszenzänderung als Folge der Reaktion von Messpunkt zu Messpunkt ergeben sich Reaktionskinetiken aus denen sich Konzentrationen der Analyten oder auch Bindungskonstanten errechnen lassen. Erfolgt die Auswertung bezogen auf einen Reaktionszyklus, wie z.B. bei der Polymerase-Kettenreaktion ist die Angabe des Durchbruchszyklus, bei dem das Signal oder die Signaländerung den Hintergrund durchbricht, als Maß für die gebildete Menge an Reaktionsprodukt entscheidend. Auf diese Weise ist eine genauere Quantifizierung der PCR-Produkte in einer Probe möglich als durch die Bestimmung der Fluoreszenzintensität. Durch Kombination mit einer Schmelzkurvenanalytik ist es möglich weitere Informationen über die Sequenz und die Hybridisierungseigenschaften von Nukleinsäureanalyten zu erlangen.

Durch die Referenzierung aller weiterer Messpunkte auf den ersten Messpunkt oder einen der darauffolgenden Messpunkte vor oder zu Beginn der Reaktion werden Messfehler deutlich reduziert, da die mögliche Referenzierung bevorzugt auf eine Mikropartikelpopulation als Positivkontrolle oder auf eine externe Referenzprobe Einflüsse wie unterschiedliche Ausleuchtung oder Mikropartikelgröße minimiert werden können. Das Verfahren sieht ebenfalls vor, dass der Einfluss von Störgrößen systematisch erfasst werden muss, um diese bei der Berechnung der Messwerte berücksichtigen zu können. Solche Störgrößen können das Ausbleichen (Bleaching/Fading) einiger Fluoreszenzfarbstoffe sein ebenso wie Einflüsse von Temperaturschwankungen auf die Messung oder die unspezifische Hintergrundfluoreszenz.

**Beispiel 1: Singleplex-Immunoassay**

Beispiel 1 stellt eine einfach auszuführende Variante bevorzugt für Singleplex-Immunoassays dar, bei dem das Antigen PSA sensitiv ($10^{-13}$-$10^{-14}$ M) nachgewiesen wird.

Schritt 1: Mikrotestplatte wird mit Antikörpern als Binder beschichtet

Eine Flachboden-Mikrotestplatte aus Polysterol, deren Boden als planarer Träger dient, wird mit 1 $\mu$g/ml der IgG-Fraktion eines Anti-prostataspezifischen polyklonalen Antikörpers als Fangantikörper in Phosphatpuffer (PBS) über Nacht beschichtet. Nicht gebundener Antikörper wird durch mehrmaliges Waschen in PBS unter Zusatz von 0,1 % Tween 20 (PBS-T) entfernt. Freie Bindungsstellen an der Oberfläche des planaren Trägers werden durch Inkubation mit PBS-T unter Zusatz 2% humanen Serumalbumins (HSA) blockiert.

Schritt 2: Inkubation der Probe und Bindung des Analyten an den Antikörper

Die Verdünnung des Patientenserums erfolgt 1:2 in PBS-T-HSA und 50$\mu$l dieser Verdünnung jeder Probe werden in eine Kavität, die mit dem Antikörper beschichtet wurde, pipettiert und 2h inkubiert.

Schritt 3: Waschen

Die ungebundenen Probenbestandteile werden durch mehrmaliges Waschen in PBS-T aus den Kavitäten entfernt.

Schritt 4: Inkubation mit dem Signalverstärkungssystem

Ein HRP-Konjugat der oben genannten IgG-Fraktion eines Anti-prostataspezifischen polyklonlen Antikörpers wird in PBS-T-HSA 0,1$\mu$g/ml verdünnt und 50$\mu$l werden in die Kavitäten der Mikrotestplatte pipettiert und 2h inkubiert, so dass das zu vor aus der Probe gebundene PSA mit HRP als Signalverstärkungssystem markiert worden ist.

Schritt 5: Waschen

Das ungebundene Nachweisreagenz Signalverstärkungssystems wird durch mehrmaliges Waschen in PBS-T aus den Kavitäten entfernt.

Schritt 6: Zugabe/teilweise Absaugen des TMB/Fluorocrome-Enzymsubstrates einschließlich Fokussiermikropartikel

Nach dem gründlichen Entfernen der Waschlösung durch Absaugen wird eine kommerzielle TMB-Fertigsubstratlösung unter Zusatz von 100ng Cy5/ml 0,05% Tween 20 und circa 5000 fluoreszierender PMMA-Mikropartikel (Anregung 480nm/Emission 520nm) mit einem Durchmesser von 2$\mu$m in die Kavitäten pipettiert und nach 30sec wieder weitgehend abgesaugt.

### Schritt 7: Überschichtung mit Öl

Danach wird der planare Träger durch pipettieren von 50μl Mineralöl in die Kavitäten überschichtet, so dass der Reaktionsraum den gesamten Kavitätenboden mit einer Schichtdicke <10μm umfasst.

### Schritt 8: Vermessung im F1100 Fluoreszenzimager

Die Mikrotestplatte wird im Fl100 Fluoreszenzimager (Attomol) ausgewertet, indem nach dem Fokussieren bei 520nm Fluoreszenzbilder bei einer Anregungswellenlänge von 640nm und einer Emissionswellenlänge von 660nm mit dem optischen Fokus im Reaktionsraum und einer Pixelauflösung von 0,65μm alle 15sec aufgezeichnet werden. Aus einem Z-Stapel wird das jeweils hellste Bild ausgewählt und dem Imagingprozess automatisch zugeführt, nachdem die Qualität der Bilder automatisch kontrolliert und ggf. korrigiert wird.

### Schritt 9: Imaging "schwarzer Löcher"

In Echtzeit, also quasi bei kontinuierlicher Beobachtung, des Aufzeichnungsprozesses werden Bereiche mit geringerer Fluoreszenz, die mehr als die 3-fache Standardabweichung von der Ausgangsfluoreszenz oder der Umgebungsfluoreszenz abweichen selektiert und deren Veränderung über mehrere Aufnahmen durch Intensitätsvektoralgorithmen berechnet und bewertet, wie viele Moleküle des Signalverstärkungssystems sich im Reaktionsansatz befinden. Sind zu viele Signalverstärkungsmoleküle im Reaktionsansatz sehr dicht gepackt immobilisiert, da die Analytkonzentration in der Probe sehr hoch ist, wird die Probe als positiv ohne Konzentrationsangabe für den Analyten ausgegeben. Eine Quantifizierung des Analyten wird alternativ über die Kinetik der Fluoreszenzlöschung, d.h. über die Verringerung des Summensignals über den gesamten Messraum, im Vergleich zu einer Referenzprobe erreicht. Die entsprechenden Konzentrationsangaben werden von der Software automatisch ausgegeben.

### Beispiel 2: Einzelmolekül-Singleplex-Immunoassay

Beispiel 2 unterscheidet sich von Beispiel 1 dadurch, dass durch die Verwendung von frei in der Probe flottierenden Nanopartikeln als Träger der Antikörper als Binder größere Probenvolumina eingesetzt werden können und dadurch die Wahrscheinlichkeit erhöht wird, dass bei sehr geringen Konzentrationen des Analyten im zu beprobenden Raum (z.B. Patientenserum oder Leitungswasser) überhaupt Analyt Moleküle bei der Probennahme erfasst werden. Außerdem werden durch die Verwendung von frei in der Probe flottierenden Nanopartikeln die Diffusionswege von Analyt Molekülen zu den Binders auf den Nanopartikeln verkürzt, so dass bei einem Überschuss von Bindern unter Berücksichtigung der Bindungskonstanten zwischen Analyt und Binder nahezu alle Analyt Moleküle in den Analyseprozess überführt werden können. Da weitgehend alle Analyten aus der gesamten Probe berücksichtigt werden, kann diese Ausführungsvariante als quantisiert ("quasi digital") betrachtet werden. Das nachfolgende Protokoll ermöglicht die ultrasensitive Detektion von Troponin, einem Frühmarker für das Herzinfarktrisiko:

### Schritt 1:

Handelsübliche paramagnetische Nanopartikel aus Polymethacrylat mit Polyakrylsäure-COOH-Oberflächenbeschichtung (PolyAn) und einem Durchmesser von 200nm werden nach EDC-Aktivierung mit 1mg Anti-Troponin-Antikörpern/ml PBS für 2h inkubiert und somit beschichtet. Ungebundene Antikörper werden durch mehrmaliges Waschen in einem Überschuss an PBS-T vollständig entfernt.

### Schritt 2:

250μl Probe werden mit 25μl 0,5M PBS unter Zusatz von 1% Tween 20 versetzt und vorsichtig homogen gemischt. Die bereits vorbereiteten Kavitäten einer Mikrotestplatte aus Polystyrol wurden mit PBS-T geblockt und nun mit der vorbereiteten Probenlösung befüllt. Alle befüllten Kavitäten werden dann mit 10μl einer Suspension mit einer definierten Zahl von Nanopartikeln versetzt, so dass diese bei vollständiger Sedimentation eine Schichtdicke von etwa 10-20μm ergibt, d.h. etwa $5\times10^{10}$ Nanopartikel pro Kavität mit ca. 6mm Durchmesser.

### Schritt 3:

Die mit Nanopartikeln versetzte Probenlösung wird bei intensivem Schütteln 2h inkubiert.

Schritt 4:

Nachfolgend werden ungebundene Bestandteile der Probe durch mehrmaliges Waschen in einem Überschuss PBS-T entfernt. Dazu werden die Nanopartikel magnetisch in der Kavität sedimentiert, bevor die Waschlösung entfernt und durch neue Waschlösung entfernt wird.

Schritt 5:

Nach dem PBST entfernt wurde, wird die Kavität mit Anti-Troponin-POD (direkter Sandwich-Assay) verdünnt in PBS-T für 2h unter intensivem Schütteln inkubiert.

Schritt 6: Waschen

Waschen wie unter Schritt 4.

Schritt 7:

Nach dem gründlichen Entfernen der Waschlösung durch Absaugen wird eine kommerzielle TMB-Fertigsubstratlösung unter Zusatz von 100ng Cy5/ml 0,05 % Tween 20 in die Kavitäten pipettiert und die Nanopartikel darin resuspendiert. Nach etwas 30sec werden die Nanopartikel gleichmäßig magnetisch sedimentiert, und die Substratlösung abpipettiert. Erst nach der Überschichtung der Nanopartikelschicht in allen Kavitäten mit $50\mu l$ Mineralöl wird der Magnet für die Immobilisierung der Nanopartikel entfernt. Im Ansatz befinden sich etwa 500 fluoreszierende Mikroartikel mit einem Durchmesser von $10\mu m$ für die Fokussierung. Bei Verwendung eines geeigneten Hardware-Autofokus kann die Zugabe der fluoreszierenden Mikropartikel als Orientierung zur Fokussierung entfallen.

Schritt 8: Vermessung im Fl100 Fluoreszenzimager

Die Mikrotestplatte wird im Fl100 Fluoreszenzimager (Attomol) ausgewertet, indem Fluoreszenzbilder bei einer Anregungswellenlänge von 640nm und einer Emissionswellenlänge von 660nm mit dem optischen Fokus im Reaktionsraum und einer Pixelauflösung von 0,65 $\mu$m alle 15sec aufgezeichnet. Aus einem Z-Stapel wird das jeweils hellste Bild ausgewählt und dem Imagingprozess automatisch zugeführt, nachdem die Qualität der Bilder automatisch kontrolliert und ggf. korrigiert wird.

Schritt 9: Imaging "schwarzer Löcher"

In Echtzeit, das heißt, ohne Unterbrechung des also quasi bei kontinuierlicher Beobachtung, Aufzeichnungsprozesses werden Bereiche mit geringerer Fluoreszenz, die mehr als die 3-fache Standardabweichung von der Ausgangsfluoreszenz oder der Umgebungsfluoreszenz abweichen selektiert und deren Veränderung über mehrere Aufnahmen durch Intensitätsvektoralgorithmen berechnet und bewertet, wie viele enzymmolekültragende Nanopartikel sich im Reaktionsansatz befinden. Sind zu viele enzymmolekültragende Nanopartikel im Reaktionsansatz sehr dicht bzw. übereinander gepackt immobilisiert, da die Analytkonzentration in der Probe zu hoch ist, wird die Probe als positiv ohne Konzentrationsangabe für den Analyten PSA ausgegeben. Eine Quantifizierung des Analyten wird alternativ über die Kinetik der Fluoreszenzlöschung, d.h. über die Verringerung des Summensignals über den gesamten Messraum, im Vergleich zu einer Referenzprobe erreicht. Die entsprechenden Konzentrationsangaben werden von der Software automatisch ausgegeben.

**Beispiel** 3a: **sensitiver Multiplex-Antikörper-Immunoassay**

Bei komplexen Erkrankungen, wie bei vielen Autoimmunerkrankungen kann es wichtig sein, mehrere Analyten gleichzeitig und insbesondere in Frühstadien der Erkrankungen auch sehr sensitiv und quantitativ zu erfassen. Mit der folgenden Ausführungsvariante ist es möglich, im 3-plex für drei Analyten sehr sensitiv die nach Inkubation mit der Probe am Träger gebundenen Antikörpermoleküle zu zählen und so zu quantifizieren, ohne dass es sich um eine absolute oder digitale Quantifizierung handelt, d.h. ohne, dass alle Analytmoleküle zwangsläufig in den Analyseprozess einbezogen wurden:

Schritt 1:

Drei unterschiedlich fluoreszenzkodierte, Polyacrylsäure-tragende, COOH-modifizierte Polymethacrylat-Mikropartikel mit einem Durchmesser von $2\mu$m werden EDC-aktiviert und mit je 1mg Polylysin, RoSS-A- bzw. SM-Antigen pro ml PBS für 2h inkubiert und somit populationsspezifisch mit Antigen beschichtet. Ungebundene Antigene werden durch mehrmaliges Waschen in einem Überschuss an PBS-T vollständig entfernt. Dazu werden die Mikropartikel durch Zentrifugation sedimentiert, bevor die Waschlösung entfernt und durch neue Waschlösung ersetzt wird. Optional können weitere Mikropartikelpopulationen mit weiteren Autoantigenen oder Kontrollantigenen in den Ansatz einbezogen werden

Schritt 2:

Danach wird die mit Polylsin beschichtete Mikropartikelpopulation in gleicher Weise mit 1mg dsDNA/ml PBS aus Heringssperma inkubiert und gewaschen.

Schritt 3:

Eine Mikrotestplatte aus Polystyrol wird mit $10\mu$g Polylysin / ml PBS über Nacht inkubiert und danach mehrmals mit PBS gewaschen. Danach wird ein Suspensionen mit 5.000 fluoreszenzkodierten, beschichteten Mikropartikel jeder Population einzeln nacheinander oder als Gemisch aller 3 Populationen in die Kavitäten der Mikrotestplatte pipettiert und vollständig und gleichmäßig durch Zentrifugation oder Absinken lassen sedimentiert.

Schritt 4:

Danach wird der Überstand abgesaugt und die Probe, 1:50 in PBS-T verdünntes Patientenserum, in die Kavitäten pipettiert und 1h inkubiert, so dass gegebenenfalls im Serum enthaltene, für eines der drei Antigene spezifische Antikörper an die jeweilige Mikropartikelpopulation binden können. Es ist ebenfalls möglich, die Probe über mehrere Verdünnungsstufen in verschiedenen Kavitäten zu titrieren, um in den Einzelmolekülbereich am Träger gebundener Analyten für jeden der drei Analyten für eine genauere Quantifizierung zu gelangen.

Schritt 5:

Nachfolgend werden ungebundene Bestandteile der Probe durch mehrmaliges Waschen in einem Überschuss PBS-T entfernt.

Schritt 6:

Nach dem PBST entfernt wurde, wird die Kavität mit Anti-human-lgG-POD als Nachweisreagenz verdünnt in PBS-T für 1h unter Schütteln inkubiert.

Schritt 7:

Nachfolgend werden ungebundene Bestandteile des Nachweisreagenzes durch mehrmaliges Waschen in einem Überschuss PBS-T entfernt.

Schritt 8:

Nach dem gründlichen Entfernen der Waschlösung durch Absaugen wird eine kommerzielle TMB-Fertigsubstratlösung unter Zusatz von 100ng Cy5/ml 0,05% Tween 20 in die Kavitäten pipettiert und nach etwa 30sec wieder aus der Kavität durch Absaugen entfernt und sofort mit $50\mu$l Mineralöl überschichtet.

Schritt 9: Vermessung im F1100 Fluoreszenzimager

Die Mikrotestplatte wird im Fl100 Fluoreszenzimager (Attomol) ausgewertet, indem Fluoreszenzbilder bei einer Anregungswellenlängen von 480nm, 530nm und 640nm und entsprechenden Emissionswellenlängen von 520nm, 560nm bzw. 660nm mit dem optischen Fokus im Reaktionsraum und einer Pixelauflösung von $0,65\mu$m alle 15sec aufgezeichnet. Das automatisch optimal fokussierte und belichtete Bild wird automatisch ausgewählt und dem Imagingprozess mittels FastFluoscan-Software (Attomol) zugeführt, nachdem die Qualität der Bilder automatisch kontrolliert und ggf. korrigiert wird.

Schritt 10: Imaging und Auswertung

Die FastFluoscan-Software erkennt alle Mikropartikel im Bild und ordnet diese nach Auswertung der Kodierungsfluoreszenzen der Mikropartikel (Emissionen bei 520nm bzw. 560nm) einer der drei Mikropartikelpopulationen zu. Danach wird die analytkonzentrationsabhängige Cy5-Fluoreszenzveränderung für die einzelnen Mikropartikel im jeweiligen Reaktionsraum ermittelt und die Kinetik der prozentualen Cy5-Löschung berechnet. Daraus wird berechnet, welche Mikropartikel einer Population positiv sind, insbesondere wenn diese dichter mit Mikropartikeln anderer Populationen oder derselben Population in der Kavität benachbart liegen, so dass deren Reaktionsräume nicht vollständig voneinander getrennt sein könnten. Das Maß dafür ist die Zeit mit der ein entsprechendes Mikropartikel eine gelöschte Cy5-Fluoreszenz aufweist im Verhältnis zur durchschnittlichen Zeit für diese Mikropartikelpopulation. Ist die Löschung signifikant geringer als die durchschnittliche Zeit und eine Nachbarpopulation zeigt eine qequenchte Cy5-Fluoreszenz, ist diese Population als negativ zu werden. Entspricht sie der durchschnittlichen Zeit, wird sie als positiv gewertet. Danach werden die positiven Mikropartikel und die negativen Mikropartikel einer Population gezählt und deren Ratio als Maß für die Analytkonzentration auf eine Referenzprobenreihe mit Kalibrierfunktion bezogen, um die Analytkonzentration in der Probe anzugeben.

**Beispiel 3b: sensitiver Multiplex-Antikörper-Immunoassay**

Schritte 1 bis 5:

Diese Schritte entsprechen den Schritten 1 bis 5 aus Beispiel 3a.

Schritt 6:

Nach dem PBS-T entfernt wurde, wird die Kavität mit biotinyliertem Anti-human-IgG als Nachweisreagenz verdünnt in PBS-T für 1h unter Schütteln inkubiert.

Schritt 7:

Nachfolgend werden ungebundene Bestandteile des Nachweis Reagenzes durch mehrmaliges Waschen in einem Überschuss PBS-T entfernt.

Schritt 8:

Nach dem PBS-T entfernt wurde, wird die Kavität mit Streptavidin und Peroxidase markierten Nano/Mikropartikeln als Nachweisreagenz verdünnt in PBS-T für 1h unter Schütteln inkubiert.

Schritte 9 bis 12:

Diese Schritte entsprechen den Schritten 7 bis 10 aus Beispiel 3a.

**Beispiel 4**

RNA-Moleküle einer Länge von ca. 15-30 Nukleotiden, die sog. mikroRNA (miRNA) sind direkt in die Regulation der Genexpression involviert. Es sind mehr als 1500 verschiedene miRNA unterschiedlicher Sequenz bekannt, die in die Regulation verschiedener physiologischer und pathophysiologischer Zustände involviert sind. Besonders die Bestimmung der miRNA-Konzentrationen im Serum von Patienten gilt als wenig invasives Verfahren von hoher klinischer Relevanz. Der Goldstandard für die quantitative Bestimmung von miRNA im Serum ist die Realtime-PCR. PCR-Bias, d.h. die Verfälschung der Mengenrelationen zwischen verschiedenen PCR durch unterschiedliche PCR-Effizienz, bzw. die mangelnde Multiplexfähigkeit der PCR oder zu geringe Sensitivität von Hybridisierungsverfahren sind jedoch wichtige Limitationen des gegenwärtigen Standes der Technik.
Durch das nachfolgend beschriebene Anwendungsbeispiel des erfindungsgemäßen Verfahrens wird die Detektion der miRNA-Moleküle auf Grund der hohen Sensitivität ohne PCR-Vervielfältigung erreicht. Die Nutzung der Chemilumineszenz als Detektionsverfahren garantiert einen sehr hohen Messbereich über 4-5 log-Stufen:

Schritt 1:

Suspensionen von drei unterschiedlich fluoreszenzkodierten, Polyacrylsäure-tragenden, COOH-modifizierte Polymethacrylat-Mikropartikel-Suspensionen, deren Mikropartikel einem Durchmesser von $10\,\mu m$ haben, werden EDC-aktiviert und danach mit je 1mg Streptavidin pro ml PBS für 2h inkubiert und somit mit Streptavidin beschichtet. Ungebundenes Streptavidin wird durch mehrmaliges Waschen in einem Überschuss an PBS-T vollständig entfernt. Dazu werden die Mikropartikel durch Zentrifugation sedimentiert, bevor die Waschlösung entfernt und durch neue Waschlösung ersetzt wird. Optional können weitere Mikropartikelpopulationen für weitere miRNA oder Kontrollen in den Ansatz einbezogen werden.

Schritt 2:

Danach werden die mit Streptavidin beschichteten Mikropartikelpopulationen mit 100nM biotinylierter miRNA-Sonde als Binder, d.h. jeweils biotinylierte DNA-Oligonukleotide komplementär zu den miRNA miR16, Anti-Sense zu miR16 sowie doppelt-mutiert zu miR16 populationsspezifisch inkubiert und gewaschen. Unter Einbeziehung weiterer Mikropartikel-Populationen kann die Analyse auf weitere miRNA im Multiplex-Ansatz ausgedehnt werden.

Schritt 3:

Eine Mikrotestplatte aus Polystyrol wird mit $10\,\mu g$ Polylysin / ml PBS über Nacht inkubiert und danach mehrmals mit PBS-T gewaschen.

Schritt 4:

Aus humanen Serumproben wird RNA isoliert (Li, Y and Kowdley, KV [2012] Method for microRNA isolation from clinical serum samples. Anal Biochem 431[1]: 69-75) und am 3'-Ende enzymatisch eine Poly-A-Verlängerung synthetisiert (Shingara J, Keiger K, Shelton J, Laosingchai-Wolf W, Owers P, Conrad R, Brown D, Labourier E [2005] An optimized isolation and labeling platform for accurate microRNA expression profiling. RNA, 11: 1461-1470).

Schritt 5:

Ein Aliquot von $20\,\mu l$ der Poly-A-miRNA wird mit einem Gemisch von $20\,\mu l$ einer Suspension der o.g. Sonden-tragenden Mikropartikelpopulationen, die etwa 500 Mikropartikel jeder Population enthalten, sowie $2\,\mu l$ 400nM Poly-$dT_{25}$-Biotin-DNA-Oligonukleotides gemischt und entsprechend Shingara et al (2012) unter Schütteln in einem Eppendorf-Reaktionsgefäß bei 42°C für 2h inkubiert, so dass die miRNA der Serumproben an die Fängersonden und die Poly-$dT_{25}$-Biotin-Sonde an das Poly-A-Ende der miRNA binden kann.

Schritt 6:

Nachfolgend werden ungebundene Bestandteile der Probe durch mehrmaliges Waschen in einem Überschuss PBS-T entfernt.

Schritt 7:

Dann werden $200\,\mu l$ PBS in die vorbereitete Mikrotestplatte pipettiert und $20\,\mu l$ der Mikropartikel-Suspension dazu gegeben und für 10min inkubiert, bis die Mikropartikel auf den Boden der Kavität der Mikrotestplatte abgesunken und am Polylysin immobilisiert sind.

Schritt 8:

Nach dem PBS entfernt wurde, wird die Kavität der Mikrotestplatte mit Extravidin-POD (Sigma) als Nachweisreagenz verdünnt in PBS-T für 1h ohne Schütteln inkubiert.

Schritt 9:

Nachfolgend werden ungebundene Bestandteile des Nachweis Reagenzes durch mehrmaliges Waschen in einem Überschuss PBS-T und dann mit PBS entfernt.

Schritt 10:

Nach dem gründlichen Entfernen der Waschlösung durch Absaugen wird eine kommerzielle Chemilumineszenz-Fertigsubstratlösung (Lumigen ECL Plus, Diarect) und nach etwa 30sec wieder aus der Kavität durch Absaugen entfernt und sofort mit 50µl Mineralöl überschichtet.

Schritt 11: Vermessung im F1100 Fluoreszenzimager

Die Mikrotestplatte wird im Fl100 Fluoreszenzimager (Attomol) ausgewertet, indem zuerst Fluoreszenzbilder bei einer Anregungswellenlängen von 480nm, 530nm und entsprechenden Emissionswellenlängen von 520nm, 560nm mit dem optischen Fokus im Reaktionsraum und einer Pixelauflösung von 2,16µm einer Kavität aufgezeichnet. Danach wird das Filterrad auf eine Position ohne Fluoreszenzfilter gefahren, um die Lumineszenzbilder dieser Kavitäten mit einer Pixelauflösung von 0,3-0,65 und einer Belichtungszeit von 1-10sec entsprechend der Lumineszenzintensität automatisch aufzuzeichnen. Danach wird das automatisch optimal fokussierte und belichtete Bild automatisch ausgewählt und dem Imagingprozess mittels FastFluoscan-Software (Attomol) zugeführt, nachdem die Qualität der Bilder automatisch kontrolliert und ggf., z.B. durch das eliminieren von Artefakten, korrigiert wurden.

Schritt 12: Imaging und Auswertung

Die FastFluoscan-Software erkennt alle Mikropartikel im Bild und ordnet diese nach Auswertung der Kodierungsfluoreszenzen (Emissionen bei 520nm bzw. 560nm) einer der drei Mikropartikelpopulationen zu. Danach wird die analytkonzentrationsabhängige Chemilumineszenz für die einzelnen Mikropartikel im jeweiligen Reaktionsraum ermittelt. Daraus wird berechnet, welche Mikropartikel einer Population positiv sind, insbesondere wenn diese dichter mit Mikropartikeln anderer Populationen derselben Population in der Kavität benachbart liegen, so dass deren Reaktionsräume nicht vollständig voneinander getrennt sein könnten. Das Maß dafür ist die Zeit mit der ein entsprechendes Mikropartikel eine Chemilumineszenz aufweist im Verhältnis zur durchschnittlichen Zeit für diese Mikropartikelpopulation. Ist die Chemilumineszenz eines Mikropartikels signifikant geringer als die durchschnittliche Zeit für die Mikropartikelpopulation und eine Nachbarpopulation zeigt eine signifikant höhere Chemilumineszenz, dann ist diese Population als negativ zu bewerten. Entspricht sie der durchschnittlichen Zeit, wird sie als positiv gewertet. Danach werden die Messwerte, d.h. die Höhe des mittleren Lumineszenzsignals, für jede Mikropartikelpopulation auf eine Referenzprobenreihe mit Kalibrierfunktion bezogen, um die Analytkonzentration der miRNA in der Probe anzugeben. Der Cut-off für die Positiv-/Negativ-Bewertung der jeweiligen miRNA wird aus den jeweiligen Negativkontrollen (Anti-sense, Doppelt-mutiert) berechnet.

**Beispiel 5**: **PCR-Nachweis**

Schritt 1:

Drei unterschiedlich fluoreszenzkodierte, Polyacrylsäure-tragende, COOH-modifizierte Polymethacrylat-Mikropartikel-Suspensionen, deren Mikropartikel einem Durchmesser von 10 µm haben, werden EDC-aktiviert und mit je 1mg Streptavidin pro ml PBS für 2h inkubiert und so mit Streptavidin beschichtet. Ungebundenes Streptavidin wird durch mehrmaliges Waschen in einem Überschuss an PBS-T vollständig entfernt. Dazu werden die Mikropartikel durch Zentrifugation sedimentiert, bevor die Waschlösung entfernt und durch neue Waschlösung ersetzt wird. Optional können weitere Mikropartikelpopulationen für weitere Primerpaare in den Ansatz einbezogen werden.

Schritt 2: Beschichtung der Mikropartikel mit Primern

Danach werden die mit Streptavidin beschichteten Mikropartikelpopulationen mit 100nM biotinylierter Vorwärts- und Rückwärtsprimer äquimolar populationsspezifisch inkubiert und gewaschen. Unter Einbeziehung weiterer Mikropartikel-Populationen kann die Beschichtung mit weiteren Primern im Multiplex-Ansatz ausgedehnt werden.

Schritt 3:

E. coli-DNA wurde entsprechend Frömmel et al. (2013) isoliert und entsprechend in den PCR-Mix pipettiert. An Stelle der E.coli-spezifischen Primer werden die Universalprimer 1 und 2 in einer finalen Konzentration von 400nm und die Sonden (siehe unten) jeweils in einer Konzentration von 100nM in den PCR-Mix pipettiert und in ein Aliquot von 5µl Mikropartikelsuspension, die 500 Mikropartikel jeder mit Vorwärts- und Rückwärtsprimer beschichteten Mikropartikelpopulation und etwa 100.000 nichtfluoreszenzkodierte und nicht oberflächenfunktionalisierte Mikropartikel aus PMMA oder Silica mit einem Durchmesser von 10µm enthält, pipettiert.

Schritt 4:

Danach wird der Mix samt Mikropartikel in die Kavität einer Nukleolink-Platte pipettiert. Nach dem Absetzen der Mikropartikel im PCR-Mix' wird der Mix vorsichtig abgesaugt, so dass am Boden der Kavität nur die Schicht mit den Mikropartikeln und der in den Spalträumen zwischen den Mikropartikeln befindliche Mix in der Kavität bleibt. Danach wird der Reaktionsansatz mit Mineralöl überschichtet und in den Thermocycler des FI100 Fluoreszenzimagers überführt und das Thermocyclerprogramm gestartet. Es werden 40 Thermocyclen folgender Konfiguration gefahren: Initial 5min, 95°C. Danach: 30sec, 95°C; 1min, 60°C; 30sec, 72°C.

Schritt 5: Vermessung im F1100 Fluoreszenzimager

Die Nukleolinkstreifen werden im FI100 Fluoreszenzimager (Attomol) ausgewertet, indem Fluoreszenzbilder bei einer Anregungswellenlängen von 480nm, 530nm und 640nm und entsprechenden Emissionswellenlängen von 520nm, 560nm bzw. 660nm mit dem optischen Fokus im Reaktionsraum und einer Pixelauflösung von $2,16\mu m$ in der Annealing-Phase eines jedem Thermozyklus aufgezeichnet. Das automatisch optimal fokussierte und belichtete Bild wird automatisch ausgewählt und dem Imaging-Prozess mittels FastFluoscan-Software (Attomol) zugeführt, nachdem die Qualität der Bilder automatisch kontrolliert und ggf. korrigiert wird.

Schritt 6: Imaging und Auswertung

Die FastFluoscan-Software erkennt alle Mikropartikel im Bild und ordnet diese nach Auswertung der Kodierungsfluoreszenzen (Emissionen bei 520nm bzw. 560nm) einer der drei Mikropartikelpopulationen zu. Danach wird die analytkonzentrationsabhängige Atto 647N-Fluoreszenzveränderung für die einzelnen Mikropartikel im jeweiligen Reaktionsraum für jeden Thermozyklus ermittelt. Daraus wird berechnet, welche Mikropartikel einer Population positiv sind, insbesondere wenn diese dichter mit Mikropartikeln anderer Populationen derselben Population in der Kavität benachbart liegen, da deren Reaktionsräume nicht vollständig voneinander getrennt sind. Das Maß dafür ist die Zeit mit der ein entsprechendes Mikropartikel eine Cy5-Fluoreszenz aufweist im Verhältnis zur durchschnittlichen Zeit der Cy5-Fluoreszenzsignalentstehung für diese Mikropartikelpopulation. Ist die Cy5-Fluoreszenzsignalentstehung signifikant geringer als die durchschnittliche Zeit und eine Nachbarpopulation zeigt eine Cy5-Fluoreszenz, ist diese Population als negativ zu werten. Entspricht sie der durchschnittlichen Zeit, wird sie als positiv gewertet. Ist durch diesen einfachen Algorithmus keine genaue Zuordnung des Cy5-Signals möglich, werden Intensitätsvektoralgorithmen eingesetzt, um die Kinetik der Signalverteilung für die Zuordnung des Cy5-Signals zu einem Mikropartikel zweier benachbarter Mikropartikel zu errechnen. Der Abfall des Intensitätssignals vom Mikropartikel weg ist für ein positives Mikropartikel steiler als für ein negatives Mikropartikel. Die Kinetik der Intensitätsvektoren ist besonders hilfreich, da die Steilheit des Intensitätsabfalls zu unterschiedlichen Reaktionszyklen unterschiedlich deutlich berechnet werden kann. Besonders sicher ist die Zuordnung der Signale in der exponentiellen Phase der Signalbildung in der PCR. In Fällen, wo keine eindeutige Zuordnung möglich ist, können eng beieinander liegende fluoreszenzkodierte Mikropartikel aus der Auswertung ausgeschlossen werden. Bei 500 Mikropartikel jeder Population bleiben durchschnittlich mehr als 50% auswertbar, was insbesondere bei stärker positiven Proben >500 Kopien pro Analyt eine sichere Auswertung ermöglicht. Schwach positive Proben <500 Kopien pro Probe ergeben ein quasi digitales Signal, d.h. es zeigen nicht mehr alle Mikropartikel einer Population ein Cy5-Signal. Hier kann jedoch eine statistische Bewertung der reaktiven Mikropartikel vorgenommen werden, da die gleiche Positivrate einzeln liegender, eindeutig auswertbarer Mikropartikel und eng beieinander liegender nicht eindeutig auswertbarer Mikropartikel angenommen werden kann.

Danach werden die positiven Mikropartikel und die negativen Mikropartikel einer Population gezählt und deren Ratio als Maß für die Analytkonzentration auf eine Referenzprobenreihe mit Kalibrierfunktion bezogen, um die Analytkonzentration in der Probe anzugeben. Alternativ ist es bei schwach positiven Proben möglich, die Zahl der positiven Mikropartikel zu zählen und über das Reaktionsraumvolumen auf das Probenvolumen zu beziehen und somit zu quantifizieren.

Folgende Oligonukleotidsequenzen wurden für die Synthese der Primer und Sonden eingesetzt:

Die für die E. coli-Pathogenitätsfaktoren spezifischen Abschnitte der Primer und Sonden wurden Frömmel et al. (2013) (Frömmel U, Lehmann W, Rödiger S, Böhm A, Nitschke J, Weinreich J, Groß J, Roggenbuck D Zinke O, Ansorge H, Vogel S, Klemm P, Wex T, Schröder C, Wieler LH, Schierack P (2013) Adhesion of human and animal Escherichia coli strains in association with their virulence-associated genes and phylogenetic origins. Applied and Environmental Microbiology 79: 3-16.) entnommen. An jeden Vorwärtsprimer wurde zusätzlich die Sequenz von Universalprimer 1 angehängt und eine 5'-Biotinmarkierung eingeführt. An jeden Rückwärtsprimer wurde zusätzlich die Sequenz von Universalprimer 2 angehängt und eine 5'-Biotinmarkierung eingeführt. Die Sonden, die als TaqMan-Sonden fungieren, wurden zusätzlich mit einer 5'-Cy5-Markierung und einer 3'-DeepDarkQuencher II-Markierung versehen und um einige Nukleotide entsprechend der GeneBank-Sequenz verlängert.

Universalprimer 1: GCAGCGTCGATGGAGTCA
Universalprimer2: ACTCACGCTCACACGGAC

### stx2e

Vorwärts-Primer mit 5'-Biotin-Markierung:
5'-GCAGCGTCGATGGAGTCAGGAGCGTTTTGACCATCTTC-3'
Rückwärts-Primer mit 5'-Biotin-Markierung:
5'-ACTCACGCTCACACGGACGCGCCAGATATGATGAAACCA-3'
Sonde mit 5'-Cy5- und 3'-DeepDarkQuencher II-Modifizierung:
5'-CCGGAATGCAAATCAGTCGTCACTCAC-3'

### stx1

Vorwärts-Primer mit 5'-Biotin-Markierung:
5'-GCAGCGTCGATGGAGTCACTGGATTTAATGTCGCATAGTG-3'

Rückwärts-Primer mit 5'-Biotin-Markierung:
5'-ACTCACGCTCACACGGACGAGAACGCCCACTGAGATCATC-3'

Sonde mit 5'-Cy5- und 3'- DeepDarkQuencher II-Modifizierung:
5'-CGGCAAATACAGAGGGGATTTCGTACAACAC-3'

### eaeA

Vorwärts-Primer mit 5'-Biotin-Markierung:
5'-GCAGCGTCGATGGAGTCACCTGGTTACAACATTATGGAACG-3'

Rückwärts-Primer mit 5'-Biotin-Markierung:
5'-ACTCACGCTCACACGGACGTGAAATAGTCTCGCCAGTATTCG-3'

Sonde mit 5'-Cy5- und 3'- DeepDarkQuencher II -Modifizierung:
5'- ATGTTGGGCTATAACGTCTTCATTGATCAGG -3'

Bei Verwendung von Primern und Sonden, die für andere Erreger spezifisch sind und die auf anderen Mikropartikelpopulationen immobilisiert werden, lässt sich das Verfahren leicht auf andere Anwenderfragestellungen wie z.B. die Detektion und Differenzierung von humanen Papillomaviren, der Detektion von Resistenzfaktoren von *Helicobacter pylori* oder zur kombinierten Detektion von Sepsiserregern nutzen. Quasi digitale Nachweise von Tumormarkern können unter Verwendung tumorspezifischer Oligonukleotidsequenzen wie z.B. Methylierungsmarker auch zum Nachweis einzelner Tumorzellen aus z.B. Blut oder Urin eingesetzt werden.

**Patentansprüche**

1.  Verfahren zum Nachweis von Analyten in einer Probe, umfassend die Schritte:

    a) Aufbringen einer Probe und einer Reaktionslösung auf einen Träger, wobei die Reaktionslösung geeignet ist in einer Signalverstärkungsreaktion in Abhängigkeit des Vorhandenseins des nachzuweisenden Analyten ein messbares Reaktionsprodukt entstehen zu lassen;
    b) Durchführung der Signalverstärkungsreaktion;
    c) optische Messung der Reaktionsprodukte; und
    d) ggf. Bereitstellung der Messdaten für die Berechnung einer Analytkonzentration in einer Probe;

    wobei vor der Durchführung der Signalverstärkungsreaktion auf dem Träger eine Mehrzahl räumlich getrennter Reaktionsräume eingerichtet wird, indem vor, während oder nach dem Aufbringen der Probe und der Reaktionslösung Mikropartikel auf dem Träger immobilisiert werden, so dass die Mikropartikel von der Reaktionslösung überschichtet sind und anschließend die Reaktionslösung entfernt wird, so dass benachbarte, sich nicht berührende immobilisierte Mikropartikel nicht mehr von einer zusammenhängenden Schicht Reaktionslösung überdeckt sind; **dadurch gekennzeichnet, dass** die Entfernung der Reaktionslösung derart erfolgt, dass die Oberfläche des Trägers lediglich in einem Bereich mit Reaktionslösung bedeckt bleibt, der unmittelbar eine Kontaktstelle zwischen Mikropartikel und Träger umgibt, während Oberflächenbereiche des Trägers, die mindestens den ≥0,1-fachen Partikeldurchmesser von einer Kontaktstelle zwischen einem Mikropartikel und dem Träger entfernt sind, nicht mehr von Reaktionslösung bedeckt sind.

2.  Verfahren nach Anspruch 1, wobei die Mikropartikel und/oder der Träger auf ihrer Oberfläche Binder aufweisen, die spezifisch sind für die nachzuweisenden Analyten.

3.  Verfahren nach Anspruch 1 oder 2, wobei die Reaktionslösung ein Substrat aufweist, welches während der Signalverstärkungsreaktion durch eine enzymatische Aktivität umgewandelt wird und dadurch das messbare Reaktionsprodukt entsteht oder ohne enzymatische Reaktion ein messbares Substrat an die Partikeloberfläche bindet.

4.  Verfahren nach einem der vorhergehenden Ansprüche, wobei die Probe oder die auf dem Träger immobilisierten Mikropartikel vor der Durchführung der Signalverstärkungsreaktion mit einem Signalverstärkungssystem kontaktiert werden.

5.  Verfahren nach Anspruch 3 oder 4, wobei der nachzuweisende Analyt und/oder das Signalverstärkungssystem die enzymatische Aktivität aufweist, wobei es sich bei der enzymatischen Aktivität bevorzugt um die Aktivität eines Enzyms aus der Klasse der DNA-Polymerasen, RNA-Polymerasen, reversen Transkriptasen, Ligasen, Oxidasen, Reduktasen, Transferasen, Peroxidasen, Phosphatasen, Proteasen, Peptidasen, Lipasen, Glykosidasen und/oder Luciferasen handelt.

6.  Verfahren nach Anspruch 4 oder 5, wobei das Signalverstärkungssystem ein erstes Element und ein zweites Element aufweist, wobei das erste Element spezifisch an den nachzuweisenden Analyten oder an einen mit dem Analyten besetzten Binder des Mikropartikels bindet, und das zweite Element die enzymatische Aktivität aufweist.

7.  Verfahren nach einem der vorhergehenden Ansprüche, wobei die Mikropartikel einen mittleren Durchmesser von unter 1 mm aufweisen, bevorzugt 0,01 bis 100 μm, besonders bevorzugt 0,05 bis 50 μm, ganz besonders bevorzugt 1 bis 30 μm.

8.  Verfahren nach einem der vorhergehenden Ansprüche, wobei mehr als eine Mikropartikelpopulation verwendet wird, wobei sich die verschiedenen Mikropartikelpopulationen mindestens darin unterscheiden, dass sie Binder tragen, die sich in ihrer Bindungsspezifität unterscheiden, bevorzugt weisen die unterschiedlichen Mikropartikelpopulationen Binder auf, die für unterschiedliche Analyten spezifisch sind.

9. Verfahren nach einem der vorhergehenden Ansprüche, wobei nach Einrichtung der räumlich getrennten Reaktionsräume eine Stabilisierung der getrennten Reaktionsräume durch Überschichtung der auf dem Träger immobilisierten Mikropartikel mit einer Trennflüssigkeit, die mit der Reaktionslösung nicht mischbar ist, stattfindet.

10. Verfahren nach einem der vorhergehenden Ansprüche, wobei die optische Messung der Reaktionsprodukte der Signalverstärkungsreaktion derart ausgeführt wird, dass die räumlich getrennten Reaktionsräume des Trägers individualisierbar erfasst werden, so dass Messwerte einzelnen Reaktionsräumen und/oder einzelnen Mikropartikeln zuordenbar sind.

11. Verfahren nach einem der vorhergehenden Ansprüche, wobei die Entfernung der Reaktionslösung derart erfolgt, dass die Oberfläche des Trägers lediglich in einem Bereich mit Reaktionslösung bedeckt bleibt, der unmittelbar eine Kontaktstelle zwischen Mikropartikel und Träger umgibt, während Oberflächenbereiche des Trägers, die mindestens den ≥1-fachen Partikeldurchmesser von einer Kontaktstelle zwischen einem Mikropartikel und dem Träger entfernt sind, nicht mehr von Reaktionslösung bedeckt sind.

12. Verfahren nach einem der vorhergehenden Ansprüche, wobei die Mikropartikel gerichtet auf dem Träger immobilisiert werden.

13. Verfahren nach einem der vorhergehenden Ansprüche, wobei die Mikropartikel vereinzelt auf dem Träger vorliegen, bevorzugt in einem durchschnittlichen Abstand, der dem 1 bis 20-fachen Partikeldurchmesser entspricht.

14. Verfahren nach einem der vorhergehenden Ansprüche, wobei nach Einrichtung der räumlich getrennten Reaktionsräume durch die Mikropartikel eine Stabilisierung der getrennten Reaktionsräume durch Immobilisierung anderer Mikropartikelpopulationen ggf. im Gemisch mit Nanopartikeln als Abstandshalter erfolgt, wobei die Porengröße der anderen Mikropartikel und ggf. Nanopartikel der Porengröße der Mikropartikel, die die Reaktionsräume gewährleisten, angepasst werden, um in den Reaktionsräumen gezielt Ausgangsprodukte der Signalverstärkungsreaktion aufzunehmen, zu speichern und abzugeben.

15. Verwendung des Verfahrens nach einem der Ansprüche 1 bis 14 für eine qualitative und quantitative Nukleinsäureamplifikation und -detektion, ultrasensitive reverse Hybridisierungsassays von DNA und RNA, ein Nukleinsäure-Sequenzscreening zur Detektion von Sequenzvariationen, ultrasensitive Immunoassays, Multiplex-Einzelzell-Assays in miniaturisierten Reaktionsräumen in vitro jeweils im Singleplex bzw. Multiplex-Format.

## Claims

1. A method for detecting analytes in a sample, comprising the steps of:

   a) depositing a sample and a reaction solution on a support, wherein the reaction solution is suited to generating a measurable reaction product in a signal amplification reaction as a function of the presence of the analyte to be detected;
   b) carrying out the signal amplification reaction;
   c) optically measuring the reaction products; and
   d) if applicable, providing the measuring data in order to calculate an analyte concentration in a sample;

   wherein a plurality of spatially separated reaction chambers is set up on the support prior to carrying out the signal amplification reaction, in that prior to, during, or after depositing the sample and the reaction solution, microparticles are immobilised on the support such that the microparticles are coated by the reaction solution and the reaction solution is then removed such that adjacent, non-contacting immobilised microparticles are no longer covered by a connected layer of the reaction solution;
   **characterised in that**
   the reaction solution is removed such that the surface of the support merely remains covered with reaction solution in a region which immediately surrounds a contact point between microparticles and the support, while surface regions of the support, which are removed at least ≥0.1 times the particle diameter from a contact point between a microparticle and the support, are no longer covered by reaction solution.

2. The method according to Claim 1, wherein the microparticles and/or the support have/has binders on their/its surface, which are specific for the analytes to be detected.

3. The method according to Claim 1 or 2, wherein the reaction solution has a substrate which is converted by enzymatic activity during the signal amplification reaction and, as a result, the measurable reaction product is generated or a measurable substrate binds to the particle surface without an enzymatic reaction.

4. The method according to any one of the preceding claims, wherein the sample or the microparticles immobilised on the support are contacted with a signal amplification system, prior to carrying out the signal amplification reaction.

5. The method according to Claim 3 or 4, wherein the analyte to be detected and/or the signal amplification system has/have enzymatic activity, wherein the enzymatic activity is preferably the activity of an enzyme from the class of DNA polymerases, RNA polymerases, reverse transcriptases, ligases, oxidases, reductases, transferases, peroxidases, phosphatases, proteases, peptidases, lipases, glycosidases and/or luciferases.

6. The method according to Claim 4 or 5, wherein the signal amplification system has a first element and a second element, wherein the first element specifically binds to the analyte to be detected or to a binder of the microparticle occupied by the analyte, and the second element has enzymatic activity.

7. The method according to any one of the preceding claims, wherein the microparticles have an average diameter of less than 1 mm, preferably 0.01 to 100 $\mu$m, particularly preferably 0.05 to 50 $\mu$m, very particularly preferably 1 to 30 $\mu$m.

8. The method according to any one of the preceding claims, wherein more than one microparticle population is used, wherein the various microparticle populations differ at least in that they support binders which differ in their binding specificity, the different microparticle populations preferably have binders which are specific for different analytes.

9. The method according to any one of the preceding claims, wherein, after setting up the spatially separated reaction chambers, a stabilisation of the separated reaction chambers takes place by overlaying the microparticles immobilised on the support with a separating fluid which is not mixable with the reaction solution.

10. The method according to any one of the preceding claims, wherein the reaction products of the signal amplification reaction are optically measured such that the spatially separated reaction chambers of the support are captured in a customisable manner so that measurement values are assignable to individual reaction chambers and/or individual microparticles.

11. The method according to any one of the preceding claims, wherein the reaction solution is removed such that the surface of the support merely remains covered with reaction solution in a region which immediately surrounds a contact point between microparticles and the support, while surface regions of the support, which are removed at least ≥1 times the particle diameter from a contact point between a microparticle and the support, are no longer covered by reaction solution.

12. The method according to any one of the preceding claims, wherein the microparticles are immobilised, in a directed manner, on the support.

13. The method according to any one of the preceding claims, wherein the microparticles are individually present on the support, preferably at an average distance which corresponds to 1 to 20 times the particle diameter.

14. The method according to any one of the preceding claims, wherein, after setting up the spatially separated reaction chambers by the microparticles, the separated reaction chambers are stabilised by immobilising other microparticle populations, if applicable in a mixture with nanoparticles as spacers, wherein the pore size of the other microparticles and, if applicable, nanoparticles are adjusted to the pore size of the microparticles which guarantee the reaction chambers, in order to deliberately receive and store initial products of the signal amplification reaction in the reaction chambers, and to deliver said initial products.

15. A use of the method according to any one of Claims 1 to 14 for a qualitative and quantitative nucleic acid amplification and detection, ultrasensitive reverse hybridisation assays of DNA and RNA, nucleic acid sequence screening for detecting sequence variations, ultrasensitive immunoassays, multiplex individual cell assays in miniaturised reaction chambers in vitro, in each case, in the singleplex or the multiplex format.

## Revendications

1. Procédé de recherche d'analytes dans un échantillon, comprenant les étapes suivantes :

   a) application d'un échantillon et d'une solution de réaction sur un support, la solution de réaction étant appropriée pour permettre la formation d'un produit de réaction mesurable par réaction d'amplification de signal en fonction de la présence de l'analyte à rechercher ;
   b) exécution de la réaction d'amplification de signal ;
   c) mesure optique des produits de réaction ; et,
   d) le cas échéant, fourniture des données de mesure pour le calcul d'une concentration d'analyte dans un échantillon ;

   où, antérieurement à l'exécution de la réaction d'amplification de signal sur le support, une pluralité de chambres de réaction spatialement séparées est créée, des microparticules étant fixées sur le support avant, pendant ou après l'application de l'échantillon et de la solution de réaction, de manière à recouvrir les microparticules par la solution de réaction, et où la solution de réaction est retirée ensuite, si bien que des microparticules adjacentes fixées qui ne sont pas en contact ne sont plus recouvertes d'une couche continue de solution de réaction ; **caractérisé en ce que**
   le retrait de la solution de réaction est effectué de telle manière que la surface du support ne reste couverte de solution de réaction que dans une zone qui entoure directement une zone de contact entre microparticules et support, cependant que des zones de surface du support distantes d'au moins ≥0,1 fois le diamètre de particule d'une zone de contact entre une microparticule et le support ne sont plus couvertes par la solution de réaction.

2. Procédé selon la revendication 1, où les microparticules et/ou le support présentent sur leur surface des liants spécifiques pour l'analyte à rechercher.

3. Procédé selon la revendication 1 ou la revendication 2, où la solution de réaction présente un substrat qui est converti par activité enzymatique pendant la réaction d'amplification de signal, le produit de réaction mesurable étant ainsi formé, ou lie sans réaction enzymatique un substrat mesurable à la surface de particule.

4. Procédé selon l'une quelconque des revendications précédentes, où l'échantillon ou les microparticules fixées sur le support sont contactées avec un système d'amplification de signal avant l'exécution de la réaction d'amplification de signal.

5. Procédé selon la revendication 3 ou la revendication 4, où l'analyte à mesurer et/ou le système d'amplification de signal présentent l'activité enzymatique, où l'activité enzymatique est préférentiellement l'activité d'une enzyme de la classe des ADN polymérases, ARN polymérases, transcriptases inverses, ligases, oxydases, réductases, transférases, peroxydases, phosphatases, protéases, peptidases, lipases, glycosidases et/ou luciférases.

6. Procédé selon la revendication 4 ou la revendication 5, où le système d'amplification de signal comporte un premier élément et un deuxième élément, le premier élément se liant spécifiquement à l'analyte à rechercher ou à un liant de la microparticule occupé par l'analyte, et le deuxième élément présentant l'activité enzymatique.

7. Procédé selon l'une quelconque des revendications précédentes, où les microparticules ont un diamètre moyen inférieur à 1 mm, avantageusement compris entre 0,01 et 100 $\mu$m, préférentiellement entre 0,05 et 50 $\mu$m, et tout particulièrement entre 1 et 30 $\mu$m.

8. Procédé selon l'une quelconque des revendications précédentes, où plus d'une population de microparticules est utilisée, où les différentes populations de microparticules se différencient au moins en ce qu'elles portent des liants distincts par leur spécificité de liaison, et où les différentes populations de microparticules présentent préférentiellement des liants spécifiques pour des analytes différents.

9. Procédé selon l'une quelconque des revendications précédentes, où, après création des chambres de réaction spatialement séparées, a lieu une stabilisation des chambres de réaction séparées par couverture des microparticules fixées sur le support par un liquide de séparation non mélangeable à la solution de réaction.

10. Procédé selon l'une quelconque des revendications précédentes, où la mesure optique des produits de réaction de la réaction d'amplification de signal est exécutée de manière à détecter individuellement les chambres de réaction spatialement séparées du support, ce qui permet d'affecter les valeurs de mesure à différentes chambres de réaction et/ou à différentes microparticules.

**11.** Procédé selon l'une quelconque des revendications précédentes, où le retrait de la solution de réaction est effectué de telle manière que la surface du support ne reste couverte de solution de réaction que dans une zone qui entoure directement une zone de contact entre microparticules et support, cependant que des zones de surface du support distantes d'au moins ≥1 fois le diamètre de particule d'une zone de contact entre une microparticule et le support ne sont plus couvertes par la solution de réaction.

**12.** Procédé selon l'une quelconque des revendications précédentes, où les microparticules sont fixées de manière orientée sur le support.

**13.** Procédé selon l'une quelconque des revendications précédentes, où les microparticules sont présentées isolément sur le support, préférentiellement à une distance moyenne correspondant à 1 à 20 fois le diamètre de particule.

**14.** Procédé selon l'une quelconque des revendications précédentes, où, après création des chambres de réaction spatialement séparées par les microparticules, a lieu une stabilisation des chambres de réaction séparées par fixation d'autres populations de microparticules, le cas échéant en mélange avec des nanoparticules en tant qu'éléments espaceurs, la grandeur de pore des autres microparticules et le cas échéant des nanoparticules étant ajustée à la grandeur de pore des microparticules assurant les chambres de réaction, pour recevoir, stocker et délivrer de manière ciblée dans les chambres de réaction des produits de départ de la réaction d'amplification de signal.

**15.** Utilisation du procédé selon l'une quelconque des revendications 1 à 14 pour une amplification et détection qualitative et quantitative d'acide nucléique, des tests d'hybridation ultrasensible inverse ADN-ARN, un screening séquentiel d'acide nucléique pour la détection de variations de séquences, des essais immunologiques ultrasensibles, des analyses multiplex de cellules uniques in vitro dans des chambres de réaction miniaturisées au format uniplexe ou multiplexe.

Figur 1

Figur 2

Figur 3

Figur 4

Figur 5

Figur 6:

A

B

Figur 7

Figur 8

Figur 9

Figur 10

Figur 11

Figur 12

Figur 13

Figur 14

Figur 15

Figur 16

Figur 17

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2006105352 A1
- DE 69907630 T2
- DE 10054382 A1


**In der Beschreibung aufgeführte Nicht-Patentliteratur**

- **RONNY SCHMIDT ; JAROSLAW JACAK ; CHRISTOPHER SCHIRWITZ ; VOLKER STADLER ; GERD MICHEL ; NICOLE MARME ; GERHARD J. SCHÜTZ ; JÖRG D. HOHEISEL ; JENS-PETER KNEMEYER.** Single-Molecule Detection on a Protein-Array Assay Platform for the Exposure of a Tuberculosis Antigen. *J. Proteome Res.,* 2011, vol. 10, 1316-1322
- **RISSIN et al.** Single-molecule enzyme-linked immunosorbent assay detects serum proteins at subfemtomolar concentrations. *Nature Biotechnology,* 2010, vol. 28, 595-599
- **WILSON et al.** Fifth-Generation Digital Immunoassay for Prostate-Specific Antigen by Single Molecule Array Technology. *Clinical Chemistry,* 2011, vol. 57 (12), 1712-1721
- **TODD et al.** Ultrasensitive Flow-based Immunoassays Using Single-Molecule Counting. *Clinical Chemistry,* 2007, vol. 53 (11), 1990-1995
- **KRISHNASWAMI et al.** *Optical Nanoscopy,* 2014
- **VOGELSTEIN, B. ; KINZLER, K.W.** Digital PCR. *Proc. Natl. Acad. Sci. USA,* 1999, vol. 96, 9236-9241

- **GALLATI ; PRACHT.** Bestimmung von Peroxidase mit H2O2 und 3,3',5,5'-Tetramethylbenzidin. *J. Clin. Chem. Clin. Biochem.,* 1985, vol. 23, 4453-460
- Microencapsulation. **A. FINCHAND ; R. BODMEIER.** Ullmann's Encyclopedia of Industrial Chemistry. 2001
- **LI, Y ; KOWDLEY, KV.** Method for microRNA isolation from clinical serum samples. *Anal Biochem,* 2012, vol. 431 (1), 69-75
- **SHINGARA J ; KEIGER K ; SHELTON J ; LAOSINGCHAI-WOLF W ; OWERS P ; CONRAD R ; BROWN D ; LABOURIER E.** An optimized isolation and labeling platform for accurate microRNA expression profiling. *RNA,* 2005, vol. 11, 1461-1470
- **FRÖMMEL U ; LEHMANN W ; RÖDIGER S ; BÖHM A ; NITSCHKE J ; WEINREICH J ; GROß J ; ROGGENBUCK D ; ZINKE O ; ANSORGE H.** Adhesion of human and animal Escherichia coli strains in association with their virulence-associated genes and phylogenetic origins. *Applied and Environmental Microbiology,* 2013, vol. 79, 3-16